# EUROPÄISCHE PATENTANMELDUNG

(11) **EP 2 172 457 A2**
(43) Veröffentlichungstag der Anmeldung: **07.04.2010**
(21) Anmeldenummer: 09165928.4
(22) Anmeldetag: 20.03.2003
(51) Int. Cl.: C07D 221/20, C07D 401/12, C07D 417/12, C07D 405/12, C07H 7/06, A61K 31/4747, A61P 35/00, A61P 33/00

(54) **Fredericamycin-Derivate**

(30) Priorität: 26.03.2002 DE 10213580; 17.10.2002 DE 10248451
(62) Teilanmeldung aus: 03714862.4
(71) Anmelder: Biofrontera Discovery GmbH, 69123 Heidelberg (DE)
(72) Erfinder: Abel, Ulrich, 69118, Heidelberg (DE); Simon, Werner, 55595, Hüffelsheim (DE)
(74) Vertreter: Simandi, Claus

(57) **Zusammenfassung**

Die Erfindung betrifft neue Fredericamycin-Derivate, Arzneimittel die diese oder deren Salze enthalten, und die Verwendung der Fredericamycin-Derivate zur Behandlung von Erkrankungen, insbesondere Tumorerkrankungen.

## Beschreibung

Die Erfindung betrifft neue Fredericamycin-Derivate, Arzneimittel die diese oder deren Salze enthalten, und die Verwendung der Fredericamycin-Derivate zur Behandlung von Erkrankungen, insbesondere Tumorerkrankungen.

Fredericamycin wurde 1981 aus Streptomyces griseus isoliert und zeigt Antitumoraktivität.

Fredericamycin und einige Fredericamycin-Derivate sind bekannt.

In Heterocycles 37 (1994) 1893 - 1912, J. Am. Chem. Soc. 116 (1994) 9921 - 9926, J. Am. Chem. Soc. 116 (1994) 11275 -11286, J. Am. Chem. Soc. 117 (1995) 11839 - 11849, JP 2000-072752 und in J. Am. Chem. Soc. 123 (2001) sind verschiedene, auch enantioselektive, Totalsynthesen von Fredericamycin A beschrieben.

In US 4673768 sind Alkalisalze des Fredericamycin A beschrieben. In US 4584377 Fredericamycin-Derivate, insbesondere am Ring E und F acylierte Derivate, beschrieben. In US 5,166,208 sind ebenso Fredericamycin-Derivate beschrieben, insbesondere Derivate, die am Ring F Thio- oder Amino-Substituenten tragen. Die Derivate werden semisynthetisch oder totalsynthetisch hergestellt.

Überraschenderweise wurde gefunden, dass Fredericamycin-Derivate, die insbesondere am Ring A derivatisiert sind, potente Arzneimittel darstellen. Es wurde außerdem eine semisynthetische Möglichkeit gefunden Reste am Ring A einzuführen, die erlauben die Wasserlöslichkeit und/oder die biologische Wirksamkeit, das Wirkprofil gegenüber dem Fredericamycin beträchtlich zu erhöhen. Es wurde des weiteren eine Alternative gefunden Fredericamycin und deren Derivate wasserlöslich zu machen, in dem Cyclodextrin Einschlussverbindungen hergestellt werden.

Die Erfindung betrifft neue Fredericamycin-Derivate der allgemeinen Formel Ia oder Ib: 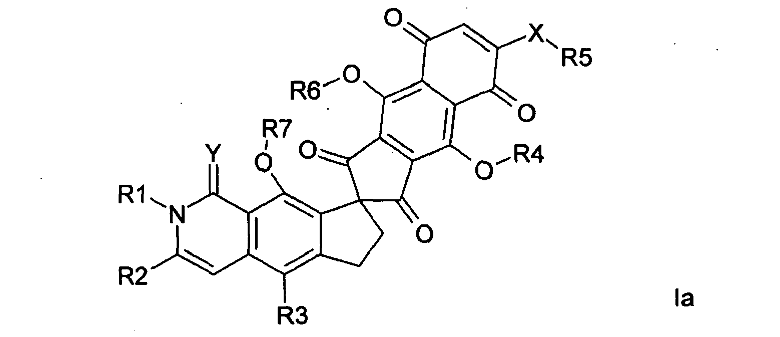 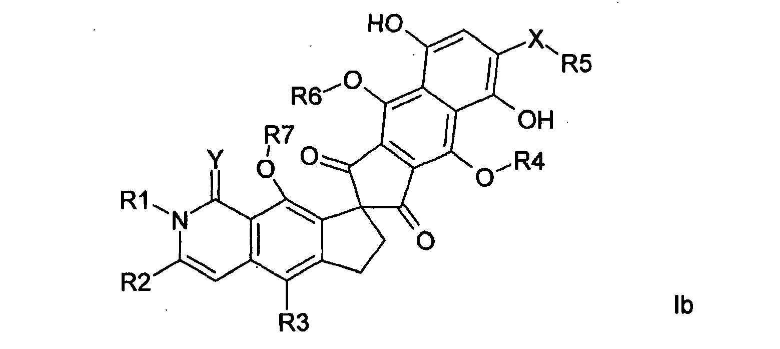 wobei jeweils
- R1: H, C₁-C₆-Alkyl, Cycloalkyl, C₁-C₄-Alkyl-Cycloalkyl,
- R2: H, C₁-C₁₄-Alkyl, C₂-C₁₄-Alkenyl, Aryl, C₁-C₄-Alkyl-Aryl, Heteroaryl, C₁-C₄-Alkyl-Heteroaryl, C₂-C₄-Alkenyl-Heteroaryl, Cycloalkyl, C₁-C₄-Alkyl-Cycloalkyl, Heterocycloalkyl, C₁-C₄-Alkyl-Heterocycloalkyl, CₘH₂ₘ₊ₒ₋ₚYₚ (mit m = 1 bis 6, für o = 1, p = 1 bis 2m+o; für m = 2 bis 6, o = -1, p = 1 bis 2m+o; für m = 4 bis 6, o = -2, p = 1 bis 2m+o; Y = unabhängig von einander ausgewählt aus der Gruppe Halogen, OH, OR21, NH₂, NHR21, NR21R22, SH, SR21), (CH₂)ᵣCH₂NHCOR21, (CH₂)ᵣCH₂OCOR21, (CH₂)ᵣCH₂NHCSR21, (CH₂)ᵣCH₂S(O)nR21 mit n = 0,1,2, (CH₂)ᵣCH₂SCOR21, (CH₂)ᵣCH₂OSO₂-R21, (CH₂)ᵣCHO, (CH₂)ᵣCH=NOH, (CH₂)ᵣCH(OH)R21, -(CH₂)ᵣCH=NOR21, (CH₂)ᵣCH=NOCOR21, (CH₂)ᵣCH=NOCH₂CONR21R22, (CH₂)ᵣCH=NOCH(CH₃)CONR21R22, -(CH₂)ᵣCH=NOC(CH₃)₂CONR21R22, (CH₂)ᵣCH=N-NHCO-R23, (CH₂)ᵣCH=N-NHC(O)NH-R23, (CH₂)ᵣCH=N-NHC(S)NH-R23, (CH₂)ᵣCH=N-NHC(NH)NH-R23, (CH₂)ᵣCH=N-NHC (NH) -R23, (CH₂)ᵣCH=N-NHCO-CH₂NHCOR21, (CH₂)ᵣCH=N-O-CH₂NHCOR21, (CH₂)ᵣCH=N-NHCS-R23, (CH₂)ᵣCH=CR24R25 ( trans oder cis ) , (CH₂)ᵣCOOH, (CH₂)ᵣCOOR21, (CH₂)ᵣCONR21R22, -(CH₂)ᵣCH=NR21,(CH₂)ᵣCH-N-NR21R22, 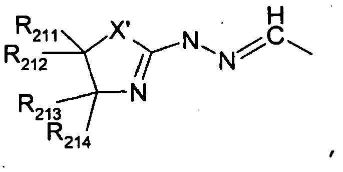 und der(CH₂)ᵣ-kettenverlängerte Rest (CH₂)ᵣCH=N-N-(C₃NX'R211R212R213R214) (mit X' = NR215, O, S und R211, R212, R213, R214, R215 unabhängig voneinander H oder C₁-C₆-Alkyl), -(CH₂)ᵣCH=N-NHSO₂-Aryl,

- (CH₂)ᵣCH=N-NHSO₂-Heteroaryl, mit r = 0,1,2,3,4,5, bevorzugt 0,
   - R21, R22: unabhängig voneinander H, C₁-C₁₄-Alkyl, C₁-C₁₄-Alkanoyl, C₁-C₆-Alkylhydroxy, C₁-C₆-Alkoxy, C₁-C₆-Alkylamino, C₁-C₆-Alkylamino-C₁-C₆-Alkyl, C₁-C₆-Alkylamino-di-C₁-C₆-Alkyl, Cycloalkyl, C₁-C₄-Alkyl-Cycloalkyl, Heterocycloalkyl, C₁-C₄-Alkyl-Heterocycloalkyl, Aryl, Aryloyl, C₁-C₄-Alkyl-Aryl, Heteroaryl, Heteroaryloyl, C₁-C₄-Alkyl-Heteroaryl, Cycloalkanoyl, C₁-C₄-Alkanoyl-Cycloalkyl, Heterocycloalkanoyl, C₁-C₄-Alkanoyl-Heterocycloalkyl, C₁-C₄-Alkanoyl-Aryl, C₁-C₄-Alkanoyl-Heteroaryl, Mono- und Di-Zuckerreste, die verknüpft sind über ein C-Atom, das im Zucker eine OH-Gruppe tragen würde, wobei die Zucker unabhängig voneinander ausgewählt sind aus der Gruppe bestehend aus Glucuronsäure und ihren Stereoisomeren an allen optischen C-Atomen, Aldopentosen, Aldohexosen einschließlich ihren Desoxyverbindungen (wie z.B. Glucose, Desoxyglucose, Ribose, Desoxyribose), oder R21 und R22 zusammen mit dem N eine 4, 5, 6, 7 oder 8 gliedrigen Ring bilden, der optional noch ein weiteres Herteroatom ausgewählt aus der Gruppe N, O, S enthalten kann,
   - R23: unabhängig von R21, die selben Bedeutungen wie R21 oder CH₂pyridinium-salze, CH₂tri-C₁-C₆-alkylammonmium-salze, CONH₂, CSNH₂, CN, CH₂CN,
   - R24: unabhängig von R21, die selben Bedeutungen wie R21 oder H, CN, COCH₃, COOH, COOR21, CONR21R22, NH₂, NHCOR21
   - R25: unabhängig von R21, die selben Bedeutungen wie R21 oder H, CN, COCH₃, COOH, COOR21, CONR21R22, NH₂, NHCOR21
   - R24,R25: zusammen mit dem N eine 4, 5, 6, 7 oder 8 gliedrigen Ring bilden, der optional noch ein weiteres Herteroatom ausgewählt aus der Gruppe N, O, S enthalten kann,
   - R3: H, F, Cl, Br, I, , OH, OR31, NO₂, NH₂, NHR31, NR31R32, NHCHO, NHCOR31, NHCOCF₃, CH₃₋ₘHalₘ (mit Hal = Cl, F, insbesondere F, und m = 1, 2, 3), OCOR31,
   - R31,R32: unabhängig voneinander C₁-C₆-Alkyl, oder R31 und R32 zusammen mit dem N eine 4, 5, 6, 7 oder 8 gliedrigen Ring bilden, der optional noch ein weiteres Herteroatom ausgewählt aus der Gruppe N, O, S enthalten kann,
   - R5: H, C₁-C₂₀-Alkyl, Cycloalkyl, C₂-C₂₀-Alkenyl, C₂-C₁₀-Alkinyle C₁-C₄-Alkyl-Cycloalkyl, Heterocycloalkyl, C₁-C₄-Alkyl-. Heterocycloalkyl, Aryl, C₁-C₄-Alkyl-Aryl, Heteroaryl, C₁-C₄-Alkyl-Heteroaryl, CₘH₂ₘ₊ₒ₋ₚYₚ (mit m = 1 bis 6, für o = 1, p = 1 bis 2m+o; für m = 2 bis 6, o = -1, p = 1 bis 2m+o; für m = 4 bis 6, o = -2, p = 1 bis 2m+o; Y = unabhängig von einander ausgewählt aus der Gruppe Halogen, OH, OR51, NH₂, NHR51, NR51R52, SH, SR51), (CH₂)ₛCH₂NHCOR51, (CH₂)ₛCH₂NHCSR51, (CH₂)ₛCH₂S(O)nR51 mit n=0,1,2, (CH₂)ₛCH₂SCOR51, (CH₂)ₛCH₂OCOR51, (CH₂)ₛCH₂OSO₂-R51, (CH₂)ₛCH(OH)R51, (CH₂)ₛCOOH, (CH₂)ₛCOOR51, (CH₂)ₛCONR51R52, mit s = 0,1,2,3,4,5, bevorzugt 0, Mono- und Di-Zuckerreste, die verknüpft sind über ein C-Atom, das im Zucker eine OH-Gruppe tragen würde, wobei die Zucker unabhängig voneinander ausgewählt sind aus der Gruppe bestehend aus Glucuronsäure und ihren Stereoisomeren an allen optischen C-Atomen, Aldopentosen, Aldohexosen einschließlich ihren Desoxyverbindungen (wie z.B. Glucose, Desoxyglucose, Ribose, Desoxyribose), wobei die Monozuckerreste wie Aldopentosen, Aldohexosen einschließlich ihren Desoxyverbindungen (wie z.B. Glucose, Desoxyglucose, Ribose, Desoxyribose) bevorzugt sind, mit R51, R52 die unabhängig voneinander die Bedeutungen von R21, R22 annehmen können,
   - R4,R6,R7: unabhängig voneinander H, C₁-C₆-Alkyl, CO-R41
   - R41: unabhängig von R21, die selben Bedeutungen wie R21
   - X: O, S, NH, N-R8, wobei R8 unabhängig von R5 die gleichen Bedeutung wie R5 annehmen kann oder R5 und R8 zusammen mit dem N eine 4, 5, 6, 7 oder 8 gliedrigen Ring bilden, der optional noch ein weiteres Herteroatom ausgewählt aus der Gruppe N, O, S enthalten kann,
   oder X-R5 zusammen gleich H,
   Y O, S, NR9, wobei R9 H oder C₁-C₆-Alkyl sein kann,

bedeutet, deren Stereoisomere, Tautomere und deren physiologisch verträglichen Salze oder Einschlussverbindungen, wobei die Reste außer bei Cyclodextrin-Einschlussverbindungen folgende Bedeutung nicht gleichzeitig annehmen dürfen für Formel Ia: R1: H, C₁-C₆-Alkyl, R2: C₁-C₆-Alkyl, C₂-C₆-Alkenyl, R3: H, R4 und R6 identisch und unabhängig voneinander H, C₁-C₆-Alkyl, CO-R41 mit R41 gleich C₁-C₆-Alkyl, Aryl, und R7 H, C₁-C₆-Alkyl, Y: O und für Formel Ib: R1: H, R2: Pentyl, 1-Pentenyl, 3- Pentenyl, 1,3-Pentdienyl, R3: H, R4 und R6 gleich H und X-R5 gleich Methoxy, Y: O. Bevorzugt nehmen die Substituenten nicht gleichzeitig folgende Bedeutung an: R1, R3: H; R2: H, Alkyl, Hydroxyalkyl, insbesondere Monohydroxyalkyl, Alkoxyalkyl, CF₃, (CH₂)ᵣCOOH, CHO, CONH₂, (CH₂)ᵣCH₂NHCOAlkyl, (CH₂)ᵣCH₂OCOAlkyl, (CH₂)ᵣCH₂NHCSAlkyl, CH=NOH, CH=NO-Alkyl, Aryl, Alkylaryl, Alkylheteroaryl, Alkenyl, Hydroxyalkenyl, insbesondere Monohydroxyalkenyl, R4, R6, R7: H, Alkyl; X-R5:H, R5: H, Alkyl,

Aryl.

Bevorzugt sind Verbindungen der Formel IIa oder IIb 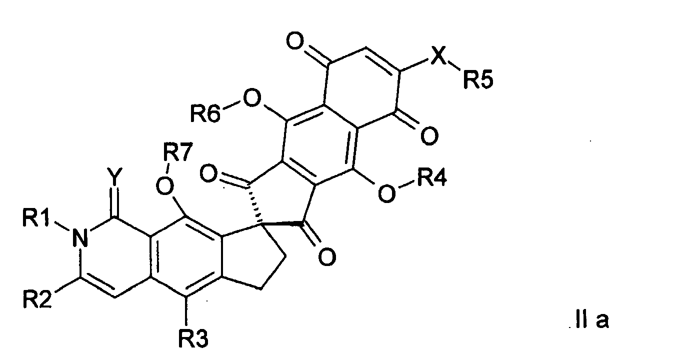 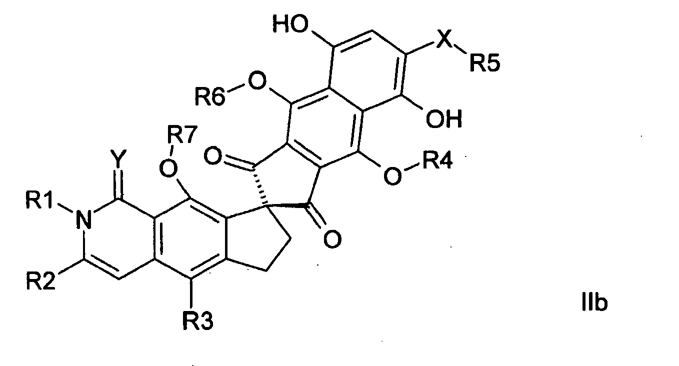 wobei die Bedeutung der Reste R1-R41, X wie oben angegeben ist, deren Tautomere und deren physiologisch verträglichen Salze oder Einschlussverbindungen, wobei die Reste außer bei Cyclodextrin-Einschlussverbindungen folgende Bedeutung nicht gleichzeitig annehmen dürfen für Formel Ia: R1: H, C₁-C₆-Alkyl, R2: C₁-C₆-Alkyl, C₂-C₆-Alkenyl, R3: H, R4 und R6 identisch und unabhängig voneinander H, C₁-C₆-Alkyl, CO-R41 mit R41 gleich C₁-C₆-Alkyl, Aryl, und R7 H, C₁-C₆-Alkyl, Y: O und für Formel Ib: R1: H, R2: Pentyl, 1-Pentenyl, 3-Pentenyl, 1,3-Pentdienyl, R3: H, R4 und R6 gleich H und X-R5 gleich Methoxy, Y: O.

Die Erfindung betrifft außerdem Verbindungen der Formel Ia, Ib, IIa oder IIb, bei denen die Reste R bis auf R2, die oben angegebenen Bedeutungen haben und R2 gegenüber R2 gleich CH=CH-CH=CH-CH₃ die Wasserlöslichkeit bei Beibehaltung aller anderer Reste mindestens verdoppelt, bevorzugt mindestens verfünffacht, mehr bevorzugt mindestens verzehnfacht, besonders bevorzugt mindestens verfünfzigfacht, insbesondere verhundertfacht, oder sogar verfünfhundertfacht. Die Erhöhung der Wasserlöslichkeit geschieht z.B. über die Einführung von Gruppen, die vermehrt Wasserstoffbrückenbindungen ausbilden können und/oder polar und/oder ionisch sind. Ein Schlüsselzwischenprodukt sind Verbindungen mit einer Aldehyd Funktion in R2.

Bevorzugt sind für R2 außerdem die Gruppe der Reste CₘH₂ₘ₊ₒ₋ₚYₚ (mit m = 1 bis 6, für o = 1, p = 1 bis 2m+o; für m = 2 bis 6, o = -1, p = 1 bis 2m+o; für m = 4 bis 6, o = -2, p = 1 bis 2m+o; Y = unabhängig von einander ausgewählt aus der Gruppe Halogen, OH, OR21, NH₂, NHR21, NR21R22, SH, SR21), (CH₂)ᵣCH₂NHCOR21, (CH₂)ᵣCH₂OCOR51, CH₂)ᵣCH₂NHCSR21, (CH₂)ᵣCH₂S(O)nR21 mit n = 0,1,2, (CH₂)ᵣCH₂SCOR21, (CH₂)ᵣCH₂OSO₂-R21, (CH₂)ᵣCH(OH)R21, (CH₂)ᵣCOOH, (CH₂)ᵣCOOR21, (CH₂)ᵣCONR21R22. Bevorzugt ist weiterhin die Gruppe der Aldehyd abgeleiteten Reste (CH₂)ᵣCHO, (CH₂)ᵣCH=NOH, -(CH₂)ᵣCH=NOR21, (CH₂)ᵣCH=NOCOR21, (CH₂)ᵣCH=NOCH₂CONR21R22, (CH₂)ᵣCH=NOCH(CH₃)CONR21R22, (CH₂)ᵣCH=NOC (CH₃)₂CONR21R22, (CH₂)ᵣCH=N-NHCO-R23, (CH₂)ᵣCH=N-NHC(O)NH-R23, (CH₂)ᵣCH=N-NHC(S)NH-R23, (CH₂)ᵣCH=N-NHC(NH)NH-R23, (CH₂)ᵣCH-N-NHC(NH)-R23, (CH₂)ᵣCH=N-NHCO-CH₂NHCOR21, (CH₂)ᵣCH=N-O-CH₂NHCOR21, (CH₂)ᵣCH=N-NHCS-R23, (CH₂)ᵣCH=CR24R25 ( trans oder cis ), (CH₂)ᵣCH=NR21, (CH₂)ᵣCH=N-NR21R22, 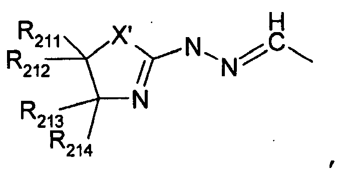 und der (CH₂)ᵣ-kettenverlängerte Rest (CH₂)ᵣCH=N-N-(C₃NX'R211R212R213R214) (mit X' = NR215, O, S und R211, R212, R213, R214, R215 unabhängig voneinander H oder C₁-C₆-Alkyl), -(CH₂)ᵣCH=N-NHSO₂-Aryl, (CH₂)ᵣCH=N-NHSO₂-Heteroaryl, (CH₂)ᵣCH=CH-Heteroaryl, mit r = 0,1,2,3,4,5, bevorzugt 0.

Bevorzugt sind bei den Aldehyden und den abgeleiteten Verbindungen, solche bei denen zumindest R1 oder R3 ungleich H ist, wenn R4 bis R7 H oder Alkyl sind.

Bevorzugte Reste bei R2 sind außerdem Heteroaryl, Cycloalkyl, C₁-C₄-Alkyl-Cycloalkyl, Heterocycloalkyl, C₁-C₄-Alkyl-Heterocycloalkyl, CₘH₂ₘ₊ₒ₋ₚYₚ (mit m = 1 bis 6, für o = 1, p = 1 bis 2m+o; für m = 2 bis 6, o = -1, p = 1 bis 2m+o; für m = 4 bis 6, o = -2, p = 1 bis 2m+o; Y = unabhängig von einander ausgewählt aus der Gruppe Halogen, OH, OR21, NH₂, NHR21, NR21R22, SH, SR21), CH₂NHCOR21, CH₂NHCSR21, CH₂S(O)nR21 mit n=0,1,2, CH₂SCOR21, CH₂OSO₂-R21, CH(OH)R21, CH=NOCOR21, -CH=NOCH₂CONR21R22, -CH=NOCH(CH₃)CONR21R22, CH=NOC(CH₃)₂CONR21R22, CH=N-NHCO-R23, -CH=N-NHCO-CH₂NHCOR21, -CH=N-O-CH₂NHCOR21, -CH=N-NHCS-R23, -CH=CR24R25 ( trans oder cis ), CONR21R22, -CH=NR21, -CH=N-NR21R22, 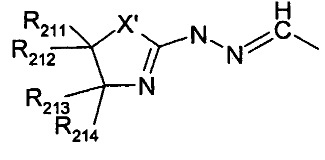 (mit X' = NR215, O, S und R211, R212, R213, R214, R215 unabhängig voneinander H oder C₁-C₆-Alkyl), -CH=N-NHSO₂-Aryl, -CH=N-NHSO₂-Heteroaryl. Bevorzugt sind außerdem Verbindungen wie oben angegeben bei denen R3 F, Cl, Br, I, OH, OR31, NO₂, NH₂, NHR31, NR31R32, NHCHO, NHCOR31, NHCOCF₃, CH₃₋ₘHalₘ (mit Hal = Cl, F, insbesondere F, und m = 1, 2, 3), OCOR31, mit den oben angegebenen Bedeutungen für R31, R32 bedeutet.

Weiterhin bevorzugt sind Verbindungen wie oben angegeben bei denen X N oder S bedeutet, insbesondere wenn R3 gleich H oder Halogen ist und/oder R2 gleich Alkenyl, insbesondere Butadienyl oder 1,3-Pentdienyl ist.

Außerdem bevorzugt sind Verbindungen wie oben angegeben bei denen X-R5 gleich OH ist und insbesondere deren Salze und bvorzugt bei Verbindungen der Formel Ia oder IIa, da diese saure OH-Gruppe leicht deprotoniert werden kann und damit die Wasserlöslichkeit und/oder biologische Wirksamkeit verbessert werden kann.

Bevorzugt sind weiterhin Verbindungen wie oben angegeben, wobei die Reste R bevorzugt unabhängig voneinander eine oder mehrere der folgenden Bedeutungen annehmen:
- R1: H, C₁-C₅-Alkyl, Cycloalkyl, insbesondere H,
- R2: C₁-C₅-Alkyl, C₁-C₄-Alkyl-Aryl, C₂-C₅-Alkenyl, Heteroaryl, C₁-C₄-Alkyl-Heteroaryl, C₂-C₄-Alkenyl-Heteroaryl, CHF₂, CF₃, Polyolseitenkette insbesondere CHOH-CHOH-CHOH-CHOH-CH₃, CHOH-CHOH-CH=CH-CH₃, CH=CH-CHOH-CHOH-CH₃, CH₂Y (Y=F,Cl,Br,I),), CH₂NH₂, CH₂NR21R22, CH₂NHCOR23, CH₂NHCSR23, CH₂SH, CH₂S(O)nR21 mit n=0,1,2, CH₂SCOR21, insbesondere CH₂OH, CH₂OR21, CH₂OSO₂-R21, insbesondere CHO, CH(OR21)₂, CH(SR21)₂, CN, CH=NOH, CH=NOR21, CH=NOCOR21, CH=N-NHCO-R23, CH=CR24,R25 ( trans oder cis ), insbesondere COOH (insbesondere deren physiologisch verträglichen Salze), COOR21, CONR21R22,-CH=NR21, -CH=N-NR21R22, 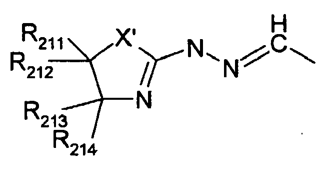 (mit X' = NR215, O, S und R211, R212, R213, R214, R215 unabhängig voneinander H oder C₁-C₆-Alkyl), -CH=N-NHSO₂-Aryl, -CH=N-NHSO₂-Heteroaryl, CH=N-NHCO-R23,
- R21, R22: unabhängig voneinander C₁-C₆-Alkyl, Cycloalkyl, Aryl, C₁-C₄-Alkyl-Aryl, Heteroaryl, C₁-C₄-Alkyl-Heteroaryl
- R23: unabhängig von R21, die selben Bedeutungen wie R21 oder CH₂pyridinium-salze, CH2tri-C₁-C₆-alkylammonmium-salze,
- R24: unabhängig von R21, die selben Bedeutungen wie R21 oder H, CN, COCH₃, COOH, COOR21, CONR21R22, NH₂, NHCOR21
- R25: unabhängig von R21, die selben Bedeutungen wie R21 oder H, CN, COCH₃, COOH, COOR21, CONR21R22, NH₂, NHCOR21
- R24,R25: zusammen C₄-C8-Cycloalkyl,
- R3: F, Cl, Br, I, NO₂, NH₂, NHCOR31,
- R31: unabhängig voneinander C₁-C₆-Alkyl,
- R5: H, C₁-C₆-Alkyl, insbesondere C₁-C₃-Alkyl, C₃-C₈-Cycloalkyl, C₃-C₈-Cycloalkenyl, C₁-C₆-Alkenyl, C₁-C₆-Alkinyle, C₁-C₄-Alkyl-Cycloalkyl, Heterocycloalkyl, C₁-C₄-Alkyl-Heterocycloalkyl, Aryl, C₁-C₄-Alkyl-Aryl, Heteroaryl, C₁-C₄-Alkyl-Heteroaryl, CₘH₂ₘ₊ₒ₋ₚYₚ (mit m = 1 bis 4, für o = 1, p = 1 bis 2m+o; für m = 2 bis 4, o = -1, p = 1 bis 2m+o; für m = 4, o = -2, p = 1 bis 2m+o; Y = unabhängig von einander ausgewählt aus der Gruppe Halogen, OH, OR21, NH₂, NHR21, NR21R22, SH, SR21), besonders bevorzugt ist Hydroxyalkyl mit einer oder mehreren OH Gruppen,
- R4,R6,R7: unabhängig voneinander H, C₁-C₅-Alkyl, CO-R41
- R41: unabhängig von R21, die selben Bedeutungen wie R21
- X: O, S, NH, N-R8
- Y: O,S,NH

bedeutet, deren Stereoisomere, Tautomere und deren physiologisch verträglichen Salze oder Einschlussverbindungen, wobei die Reste außer bei Cyclodextrin-Einschlussverbindungen folgende Bedeutung nicht gleichzeitig annehmen dürfen für Formel Ia: R1: H, C₁-C₆-Alkyl, R2: C₁-C₆-Alkyl, C₂-C₆-Alkenyl, R3: H, R4 und R6 identisch und unabhängig voneinander H, C₁-C₆-Alkyl, CO-R41 mit R41 gleich C₁-C₆-Alkyl, Aryl, und R7 H, C₁-C₆-Alkyl und für Formel Ib: R1: H, R2:
Pentyl, 1-Pentenyl, 3- Pentenyl, 1,3-Pentdienyl, R3: H, R4 und R6 gleich H und X-R5 gleich Methoxy.
Bevorzugt ist für Y O,S, insbesondere O
Bevorzugt für X ist O, NH, N-R8.
Bevorzugt für R5 ist H, Methyl, Ethyl, Propyl, insbesondere Methyl.
Bevorzugt für R8 ist H, Methyl, Ethyl, Propyl, insbesondere Methyl.
Bevorzugt für XR5 ist OCH₃, NH₂, N(CH₃)₂.
Außerdem bevorzugt für R2 ist der Rest -CHOHCHOHCHOHCHOHCH3.
Deweiteren sind für R2 folgende Reste bevorzugt: -CHCH-2-Methyl-4-
Thiazyl, insbesondere 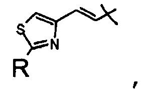 wobei R insbesonder Alkyl oder NHCOAlkyl ist, CH=NOR21, mit R21 gleich Methyl, Ethyl, n-Propyl, Isopropyl, n-Butyl, n-Hexyl, Benzyl, Halogenbenzyl, insbesondere Fluorbenzyl und Chlorbenzyl, -CH₂CH₂-Morpholinyl.

Ganz besonders bevorzugt sind die Verbindungen, deren Stereoisomere, Tautomere und deren physiologisch verträglichen Salze oder Einschlussverbindungen, ausgewählt aus der Gruppe bestehend aus den Verbindungen der Beispiele und den Verbindungen, die Kombinationen der verschiedenen Substituenten der Verbindungen der Beispiele aufweisen.

Besonders bevorzugt sind R3 gleich H, F, Cl, Br, J, insbesondere F, Cl, Br, J.

Besonders bevorzugt für R2 ist C1-C8-Alkyl, C2-C8-Alkenyl, CH=NOR21, mit R21 gleich C1-C8-Alkyl, C1-C8-Alkenyl, Aryl oder Heteroaryl C1-C2-Alkyl-Aryl, insbesondere Benzyl, C1-C2-Alkyl-Heteroaryl, wobei Aryl oder Heteroaryl insbesondere nur eine Ringssystem aufweisen, das gegebenenfalls einfach oder zweifach mit einem Substituenten wie Halogen, Methyl, CF3, OH, OMe substituiert ist.

Insbesondere bevorzugt sind Fredericamycin A Derivate, bei denen nur die oben angegebenen besonders bevorzugten Bedeutungen von R2 und/oder R3 verwirklicht sind.

Die Erfindung betrifft außerdem Arzneimittel enthaltend obige Verbindungen der Formel I oder II neben den üblichen Träger und

Hilfsstoffen.

Bevorzugt sind auch die oben genannten Arzneimittel in Kombination mit weitere Wirkstoffen zur Tumorbehandlung.

Diese erfindungsgemäßen Verbindungen werden zur Herstellung von Arzneimitteln zur Behandlung von Tumoren, insbesondere von solchen, die durch die Inhibierung der Topoisomerasen I und/oder II behandelt werden können, verwendet. Tumoren, die mit den erfindungsgemäßen Substanzen behandelt werden können sind z.B. Leukemie, Lungenkrebs, Melanome, Uterustumore, Prostatatumore und Colontumore.

Außerdem wirkt Fredericamycin A und seine Derivate gegen ein unbekanntes Tearget im Zellzyklus, welches bei Tumorzellen zur Apoptose führt.

Des weiteren werden die erfindungsgemäßen Verbindungen und

Verbindungen bei denen folgende Bedeutungen gleichzeitig angenommen werden bei Formel Ia: R1: H, C₁-C₆-Alkyl, R2: C₁-C₆-Alkyl, C₂-C₆-Alkenyl, R3: H, R4 und R6 identisch und unabhängig voneinander H, C₁-C₆-Alkyl, CO-R41 mit R41 gleich C₁-C₆-Alkyl, Aryl, und R7 H, C₁-C₆-Alkyl und bei Formel Ib: R1: H, R2: Pentyl, 1-Pentenyl, 3- Pentenyl, 1,3-Pentdienyl, R3: H, R4 und R6 gleich H und X-R5 gleich Methoxy zur Herstellung von Arzneimitteln zur Behandlung von Neurodermitis, Parasiten und zur Immunsuppression verwendet.

Außerdem betrifft die Erfindung ein Verfahren zur Herstellung von Fredericamycinderivaten, bei dem intermediär R2 -CHOHCHOHCHOHCHOHCH3 bedeutet. Diese Verbindunegn werden bevorzugt zur weiteren Derivatisierung in Aldehyde umgewandelt.

In der Beschreibung und den Ansprüchen gelten für die einzelnen Substituenten folgende Definitionen:

Der Term "Alkyl" für sich oder als Teil eines anderen Substituenten bedeutet ein lineares oder verzweigtes Alkylketten-Radikal der jeweils angegebenen Länge und optional eine CH₂-Gruppe durch eine Carbonylfunktion ersetzt sein kann. So bedeutet C₁₋₄-Alkyl z.B. Methyl, Ethyl, 1-Propyl, 2-Propyl, 2-Methyl-2-propyl, 2-Methyl-1-propyl, 1-Butyl, 2-Butyl, C₁₋₆-Alkyl z.B. C₁₋₄-Alkyl, Pentyl, 1-Pentyl, 2-Pentyl, 3-Pentyl, 1-Hexyl, 2-Hexyl, 3-Hexyl, 4-Methyl-1-pentyl oder 3,3-Dimethyl-butyl.

Der Term "C₁-C₆-Alkylhydroxy" für sich oder als Teil eines anderen Substituenten bedeutet ein lineares oder verzweigtes Alkylketten-Radikal der jeweils angegebenen Länge, das gesättigt oder ungesättigt sein kann und eine OH Gruppe trägt, z.B. Hydroxymethyl, Hydroxyethyl, 1-Hydroxypropyl, 2-Hydroxypropyl.

Der Term "Alkenyl" für sich oder als Teil eines anderen Substituenten bedeutet ein lineares oder verzweigtes Alkylketten-Radikal mit eine oder mehreren C=C-Doppelbindungen der jeweils angegebenen Länge, wobei mehrere Doppelbindungen bevorzugt konjugiert sind. So bedeutet C₂₋₆-Alkenyl z.B. Ethenyl, 1-Propenyl, 2-Propenyl, 2-Methyl-2-propenyl, 2-Methyl-1-propenyl, 1-Butenyl, 2-Butenyl, 1,3-Butdienyl, 2,4-Butdienyl, 1-Pentenyl, 2-Pentenyl, 3-Pentenyl, 1,3-Pentdienyl, 2,4-Pentdienyl, 1,4-Pentdienyl, 1-Hexenyl, 2-Hexenyl, 1,3-Hediexyl, 4-Methyl-1-pentenyl oder 3,3-Dimethylbutenyl.

Der Term "Alkinyl" für sich oder als Teil eines anderen Substituenten bedeutet ein lineares oder verzweigtes Alkylketten-Radikal mit eine oder mehreren C-C-Dreifachbindungen der jeweils angegebenen Länge. So bedeutet C₂₋₆-Alkinyl z.B. Ethinyl, 1-Propinyl, 2-Propinyl, 2-Methyl-2-propinyl, 2-Methyl-1-propinyl, 1-Butinyl, 2-Butinyl, 1,3-Butdiinyl, 2,4-Butdiinyl, 1-Pentinyl, 2-Pentinyl, 3-Pentinyl, 1-Hexinyl, 2-Hexinyl, 4-Methyl-1-pentinyl oder 3,3-Dimethyl-butinyl.

Der Term "Halogen" steht für Fluor, Chlor, Brom, Jod, bevorzugt Brom und Chlor.

Der Term "NR21R22" steht bevorzugt für eine Dialkylaminogruppe, wobei die beiden Alkylgruppen zusammen mit dem N auch einen 5- oder 6-gliedrigen Ring mit optional einem weiteren Heteroatom N oder O, bilden können.

Der Term "Cycloalkyl" für sich oder als Teil eines anderen Substituenten beinhaltet ungesättigte (einfach oder mehrfach, bevorzugt einfach) oder gesättigte, cyclische Kohlenwasserstoffgruppen, mit 3 bis 10 C-Atomen, bevorzugt 3 bis 8 C-Atomen, wie z.B. Cyclopropyl, Cyclobutyl, Cyclopentyl, Cyclohexyl, Cyclohex-2-enyl, Cyclohex-3-enyl, Cyclohex-2,4-dienyl, 4-Methyl-cyclohexyl, 3-Methyl-cyclohexyl, Cycloheptyl oder Cyclooctyl. Gesättigte Cycloalkyle sind bevorzugt. Die Cycloalkyle können substituiert sein mit bis zu 3 Substituenten, bevorzugt bis zu 1 Substituenten, wobei die Substituenten unabhängig voneinander die Bedeutung C₁-C₆-Alkyl, OH, NO₂, CN, CF₃, OR11, SH, SR11, C₁-C₆-Alkylhydroxy, C₁-C₆-Alkyl-OR11, COOH, COOR11, NH₂, NHR11, NR11R12, Halogen, Aryl, C₁-C₄-Alkylaryl, Heteroaryl, C₁-C₄-Heteroalkylaryl haben können, wobei die Reste R11 und R12 unabhängig von einander C₁-C₁₀-Alkyl, Cycloalkyl, C₁-C₄-AlkylCycloalkyl bedeuten können.

Der Term "Heterocycloalkyl" für sich oder als Teil eines anderen Substituenten beinhaltet Cycloalkylgruppen worin bis zu zwei CH₂-Gruppen durch Sauerstoff-, Schwefel- oder Stickstoffatome ersetzt sein können und eine oder zwei weitere CH₂-gruppe durch eine oder zwei Carbonylfunktion(en), Carbothionylfunktion(en) oder eine Carbonylfunktion und eine Carbothionylfunktion ersetzt sein kann, z.B. Pyrrolidin, Piperidin, Morpholin oder

| | | |
|---|---|---|
| 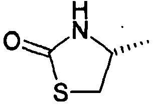 | 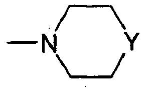 Y=CH2, S, O, NH, NC1-C6-Alkyl | 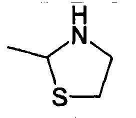 |

Die Heterocycloalkyle können wie die Cycloalkyle substituiert sein. Der Term "Aryl" für sich oder als Teil eines anderen Substituenten beinhaltet aromatische Ringsysteme mit bis zu 3 Ringen, bei denen mindestens 1 Ringsystem aromatisch ist und die mit bis zu 3 Substituenten, bevorzugt bis zu 1 Substituenten, wobei die Substituenten unabhängig voneinander die Bedeutung C₁-C₆-Alkyl, OH, NO₂, CN, CF₃, OR11, SH, SR11, C₁-C₆-Alkylhydroxy, C₁-C₆-Alkyl-OR11, COOH, COOR11, NH₂, NHR11, NR11R12, Halogen haben können, wobei die

Reste R11 und R12 unabhängig von einander C₁-C₁₀-Alkyl, Cycloalkyl, C₁-C₄-Alkyl-Cycloalkyl oder R11 und R12 zusammen mit dem N eine 4, 5, 6, 7 oder 8 gliedrigen Ring bilden, der optional noch ein weiteres Herteroatom ausgewählt aus der Gruppe N, O, S enthalten kann, bedeuten können.

Bevorzugte Aryle sind neben Phenyl und 1-Naphtyl und 2-Naphtyl:

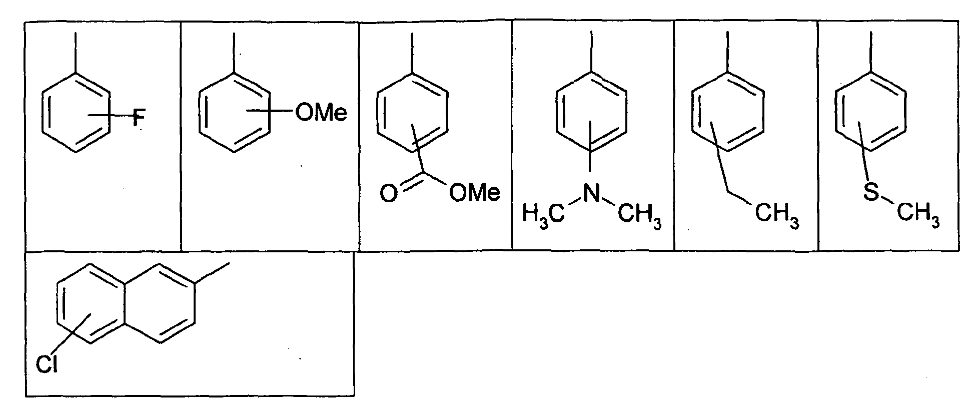

Der Term "Heteroaryl" für sich oder als Teil eines anderen Substituenten beinhaltet aromatische Ringsysteme mit bis zu 3 Ringen, und bis zu 3 gleichen oder verschiedenen Heteroatomen N, S, O bei denen mindestens 1 Ringsystem aromatisch ist und die mit bis zu 3 Substituenten, bevorzugt bis zu 1 Substituenten, wobei die Substituenten unabhängig voneinander die Bedeutung C₁-C₆-Alkyl, OH, NO_{2,} CN, CF₃, OR11, SH, SR11, C₁-C₆-Alkylhydroxy, C₁-C₆-Alkyl-OR11, COOH, COOR11, NH₂, NHCOR11, NHR11, NR11R12, Halogen oder Phenyl, haben können, wobei die Reste R11 und R12 unabhängig von einander die oben angegebenen Bedeutungen haben können.

Bevorzugte Heteroaryle sind:

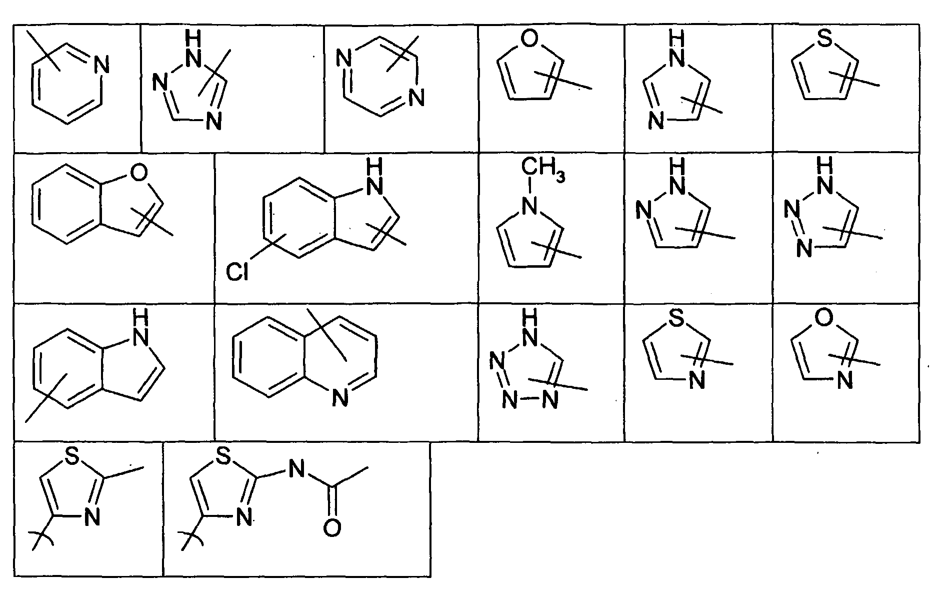

Der Term "Ringsystem" bezieht sich im Allgemeinen auf 3, 4, 5, 6, 7, 8, 9 oder 10 gliedrige Ringe. Bevorzugt sind 5 und 6 gliedrige Ringe. Des weiteren sind Ringsysteme mit einem oder 2 anellierten Ringen bevorzugt.

Die Verbindungen der Formel I können als solche oder falls sie acidische oder basische Gruppen aufweisen in Form ihrer Salze mit physiologisch verträglichen Basen oder Säuren vorliegen. Beispiele für solche Säuren sind: Salzsäure, Zitronensäure, Trifluoressigsäure, Weinsäure, Milchsäure, Phosphorsäure, Methansulfonsäure, Essigsäure, Ameisensäure, Maleinsäure, Fumarsäure, Bernsteinsäure, Hydroxybernsteinsäure, Schwefelsäure, Glutarsäure, Asparaginsäure, Brenztraubensäure, Benzoesäure, Glucuronsäure, Oxalsäure, Ascorbinsäure und Acetylglycin. Beispiele für Basen sind Alkaliionen, bevorzugt Na, K, besonders bevorzugt das tri Kalium und tri Natriumsalz, Erdalkaliionen, bevorzugt Ca, Mg, Ammoniumionen.

Die erfindungsgemäßen Verbindungen können in üblicher Weise oral verabfolgt werden. Die Applikation kann auch i.v., i.m., mit Dämpfen oder Sprays durch den Nasen-Rachenraum erfolgen.

Die Dosierung hängt vom Alter, Zustand und Gewicht des Patienten sowie von der Applikationsart ab. In der Regel beträgt die tägliche Wirkstoffdosis pro Person zwischen etwa 0.1 µg/kg und 1 g/Kg bei oraler Gabe. Diese Dosis kann in 2 bis 4 Einzeldosen oder einmalig am Tag als Slow-release-Form gegeben werden.

Die neuen Verbindungen können in den gebräuchlichen galenischen Applikationsformen fest oder flüssig angewendet werden, z.B. als Tabletten, Filmtabletten, Kapseln, Pulver, Granulate, Dragees, Lösungen, oder Sprays. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln, Antioxidantien und/oder Treibgasen verarbeitet werden (vgl. H. Sucker et al.: Pharmazeutische Technologie, Thieme-Verlag, Stuttgart, 1978). Die so erhaltenen Applikationsformen enthalten den Wirkstoff normalerweise in einer Menge von 0,1 bis 99 Gew.-%.

### Experimenteller Teil

Fredericamycin A ist fermentativ oder totalsynthetisch nach den bekannten Methoden zugänglich. Die reduzierten Formen der Formel I b und II b lassen sich durch milde Reduktionsmittel aus den entsprechenden Verbindungen der Formel I a und II a herstellen. Herstellung der Substanzen

Für die Synthese von wasserlöslichen Fredericamycin Derivaten wurde Fredericamycin **(1)** zunächst mit Osmium(IV)oxid an der Dienseitenkette hydroxyliert. Die entstehende Verbindung **(2)** zeigte ein erheblich größere wasserlöslichkeit als die Ausgangsverbindung Fredericamycin (**1). (2)** wurde zur weiteren Steigerung der Wasserlöslichkeit in das Trikaliumsalz **(3)** umgewandelt ( s. Schema 1 ). 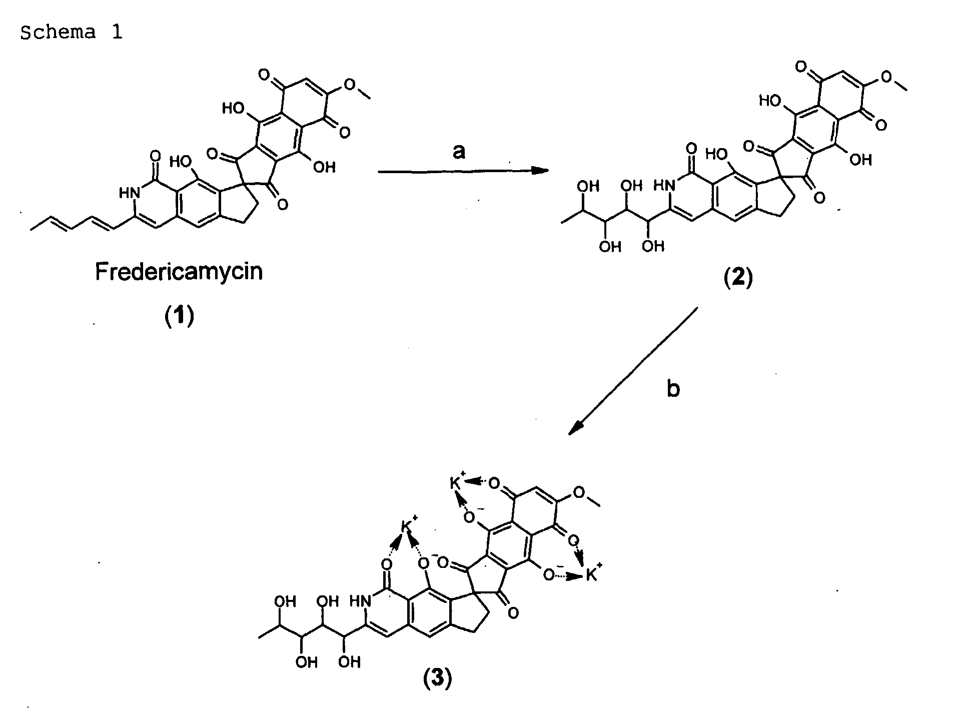
a) OSO₄, N-Methylmorpholin-N-oxid, CH₂Cl₂, CH₃OH, H₂O
b) KOH-Pyridin
Das Fredericamycin-tetrol **(2)** dient unter anderem als wichtige Zwischenstufe für die Synthese weiterer Fredericamycin Derivate mit erhöhtem Löslichkeit und/oder Wirkprofil. Durch Jodatspaltung mit Natriummetaperjodat bzw. trägergebundenem Perjodat läßt sich die Tetrolseitenkette in sehr hohen Ausbeuten zum Fredericamycin-aldehyd **(4)** abbauen (s. Schema 2). 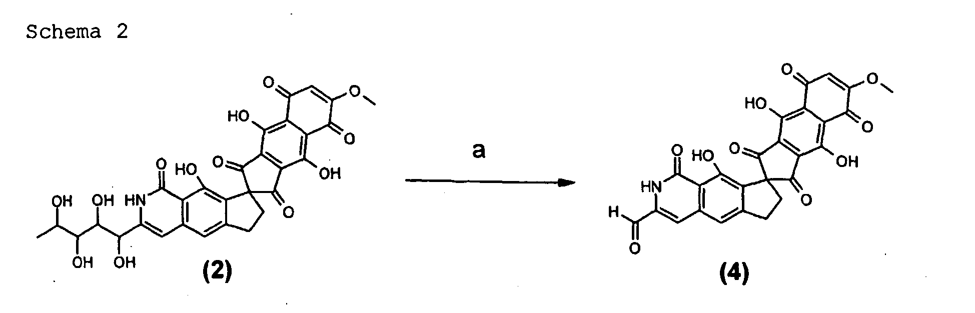
a) NaIO₄-H₂O-DMF oder trägergebundenes-IO₄-H₂O-DMF
Der Fredericamycin-Aldehyd **(4)** läßt sich mit Acylhydrazonen, Hydroxylamin und O-Alkylhydroxylamine in die entsprechenden Hydrazone ( s. Schema 3 ), bzw. Oxim und Oximether ( s. Schema 4 ) umsetzen. Die Reaktion kann bei Raumtemperatur in Lösungsmittel wie DMF oder Pyridin durchgeführt werden und ist nach wenigen Minuten bis Stunden beendet.

### Synthese von Hydrazonen

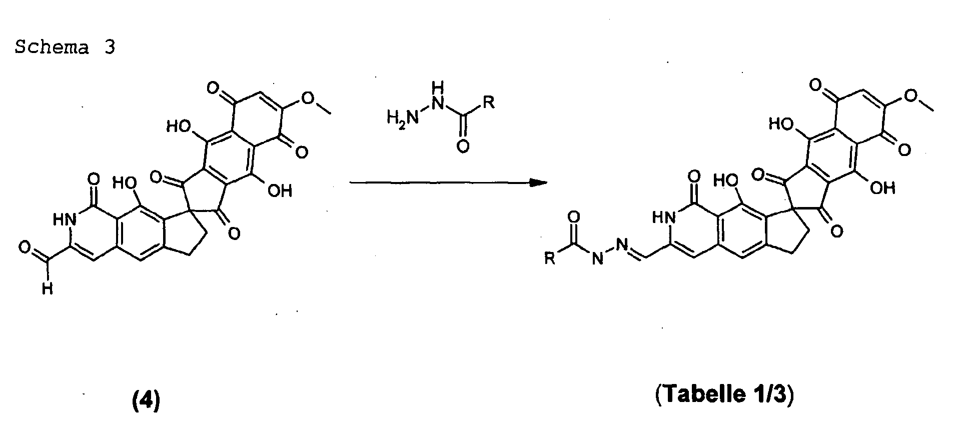

**Tabelle 1**

| Beispiel/Verbindung | R | **m/e** | λ**ₘₐₓ(nm)** |
|---|---|---|---|
| **5/118** | 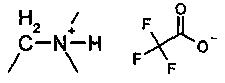 | 601.3 | 504.0 |
| **6/119** | 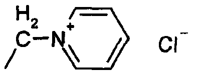 | 635,2 | 486.0 |

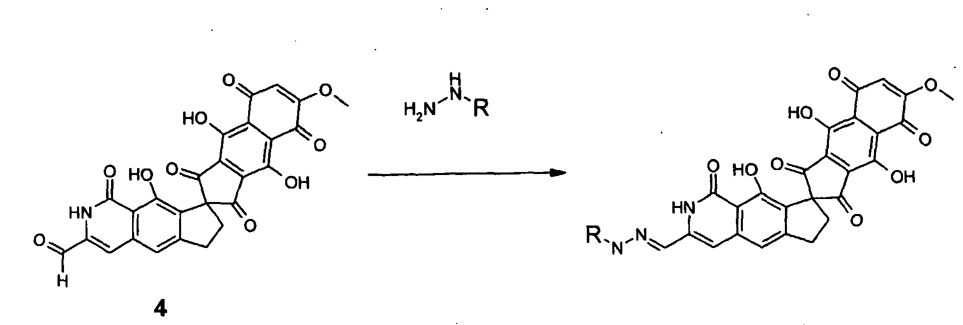

| R | Verbindung | Beispiel |
|---|---|---|
| 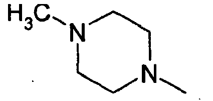 | **111** | **18** |
| 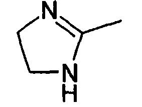 | **105** | **19** |
| 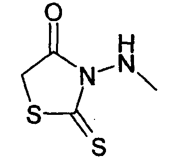 | **113** | **20** |

### Synthese von Oximether

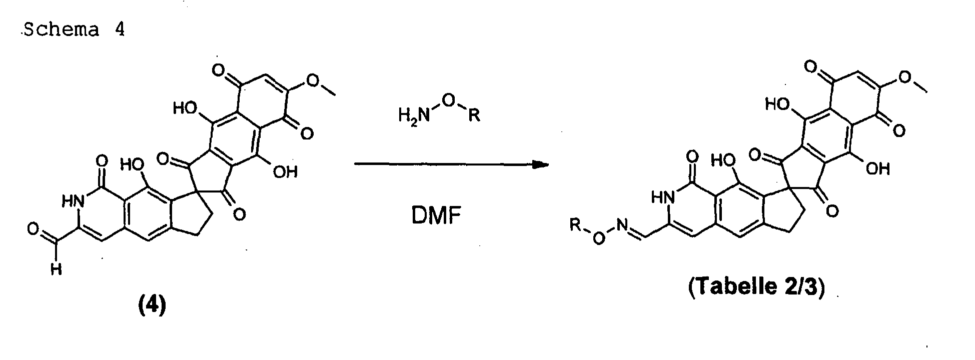

**Tabelle 2**

| **Beispiel/Verbindung** | **R** | **m/e** | λ**ₘₐₓ(nm)** | | |
|---|---|---|---|---|---|
| **7/122** | -H | 516.1 | 500.0 | | |
| **8/120** | -CH₃ | 531.2 | 500.0 | | |
| **9/121** | 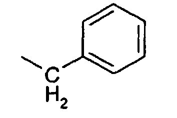 | 607.2 | 504.0 | | |
| **10/123** | 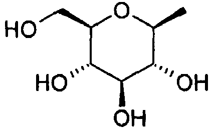 | 678.1 | 504.0 | | |
| **21/116** | 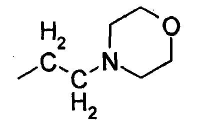 | 630.1 | 504.0 | | |

Analog können nach der unten aufgeführten Vorschrift die Verbindungen 100 - 242 hergestellt werden ( Tabelle 3 ). Die hierbei verwendeten Hydrazine, Hydrazone und Hydroxylamine sind kommerziell erhältlich oder wurden nach literaturbekannter Vorschrift hergestellt.

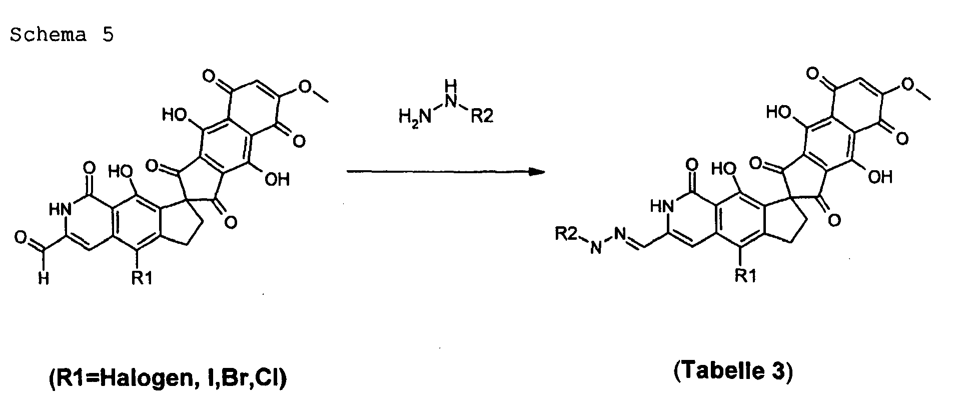

**Tabelle 3**

| Formel für Tabelle 3: | | | | | | |
|---|---|---|---|---|---|---|
| 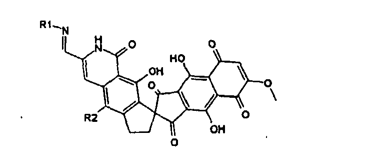 | | | | | | |

| **Beispiel/Verbindung** | **R1** | **R2** | **Masse berechnet** | **Masse gefunden** | **UVMax** | **Ausbeute** |
|---|---|---|---|---|---|---|
| 100 | 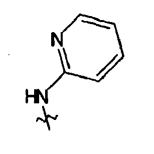 | 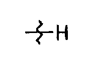 | | | | |
| | C5H5N2 | H | 592,1230 | 593,10 | 500 | 95 |
| 101 | 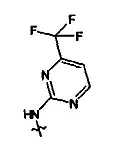 | 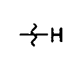 | | | | |
| | C5H3F3N3 | H | 661,1056 | 662,11 | 500 | 95 |
| 102 | 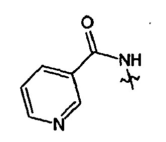 | 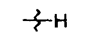 | | | | |
| | C6H5N2O | H | 620,1179 | 621,11 | 492 | 95 |
| 103 | 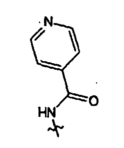 | 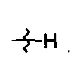 | | | | |
| | C6H5N2O | H | 620,1179 | 621,11 | 500 | 95 |
| 104 | 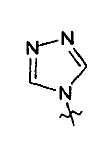 | 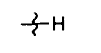 | | | | |
| | C2H2N3 | H | 567,1026 | 568, 11 | 500 | 80 |
| 105 (19) | 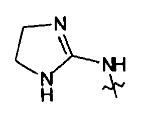 | 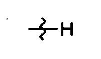 | | | | |
| | C3H6N3 | H | 583,1339 | 584,10 | 492 | 95 |
| 106 | 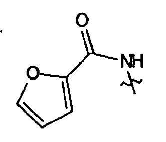 | 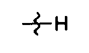 | | | | |
| | C5H4NO2 | H | 609,1019 | 610,09 | 492 | 95 |
| 107 | 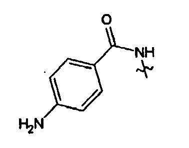 | 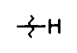 | | | | |
| | C7H7N2O | H | 634,1335 | 635,13 | 492 | 95 |
| 108 | 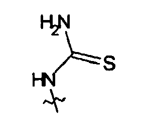 | 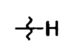 | | | | |
| | NHCSNH2 | H | 574,0794 | 558,05 | 492 | 95 |
| 109 | 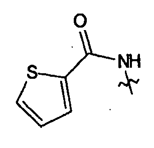 | 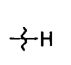 | | | | |
| | C5H4NOS | H | 625,0791 | 626,08 | 492 | 95 |
| 110 | 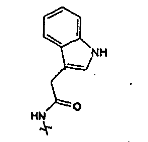 | 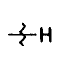 | | | | |
| | C10H9N20 | H | 672,1492 | 673,15 | 492 | 95 |
| 111 | 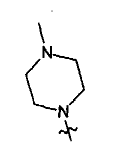 | 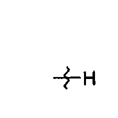 | | | | |
| | C5H11N2 | H | 598,1699 | 599,14 | 492 | 95 |
| 112 | 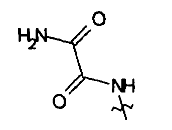 | 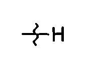 | | | | |
| | C2H3N2O2 | H | 586,0971 | 587,10 | 492 | 95 |
| 113 (20) | 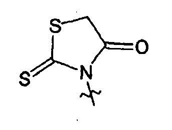 | 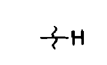 | | | | |
| | C3H2NOS2 | H | 631,0355 | 632,05 | 500 | 95 |
| 114 | 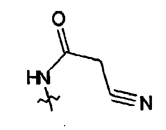 | 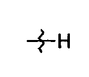 | | | | |
| | C3H3N2O | H | 582,1022 | 583,13 | 492 | 95 |
| 115 | 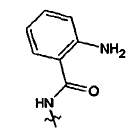 | 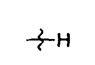 | | | | |
| | C7H7N2O | H | 634,1335 | 635,16 | 492 | 70 |
| 116 | 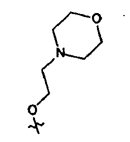 | 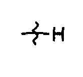 | | | | |
| | C6H12NO2 | H | 629,1645 | 630,14 | 492 | 85 |
| 117 | 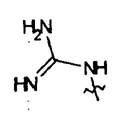 | 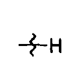 | | | | |
| | CH4N3 | H | 557,1182 | 558,11 | 500 | 95 |
| 118 | 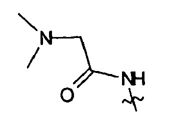 | 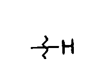 | | | | |
| | C4H9N2O | H | 600,1492 | 601,16 | 492 | 85 |
| 119 | 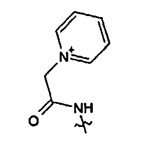 | 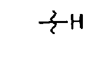 | | | | |
| | C7H8N2O | H | 635,1414 | 635,13 | 492 | 85 |
| 120(8) | 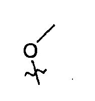 | 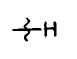 | | | | |
| | OMe | H | 530,0961 | 531,12 | 492 | 90 |
| 121(9) | 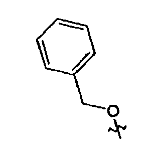 | 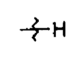 | | | | |
| | OCH2Ph | H | 606,1274 | 607,16 | 492 | 95 |
| 122(7) | 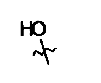 | 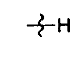 | | | | |
| | OH | H | 516,0804 | 517,11 | 482 | 95 |
| 123 (10) | 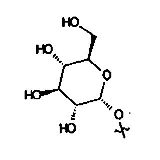 | 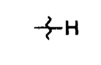 | | | | |
| | C6H11O6 | H | 678,1332 | 679,14 | 500 | 95 |
| 124 | 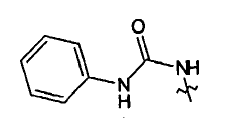 | 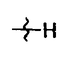 | | | | |
| | C7H7N2O | H | 634,1335 | 635,15 | 492 | 95 |
| 125 | 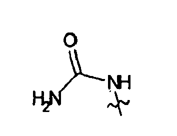 | 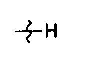 | | | | |
| | NHCONH2 | H | 558,1022 | 559,12 | 492 | 95 |
| 126 | 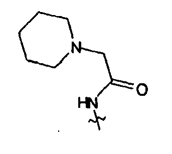 | 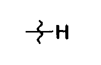 | | | | |
| | C7H13N2O | H | 640,1805 | 641,13 | 492 | 95 |
| 127 | 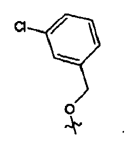 | 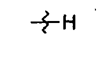 | | | | |
| | C7H6C1O | H | 640,0884 | 641,10 | 492 | 95 |
| 128 | 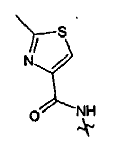 | 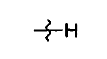 | | | | |
| | C5H5N2OS | H | 640,0900 | 641,10 | 492 | 95 |
| 129 | 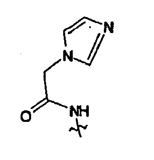 | 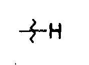 | | | | |
| | C5H6N3O | H | 623,1288 | 624,13 | 500 | 90 |
| 130 | 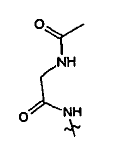 | 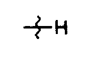 | | | | |
| | C4H7N2O2 | H | 614,1284 | 615,13 | 492 | 95 |
| 131 | 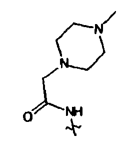 | 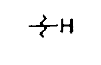 | | | | |
| | C7H14N3O | H | 655,1914 | 656,19 | 492 | 50 |
| 132 | 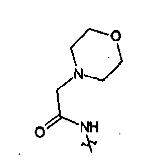 | 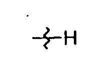 | | | | |
| | C6H11N2O2 | H | 642,1597 | 643,17 | 492 | 60 |
| 133 | 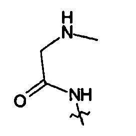 | 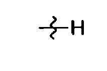 | | | | |
| | C3H7N20 | H | 586,1335 | 587,15 | 492 | 70 |
| 134 | 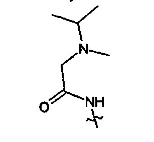 | 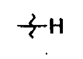 | | | | |
| | C6H13N20 | H | 628,1805 | 629,17 | 492 | 70 |
| 135 | 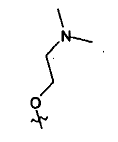 | 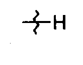 | | | | |
| | C4H10NO | H | 587,1539 | 588,14 | 492 | 90 |
| 136 | 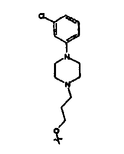 | 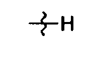 | | | | |
| | C13H18ClN2O | H | 752,1885 | 753,19 | 492 | 85 |
| 137 | 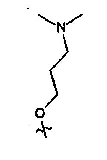 | 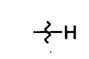 | | | | |
| | C5H12NO | H | 601,1696 | 602,19 | 492 | 70 |
| 138 | 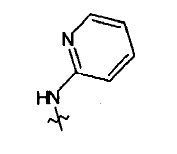 | 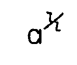 | | | | |
| | C5H5N2 | Cl | 626,0840 | 627,07 | 500 | 95 |
| 139 | 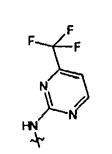 | 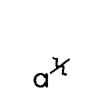 | | | | |
| | C5H3F3N3 | Cl | 695,0666 | 696,06 | 500 | 95 |
| 140 | 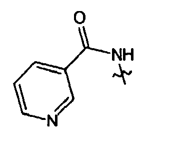 | 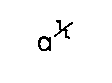 | | | | |
| | C6H5N2O | Cl | 654,0789 | 655,07 | 500 | 95 |
| 141 | 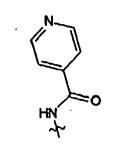 | 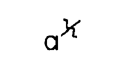 | | | | |
| | C6H5N20 | Cl | 654,0789 | 655,07 | 500 | 95 |
| 142 | 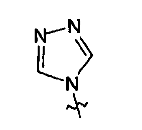 | 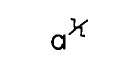 | | | | |
| | C2H2N3 | Cl | 601,0636 | 602,06 | 500 | 90 |
| 143 | 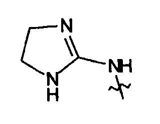 | 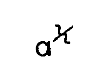 | | | | |
| | C3H6N3 | Cl | 617,0949 | 618,08 | 500 | 95 |
| 144 | 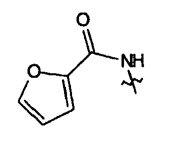 | 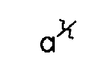 | | | | |
| | C5H4NO2 | Cl | 643,0629 | 644,05 | 500 | 95 |
| 145 | 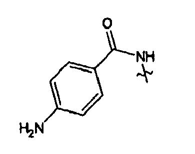 | 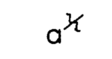 | | | | |
| | C7H7N2O | Cl | 668,0946 | 669,07 | 500 | 95 |
| 146 | 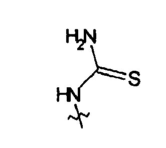 | 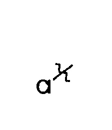 | | | | |
| | NHCSNH2 | Cl | 608,0404 | 609,07 | 500 | 95 |
| 147 | 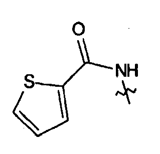 | 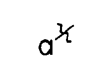 | | | | |
| | C5H4NOS | Cl | 659,0401 | 660,07 | 500 | 95 |
| 148 | 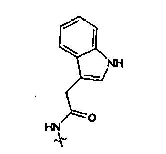 | 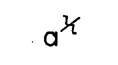 | | | | |
| | C10H9N2O | Cl | 706,1102 | 707,16 | 500 | 95 |
| 149 | 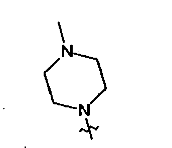 | 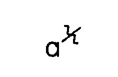 | | | | |
| | C5H11N2 | Cl | 632,1309 | 633,16 | 500 | 95 |
| 150 | 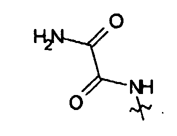 | 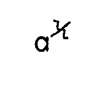 | | | | |
| | C2H3N2O2 | Cl | 620,0582 | 621,09 | 500 | 95 |
| 151 | 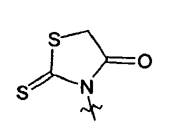 | 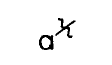 | | | | |
| | C3H2NOS2 | Cl | 664,9965 | 645,31 | 500 | 95 |
| 152 | 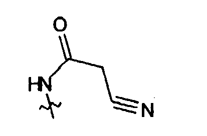 | 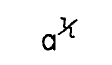 | | | | |
| | C3H3N2O | Cl | 616,0633 | 617,10 | 500 | 95 |
| 153 | 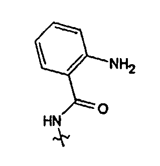 | 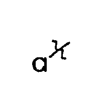 | | | | |
| | C7H7N2O | Cl | 668,0946 | 669,13 | 500 | 95 |
| 154 | 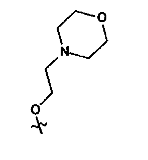 | 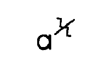 | | | | |
| | C6H12NO2 | Cl | 663,1255 | 664,16 | 500 | 95 |
| 155 | 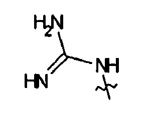 | 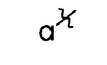 | | | | |
| | CH4N3 | Cl | 591,0792 | 592,11 | 500 | 95 |
| 156 | 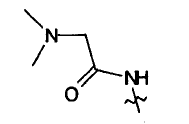 | 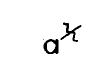 | | | | |
| | C4H9N2O | Cl | 634,1102 | 635,14 | 500 | 95 |
| 157 | 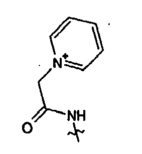 | 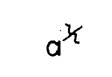 | | | | |
| | C7H8N2O | Cl | 669,1024 | 669,12 | 500 | 95 |
| 158 | 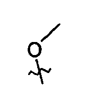 | 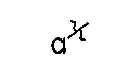 | | | | |
| | OMe | Cl | 564,0571 | 565,09 | 500 | 95 |
| 159 | 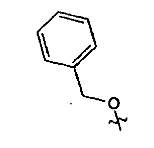 | 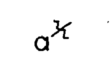 | | | | |
| | OCH2Ph | Cl | 640,0884 | 641,12 | 500 | 95 |
| 160 | 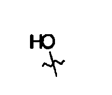 | 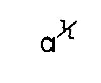 | | | | |
| | OH | Cl | 550,0415 | 551,06 | 500 | 95 |
| 161 | 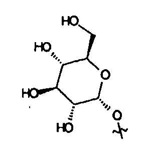 | 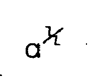 | | | | |
| | C6H11O6 | Cl | 712,0943 | 713,10 | 500 | 95 |
| 162 | 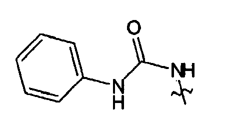 | 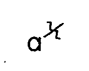 | | | | |
| | C7H7N20 | Cl | 668,0946 | 669,09 | 500 | 95 |
| 163 | 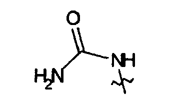 | 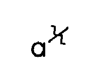 | | | | |
| | NHCONH2 | Cl | 592,0633 | 593,07 | 500 | 90 |
| 164 | 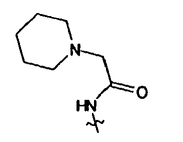 | 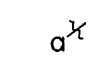 | | | | |
| | C7H13N2O | Cl | 674,1415 | 675,11 | 500 | 95 |
| 165 | 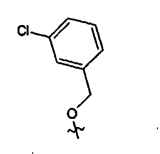 | 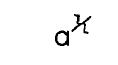 | | | | |
| | C7H6C1O | Cl | 674,0494 | 675,03 | 500 | 90 |
| 166 | 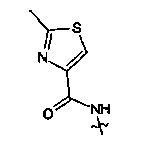 | 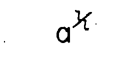 | | | | |
| | C5H5N2OS | Cl | 674,0510 | 675,02 | 500 | 95 |
| 167 | 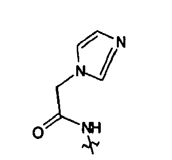 | 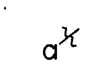 | | | | |
| | C5H6N3O | Cl | 657,0898 | 658,06 | 500 | 90 |
| 168 | 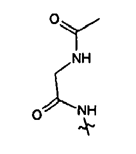 | 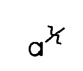 | | | | |
| | C4H7N2O2 | Cl | 648,0895 | 649,07 | 500 | 95 |
| 169 | 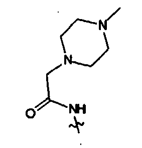 | 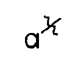 | | | | |
| | C7H14N3O | Cl | 689,1524 | 690,15 | 500 | 60 |
| 170 | 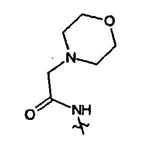 | 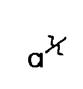 | | | | |
| | C6H11N2O2 | Cl | 676,1208 | 677,13 | 500 | 60 |
| 171 | 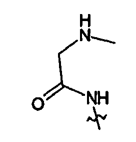 | 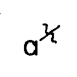 | | | | |
| | C3H7N2O | Cl | 620,0946 | 621,11 | 500 | 70 |
| 172 | 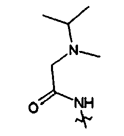 | 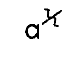 | | | | |
| | C6H13N2O | Cl | 662,1415 | 663,12 | 500 | 70 |
| 173 | 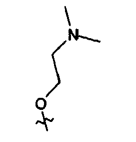 | 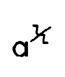 | | | | |
| | C4H10NO | Cl | 621,1150 | 622,10 | 500 | 60 |
| 174 | 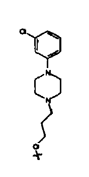 | 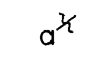 | | | | |
| | C13H18C1N2O | Cl | 786,1495 | 787,16 | 500 | 90 |
| 175 | 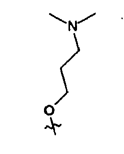 | 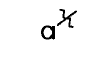 | | | | |
| | C5H12NO | Cl | 635,1306 | 636,10 | 500 | 75 |
| 176 | 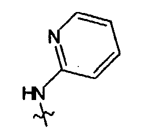 | 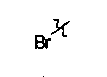 | | | | |
| | C5H5N2 | Br | 670,0334 | 670,99 | 500 | 95 |
| 177 | 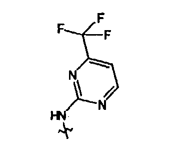 | 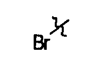 | | | | |
| | C5H3F3N3 | Br | 739,0161 | 739,99 | 500 | 95 |
| 178 | 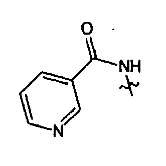 | 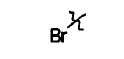 | | | | |
| | C6H5N20 | Br | 698,0284 | 699,00 | 500 | 90 |
| 179 | 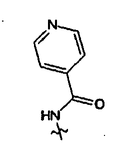 | 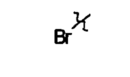 | | | | |
| | C6H5N2O | Br | 698,0284 | 699,00 | 500 | 90 |
| 180 | 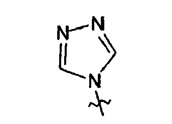 | 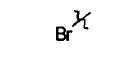 | | | | |
| | C2H2N3 | Br | 645,0130 | 645,99 | 492 | 70 |
| 181 | 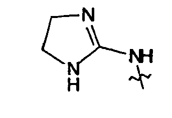 | 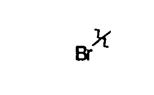 | | | | |
| | C3H6N3 | Br | 661,0443 | 662,01 | 492 | 95 |
| 182 | 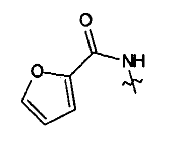 | 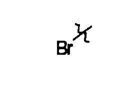 | | | | |
| | C5H4NO2 | Br | 687,0124 | 688,99 | 492 | 95 |
| 183 | 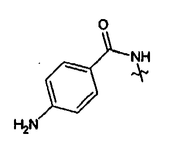 | 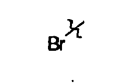 | | | | |
| | C7H7N2O | Br | 712,0440 | 713,03 | 500 | 95 |
| 184 | 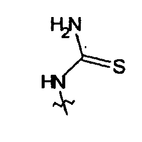 | 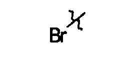 | | | | |
| | NHCSNH2 | Br | 651,9899 | 653,04 | 500 | 95 |
| 185 | 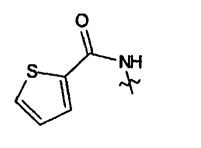 | 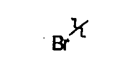 | | | | |
| | C5H4NOS | Br | 702,9895 | 704,02 | 492 | 95 |
| 186 | 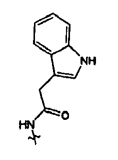 | 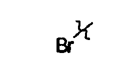 | | | | |
| | C10H9N2O | Br | 750,0597 | 751,10 | 500 | 95 |
| 187 | 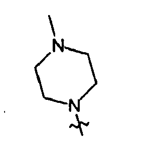 | 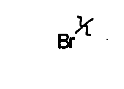 | | | | |
| | C5H11N2 | Br | 676,0804 | 677,10 | 492 | 95 |
| 188 | 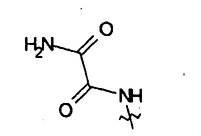 | 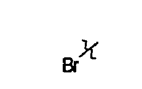 | | | | |
| | C2H3N2O2 | Br | 664,0076 | 665,05 | 500 | 95 |
| 189 | 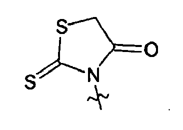 | 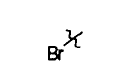 | | | | |
| | C3H2NOS2 | Br | 708,9460 | 709,99 | 492 | 95 |
| 190 | 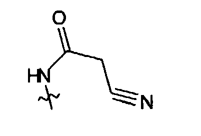 | 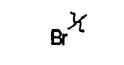 | | | | |
| | C3H3N2O | Br | 660,0127 | 661,05 | 492 | 95 |
| 191 | 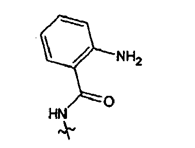 | 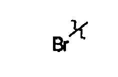 | | | | |
| | C7H7N2O | Br | 712,0440 | 713,08 | 492 | 70 |
| 192 | 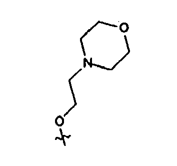 | 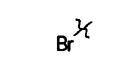 | | | | |
| | C6H12NO2 | Br | 707,0750 | 708,06 | 500 | 95 |
| 193 | 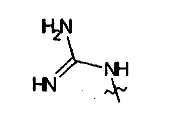 | 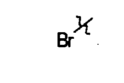 | | | | |
| | CH4N3 | Br | 635,0287 | 636,02 | 500 | 95 |
| 194 | 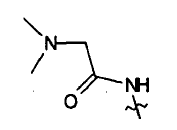 | 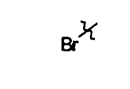 | | | | |
| | C4H9N2O | Br | 678,0597 | 679,06 | 500 | 95 |
| 195 | 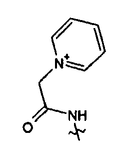 | 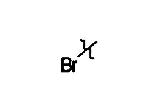 | | | | |
| | C7H8N2O | Br | 713,0518 | 713,03 | 500 | 95 |
| 196 | 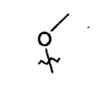 | 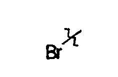 | | | | |
| | OMe | Br | 608,0066 | 609,03 | 492 | 95 |
| 197 | 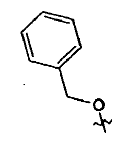 | 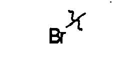 | | | | |
| | OCH2Ph | Br | 684,0379 | 685,05 | 492 | 95 |
| 198 | 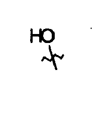 | 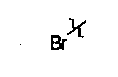 | | | | |
| | OH | Br | 593,9909 | 595,01 | 492 | 95 |
| 199 | 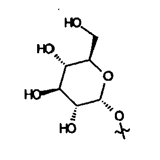 | 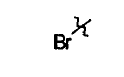 | | | | |
| | C6H11O6 | Br | 756,0437 | 757,00 | 500 | 90 |
| 200 | 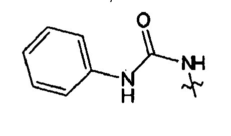 | 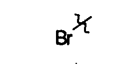 | | | | |
| | C7H7N2O | Br | 712,0440 | 713,00 | 500 | 90 |
| 201 | 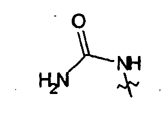 | 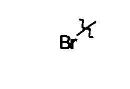 | | | | |
| | NHCONH2 | Br | 636,0127 | 637,00 | 492 | 90 |
| 202 | 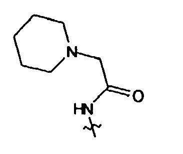 | 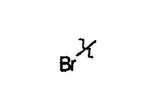 | | | | |
| | C7H13N2O | Br | 718,0910 | 719,00 | 500 | 90 |
| 203 | 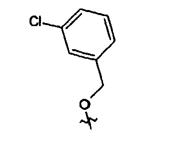 | 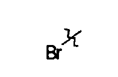 | | | | |
| | C7H6C1O | Br | 717,9989 | 718,00 | 492 | 95 |
| 204 | 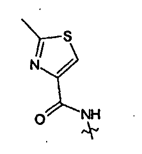 | 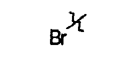 | | | | |
| | C5H5N2OS | Br | 718,0004 | 718,97 | 492 | 95 |
| 205 | 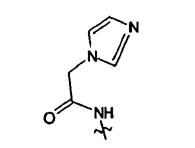 | 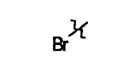 | | | | |
| | C5H6N30 | Br | 701,0392 | 702,01 | 500 | 95 |
| 206 | 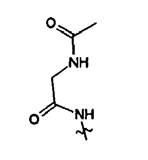 | 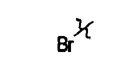 | | | | |
| | C4H7N2O2 | Br | 692,0389 | 693,03 | 492 | 95 |
| 207 | 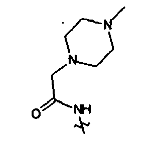 | 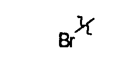 | | | | |
| | C7H14N3O | Br | 733,1018 | 734,10 | 500 | 90 |
| 208 | 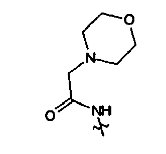 | 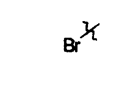 | | | | |
| | C6H11N2O2 | Br | 720,0702 | 721,10 | 500 | 95 |
| 209 | 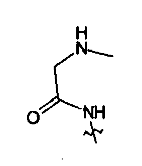 | 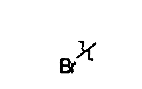 | | | | |
| | C3H7N2O | Br | 664,0440 | 665,08 | 500 | 95 |
| 210 | 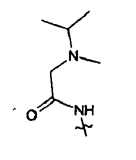 | 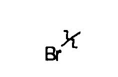 | | | | |
| | C6H13N2O | Br | 706,0910 | 707,09 | 500 | 90 |
| 211 | 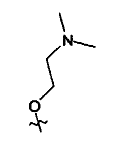 | 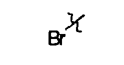 | | | | |
| | C4H10NO | Br | 665,0644 | 666,08 | 500 | 95 |
| 212 | 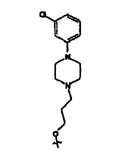 | 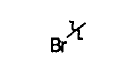 | | | | |
| | C13H18C1N2O | Br | 830,0989 | 831,11 | 500 | 95 |
| 213 | 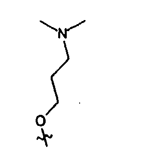 | 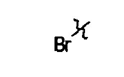 | | | | |
| | C5H12NO | Br | 679,0801 | 680,09 | 492 | 95 |
| 214 | 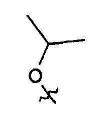 | 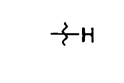 | | | | |
| | Oi-Pr | H | 558,1274 | 559,21 | 500 | 99 |
| 215 | 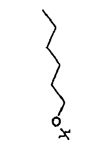 | 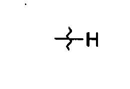 | | | | |
| | O-n-Hex | H | 600,1743 | 601,30 | 500 | 99 |
| 216 | 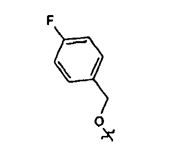 | 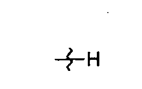 | | | | |
| | C7H6FO | H | 624,1180 | 625,28 | 500 | 99 |
| 217 | 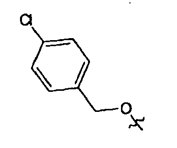 | 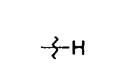 | | | | |
| | C7H6C1O | H | 640,0884 | 641,27 | 500 | 99 |
| 218 | 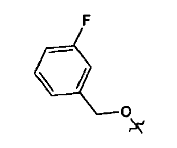 | 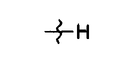 | | | | |
| | C7H6FO | H | 624,1180 | 625,31 | 500 | 99 |
| 219 | 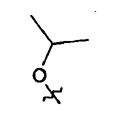 | 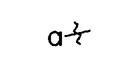 | | | | |
| | Oi-Pr | Cl | 592,0884 | 593,28 | 500 | 80 |
| 220 | 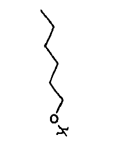 | 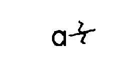 | | | | |
| | O-n-Hex | Cl | 634,1354 | 635,36 | 500 | 90 |
| 221 | 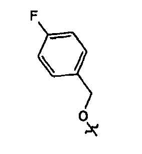 | 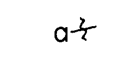 | | | | |
| | C7H6FO | Cl | 658,0790 | 659,32 | 500 | 85 |
| 222 | 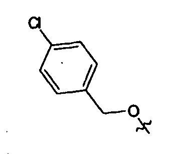 | 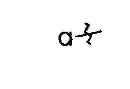 | | | | |
| | C7H6C1O | Cl | 674,0494 | 675,31 | 500 | 80 |
| 223 | 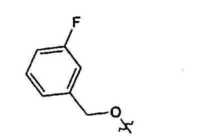 | 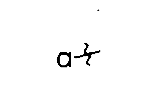 | | | | |
| | C7H6FO | Cl | 658,0790 | 659,34 | 500 | 80 |
| 224 | 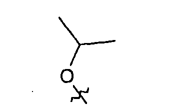 | 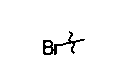 | | | | |
| | Oi-Pr | Br | 636,0379 | 639,30 | 492 | 90 |
| 225 | 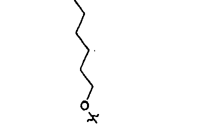 | 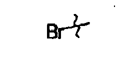 | | | | |
| | O-n-Hex | Br | 678,0848 | 679,37 | 492 | 95 |
| 226 | 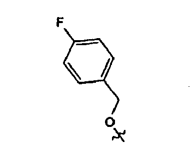 | 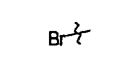 | | | | |
| | C7H6FO | Br | 702,0284 | 703,34 | 492 | 95 |
| 227 | 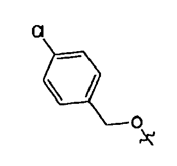 | 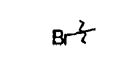 | | | | |
| | C7H6C1O | Br | 717,9989 | 719,34 | 492 | 95 |
| 228 | 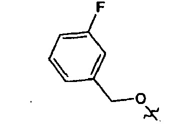 | 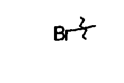 | | | | |
| | C7H6FO | Br | 702,0284 | 705,35 | 492 | 95 |
| 229 | 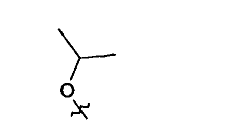 | 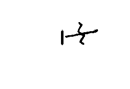 | | | | |
| | Oi-Pr | I | 684,0200 | 685,30 | 500 | 99 |
| 230 | 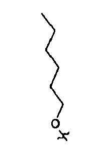 | 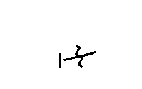 | | | | |
| | O-n-Hex | I | 726,0669 | 727,41 | 500 | 99 |
| 231 | 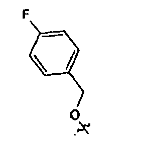 | 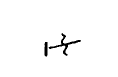 | | | | |
| | C7H6FO | I | 750,0105 | 751,38 | 500 | 99 |
| 232 | 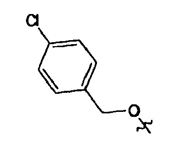 | 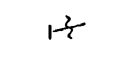 | | | | |
| | C7H6ClO | I | 765,9810 | 767,36 | 500 | 99 |
| 233 | 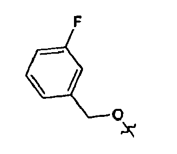 | 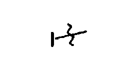 | | | | |
| | C7H6FO | I | 750,0105 | 751,38 | 500 | 99 |
| 234 | 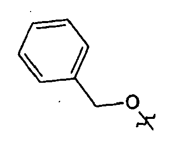 | 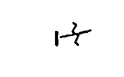 | | | | |
| | OCH2Ph | I | 732,0200 | 733,38 | 500 | 99 |
| 235 | 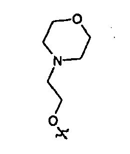 | 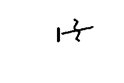 | | | | |
| | C6H12NO2 | I | 755,0571 | 756,33 | 500 | 99 |
| 236 | 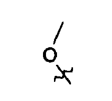 | 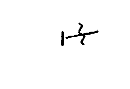 | | | | |
| | OMe | I | 655,9887 | 657,32 | 492 | 95 |
| 237 | 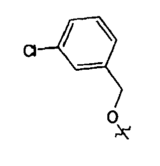 | 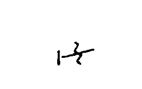 | | | | |
| | C7H6ClO | I | 765,9810 | 767,38 | 492 | 99 |
| 238 | 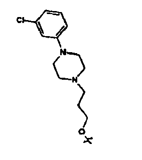 | 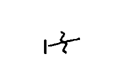 | | | | |
| | C13H18ClN2O | I | 878,0810 | 879,45 | 500 | 99 |
| 239 | 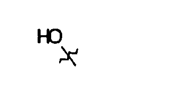 | 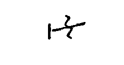 | | | | |
| | OH | I | 641,9730 | 643,31 | 492 | 99 |
| 240 | 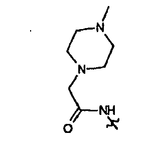 | 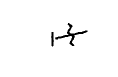 | | | | |
| | C7H14N3O | I | 781,0840 | 782,39 | 500 | 99 |
| 241 | 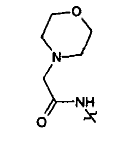 | 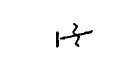 | | | | |
| | C6H11N2O2 | I | 768,0523 | 769,38 | 500 | 99 |
| 242 | 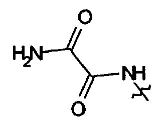 | 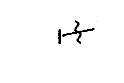 | | | | |
| | C2H3N202 | I | 711,9897 | 713,37 | 500 | 99 |

### Reduktion und Oxidation von Fredericamycin Aldehyd (4)

Fredericamycin Aldehyd **(4)** lässt sich mit einem gebräuchlichen Reduktionsmittel wie Natriumborhydrid in einem Lösungsmittel wie DMF oder Pyridin zum Hydroxymethyl Fredericamycin **(11)** umsetzen. Die Reaktion lässt sich auch als Eintopfreaktion ( Jodatspaltung von Fredericamycin tetrol **(2)** zum Fredericamycin Aldehyd **(4)** ( s. Schema 2 ) und Reduktion ohne Isolierung der Zwischenprodukte zum Fredericamycin Alkohol **(11)** ) zusammenfassen. 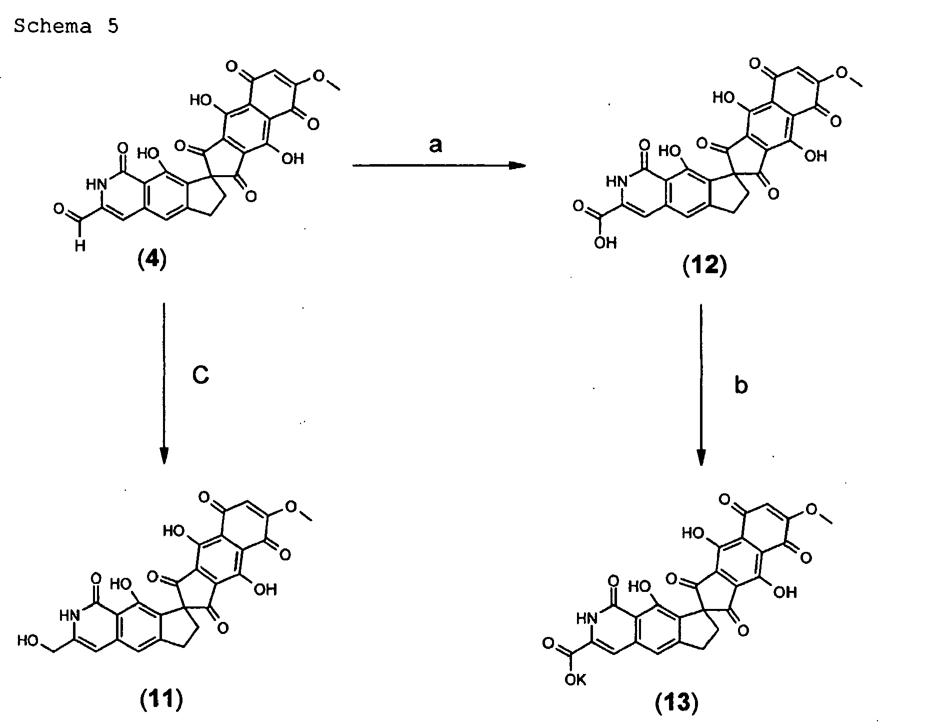
a) NaClO₂, NaH₂PO4, 2,3-Dimethylbuten-2
b) KOH.-H₂O, DMF
c) NaBH₄

Fredericamycin Aldehyd **(4)** kann mit dem Oxidationsmittel Natrium-chlorit (NaClO₂), einem Puffer wie Natriumdihydrogenphosphat in Gegenwart eines Alkens wie 2,3-Dimethylbuten In sehr guten Ausbeuten zur Fredericamycin carbonsäure **(12)** oxidiert werden. Die üblicherweise eingesetzten Oxidationsmethoden, wie man sie in der präparativen Chemie für die Oxidation von Aldehyden zu Carbonsäuren einsetzt ( Oxidation mit Chrom(VI) Verbindungen, Mangan(VII) Verbindungen sowie Persäuren ) führten nicht zum Erfolg. Erst der Einsatz des oben beschriebenen Oxidationsverfahrens ergab das gewünschte Produkt. Die Literatur beschreibt Oxidationen von 2-Pyridon-6-aldehyden mit Silberionen und Kaliumpermangant im alkalischen Medium. Dieses Verfahren ist jedoch für Fredericamycin und seine Derivate nicht geeignet, da Fredericamycin **(1)** basenlabile (-reaktive) Gruppierungen ( OH-Gruppen ) besitzt, die zu unerwünschten Nebenreaktionen führen können.

Das Kaliumsalz der Fredericamycinsäure **(13)** wurde nach herkömmlichem Verfahren durch stöchiometrische Neutralistion gewonnen.

### Substitution am B-Ring

Fredericamycin (1) lässt sich mit Halogenierungsmitteln wie N-Bromsuccinimid (NBS) und N-Jodsuccinimid (NIS) in guten Ausbeuten zu den substituierten 5-Brom- bzw. 5-Jod Fredericamycin Derivaten **(14)** und **(15)** umsetzen ( Schema 6). Der Fredericamycinaldehyd (4) und (36) kann mit elementarem Brom, NBS, BrI, NIS und NCS in die entsprechenden halogensubstituierten Fredericamycinaldehyde (37), (38) und (39) umgewandelt werden.

Die ensprechende Fluorverbindung ist ebenfalls zugänglich. 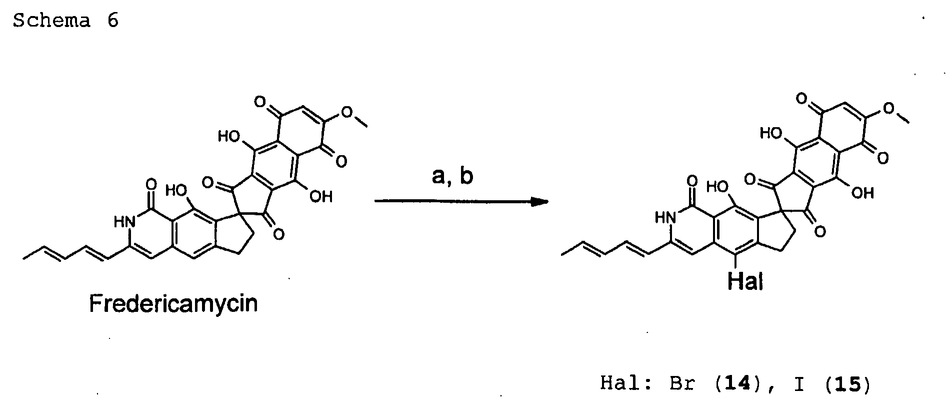
a) N-Bromsuccinimid, DMF, 0°C;
b) N-Jodsuccinimid, DMF, 0°C

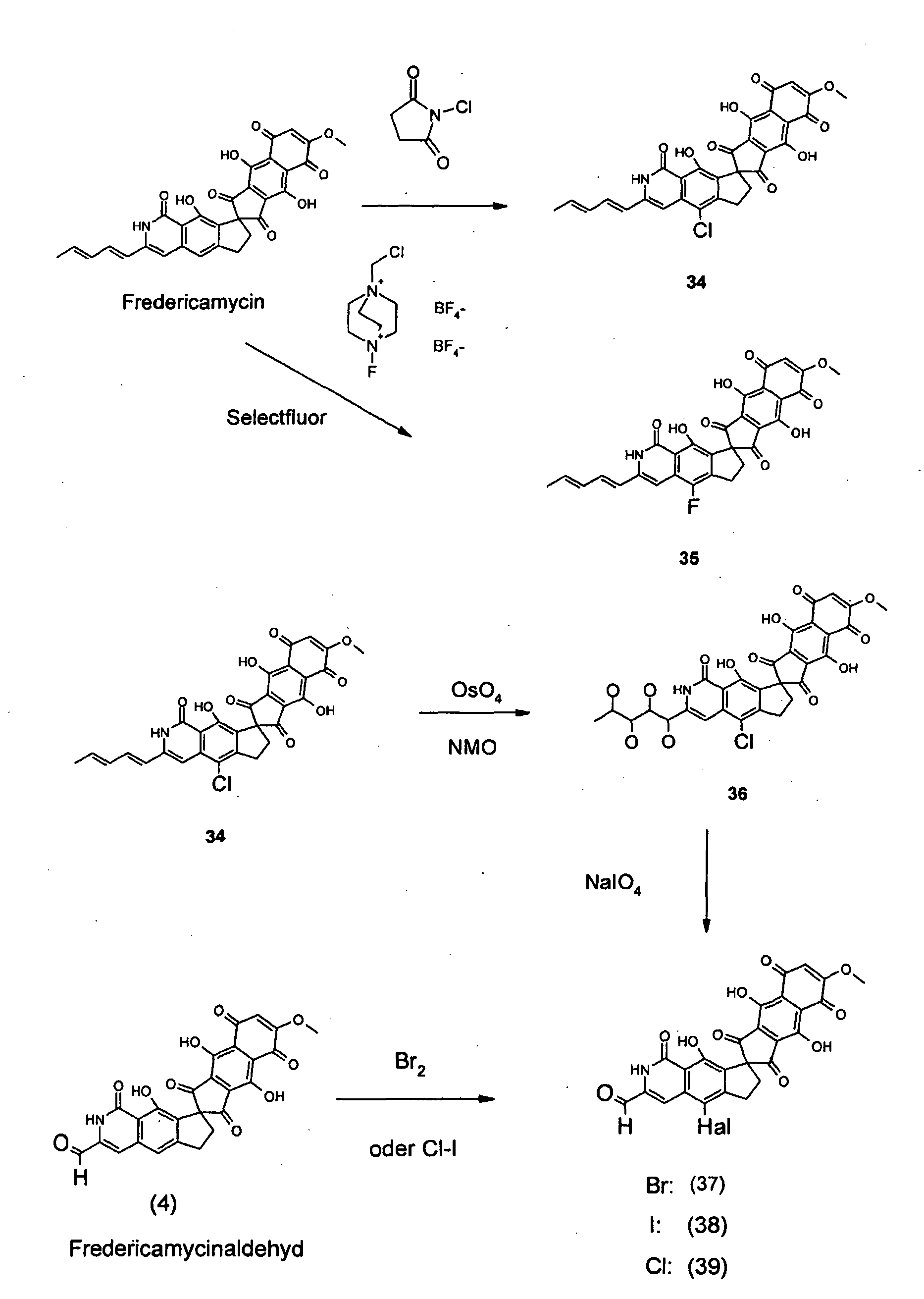

Die beiden nachstehenden Fredericamycin Verbindungen **(23)** und **(24)** sind beides auch Vorstufen. **(23)** ist die Vorstufe für ein aminosäureverknüpftes Fredericamycinderivat.

Die Herstellung von **(23)** ist auch als Beleg anzusehen, dass der Aldehyd **(4)** mit Phosphoryliden nach Wittig oder Wittig-Horner umgesetzt werden kann ( s. Schema 7 ).

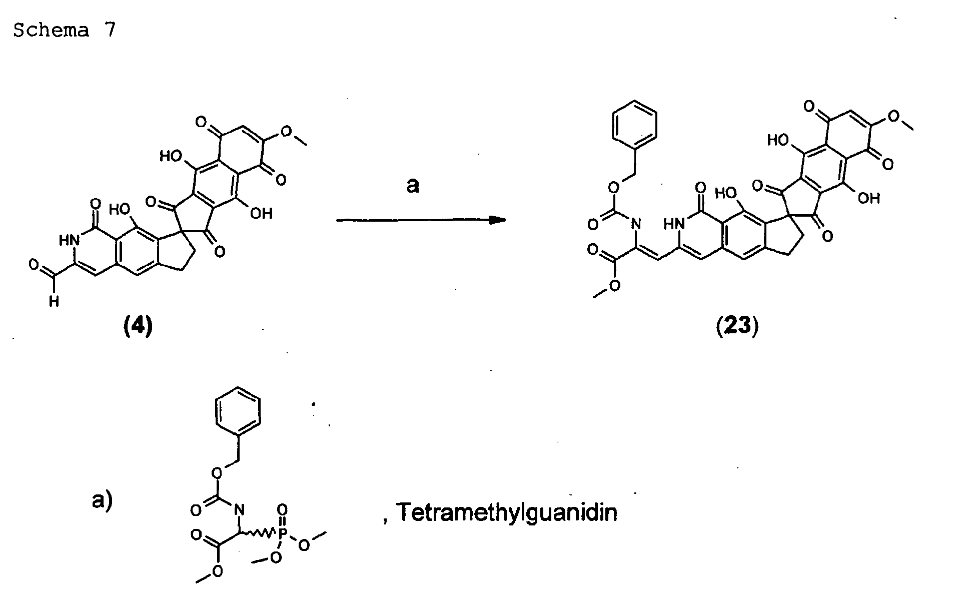

Die Verbindung **(24)** ist die Vorstufe eines N-Methylierten Fredericamycinderivates ( Schema 8 ).

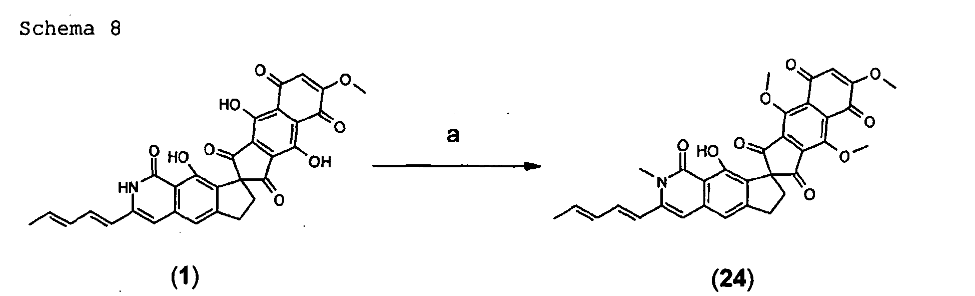

### a) CH₃I, K₂CO₃, DMF, RT

Fredericamycin kann durch Palladium/Wasserstoff nahezu quantitativ in das Tetrahydro Fredericamycin 25 umgewandelt und nach den oben beschriebenen Methoden kernhalogeniert z.B. zur Bromverbindung 26 umgesetzt werden (Schema 9):

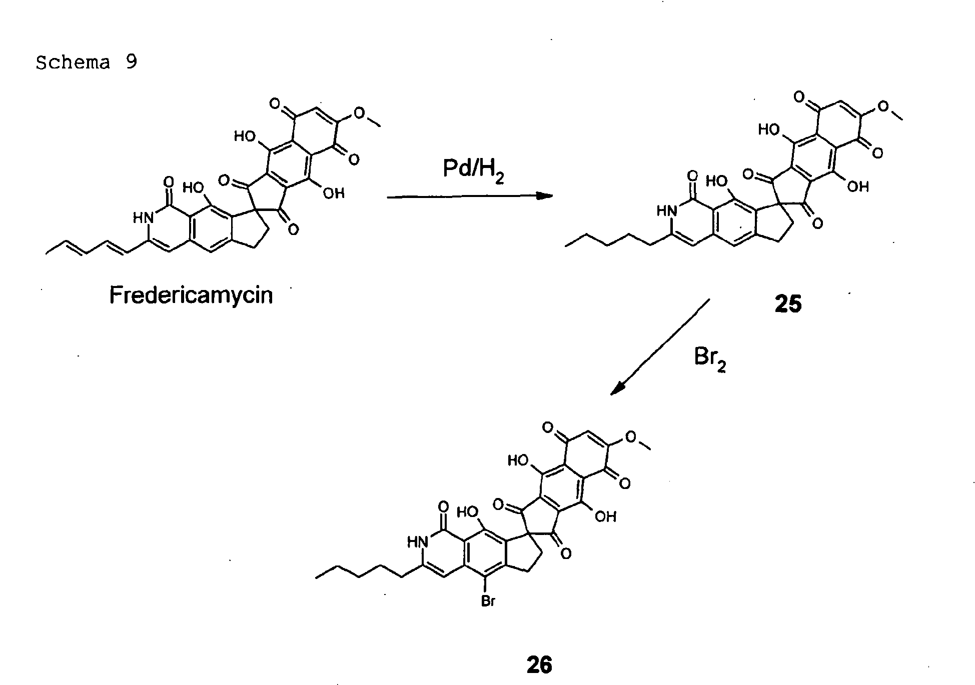

Überraschend wurde auch gefunden, dass sich die Methoxygruppierung im Fredericamycin und den erfindungsgemäßen Derivaten unter Alkali-und Erdalkaliacetat Katalyse durch Sauerstoff Nukleophile wie Alkohole oder Polyole austauschen lässt. Dabei können die können die Alkohole eine Vielzahl verschiedener Substituenten tragen (Tabelle 4).

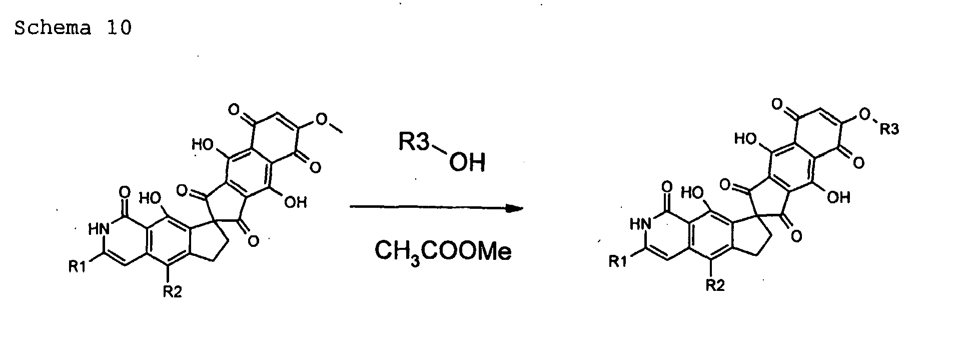

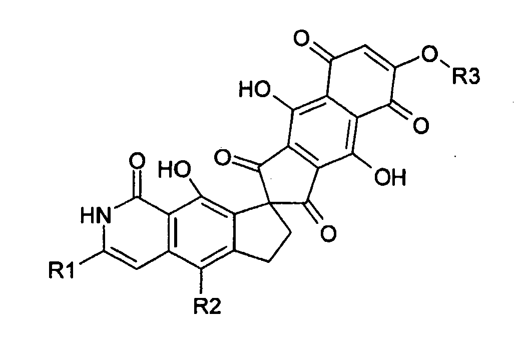

**Tabelle 4**

| **Beispiel** | **R1** | **R2** | **R3** | **UVmax. (nm)** | **M/e** | **Ausbeute (%)** |
|---|---|---|---|---|---|---|
| 243 | 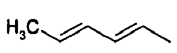 | H | 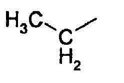 | 504 | (M+H)554 | 97 |
| 244 | 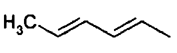 | H | 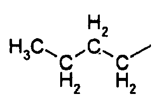 | 500 | (M+) 582 | 96 |
| 245 | 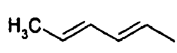 | H | 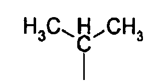 | 500 | (M+H) 568 | 70 |
| 246 | 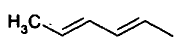 | H | 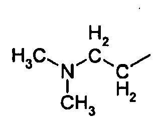 | 504 | (M+H) 597 | 36 |
| 247 | 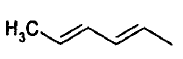 | Br | 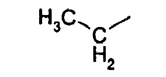 | 504 | (M+) 632/634 | 71 |
| 248 | 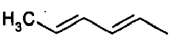 | H | 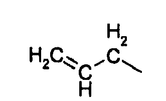 | 500 | (M+H) 566 | 91 |
| 249 | 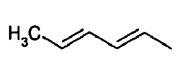 | H | 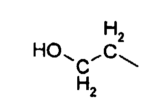 | 499 | (M+) 569 | 52 |
| 250 | 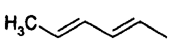 | H | 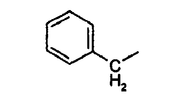 | 504 | (M+H) 616 | 99 |
| 251 | 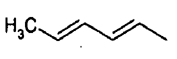 | H | 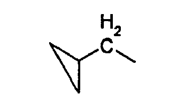 | 500 | (M+) 580 | 99 |
| 252 | 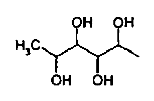 | H | 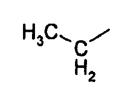 | 499 | (M+H) 622 | 20 |
| 253 | 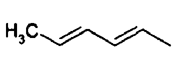 | H | 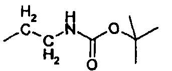 | 500 | (M+H) 669 | 99 |
| 254 | 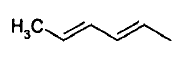 | H | 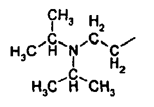 | 504 | (M+H) 653 | 48 |
| 255 | 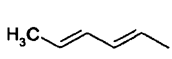 | H | 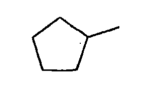 | 504 | (M+H) 594 | 50 |
| 256 | 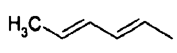 | H | 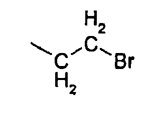 | 499 | (M+H) 632/634 | 99 |

### Austausch der Methoxygruppe am F Ring

Der Austausch der Methoxygruppierung am F-Ring des Fredericamycins sowie an den Derivaten ist durch primäre, sekundäre oder aromatische Amine möglich. Dabei werden die Komponenten mit den entsprechenden primären oder sekundären Aminen bei Raumtemperatur in DMF oder einem anderen inerten Lösungsmittel gerührt. Bei aromatischen Aminen ist eine Katalyse mit Lewissäuren wie Zinn(IV)chlorid etc. erforderlich.

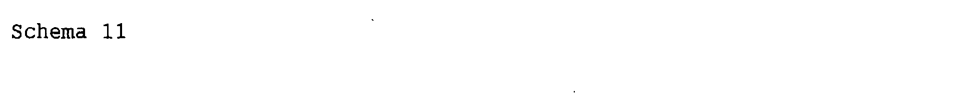

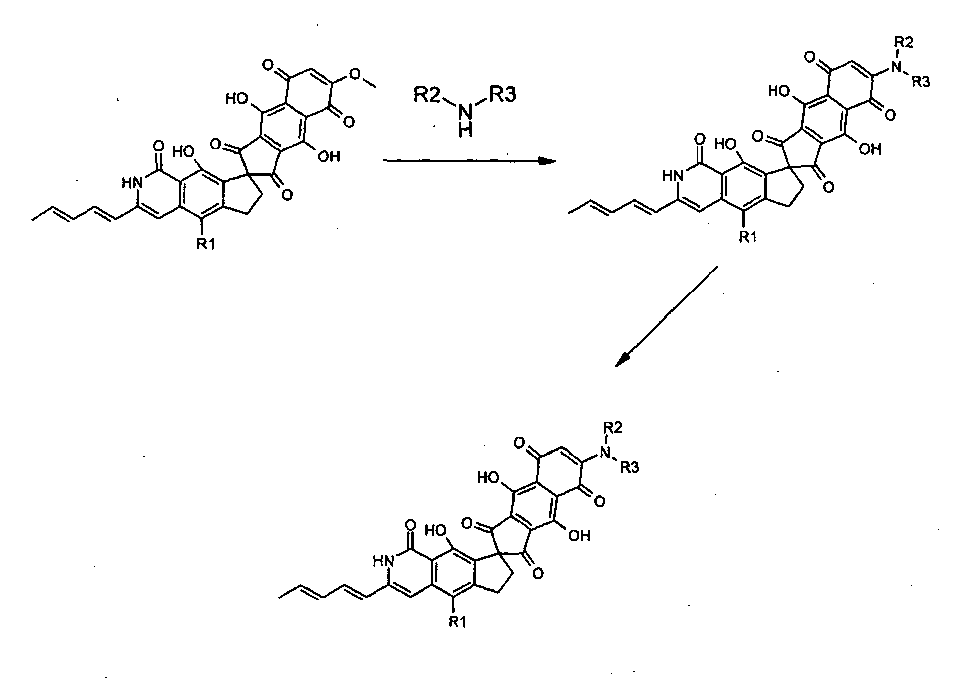

**Tabelle 5**

| R1 | 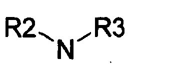 | **Beispiel** |
|---|---|---|
| I | 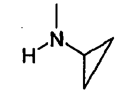 | 257 |
| I | 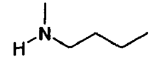 | 258 |
| Br | 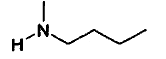 | 259 |
| H | 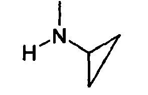 | 260 |
| H | 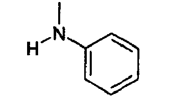 | 261 |
| H | 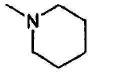 | 262 |
| H | 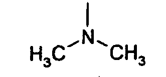 | 263 |
| H | 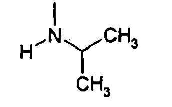 | 264 |
| H | 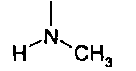 | 265 |
| I | 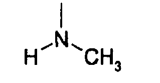 | 266 |
| H | 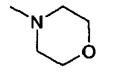 | 267 |
| H | 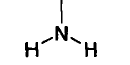 | 268 |
| H | 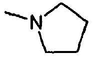 | 269 |
| Br | 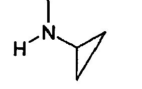 | 270 |

### Herstellung von heterocyclischen Fredericamycin Derivaten

Der Fredericamycin Aldehyd **(4)** laßt sich nach Wittig oder Wittig-Horner zum Pyridalaceton **(271)** umsetzen. Bromierung mit Brom in DMF liefert das in der Seitenkette und am B Ring substituierte Dibromderivat **(272).** Mit den entsprechend substituierten Thioamiden oder Thioharnstoffen sind die entsprechenden Thiazolabkömmlinge **(273-276)** zugänglich. 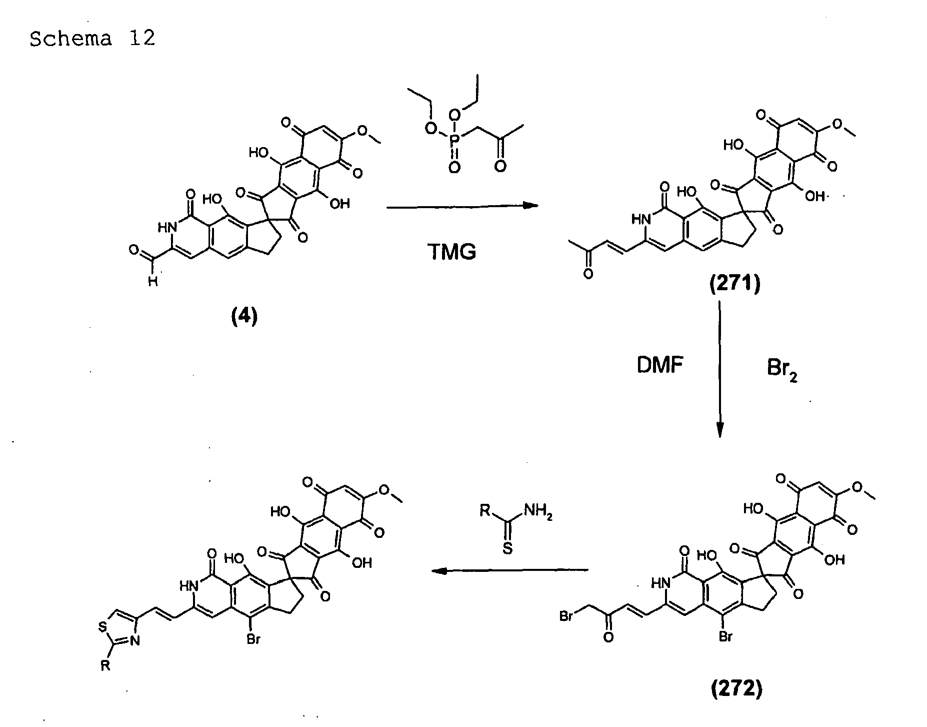 TMG: Tetramethylguanidin

**Tabelle 6**

| **R** | **Beispiel** |
|---|---|
| NH₂ | 273 |
| Ph | 274 |
| CH₃CONH | 275 |
| CH₃ | 276 |

### Herstellung thioanaloger Fredericamycin Derivate

Durch Beschwefelung von Fredericamycin oder seine Derivate mit Lawesson Reagenz oder P₄S₁₀ in Pyridin sind die Thiopyridon analogen Derivate zugänglich (s. Schema 13)

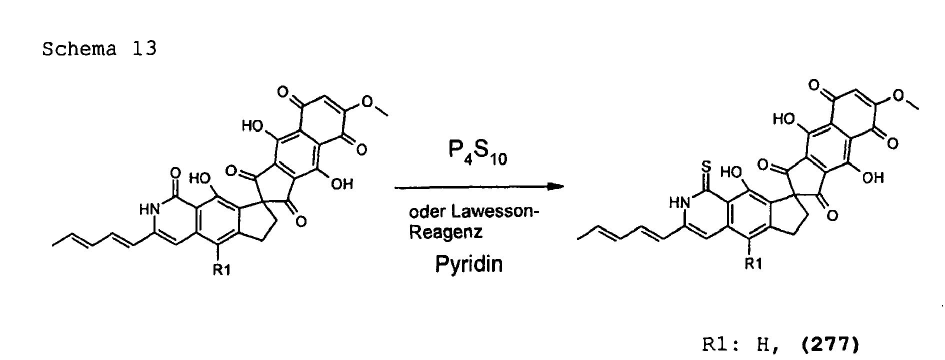

Fredericamycin **(1)** bildet mit Polyzuckern wie α-Cyclodextrin Einschlussverbindungen, wie **(25),** die gegenüber der Ausgangssubstanz gut wasserlöslich sind.

Die Dextrineinschlussverbindungen bilden sich leicht, wenn man die Komponenten im entsprechenden stöchiometrischen Verhältnis in einem geeigneten Lösungsmittel wie DMSO mischt ( s. Schema 11).

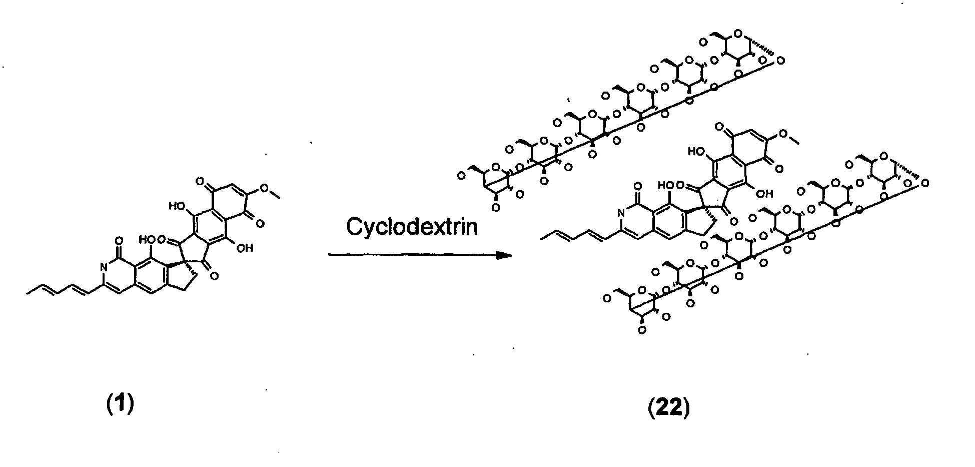

**Biologische Aktivität gegen 12 Krebs Zell-Linien:** LCL(H460/Lunge), MACL(MCF7,Brust), LXFL(529L,Lunge), LXFA(629L,Lunge), MEXF(462NL,Melanom), MEXF(514L,Melanom), MAXF(401NL,Brust), RXF(944L,Renal), RXF(486L,Renal), UXF(1138L,Uterus), PRXF(PC3M,Prostata), PRXF(22RVl).

Wirksamkeit (IC70) gemittelt über alle Zell-Linien in µg/ml bei 5 Testkonzentrationen

**Tabelle 7**

| **Beispiel/Referenz** | **IC70 µg/ml** |
|---|---|
| **Adriamycin** | **0.0210** |
| **Cis-Platin** | **37.1020** |
| **Fredericamycin** | **0.2790** |
| 1 | 0.1130 |
| 13 | 0.0050 |
| 14 | 0.0070 |
| 22 | 0.0080 |
| 23 | 0.0110 |
| 121 | 0.2020 |
| 127 | 0.1550 |
| 192 | 0.0750 |
| 196 | 0.0950 |
| 197 | 0.0340 |
| 198 | 0.2560 |
| 203 | 0.1590 |
| 212 | 0.2100 |
| 214 | 0.0220 |
| 215 | 0.0720 |
| 217 | 0.1290 |
| 218 | 0.0760 |
| 224 | 0.0470 |
| 225 | 0.1110 |
| 230 | 0.0910 |
| 232 | 0.3170 |
| 233 | 0.1000 |
| 234 | 0.0520 |
| 235 | 0.0810 |
| 236 | 0.1210 |
| 265 | 0.1330 |
| 275 | 0.3680 |
| 276 | 0.0840 |

### Beispiele

### Beispiel 1

**1-Deoxy-5-C-[(8R)-4',9,9'-trihydroxy-6'-methoxy-1,1',3',5',8'-pentaoxo-1,1',2,3',5',6,7,8'-octahydrospiro[cyclopenta[g]-isoquinoline-8,2'-cyclopenta[b]-naphtalen]-3-yl]pentitol. (2)**

200mg (0.38 mmol) Fredericamycin A **(1)** werden in 30ml Dichlormethan gelöst. Nach der Zugabe von 20ml Methanol und 4.4ml Wasser werden 350mg (2.6mmol ) N-methylmorpholin-N-oxid eingetragen. Unter kräftigem Rühren tropft man 0.2ml einer 2.5%igen Osmium(IV)oxid-Lösung in t-Butanol zu. Man säuert mit die Reaktionsmischung mit 2-3 Tropfen Trifluoressigsäure an. Nach 48 stündigem Rühren ist die Reaktion laut HPLC-Kontrolle ( RP18, Acetonitril-Wasser (0.2%Essigsäure ) vollständig. Die Reaktionsmischung wird unter kräftigem Rühren in 400ml Wasser eingetragen und der dunkelrote kristalline Feststoff über einen Filter abgesaugt. Im HV trocknen. Ausbeute: 195mg ( 87% d.Th. ) dunkelrotes Pulver. ES⁻: M/e= 606.2 (M+-H), λₘₐₓ: 504.0.

### Beispiel 2

**Tri-Kalium 1-deoxy-5-C-[(8R)-4',9,9'-trihydroxy-6'-methoxy-1,1',3'-,5',8'-pentaoxo-1,1',2,3',5',6,7,8'-octahydrospiro[cyclopenta[g]-isoquinoline-8,2'-cyclopenta[b]-naphtalen]-3-yl]pentitol (3)**

12.0mg ( 19.8 µmol ) Fredericamycin tetrol (2) werden unter Stickstoffatmosphäre in 1.5ml absolutem Pyridin gelöst. Die Lösung wird bei 0°C 30 Minuten lang mit Argon begast. Unter Argonatmosphäre werden bei 0°C 5.94ml einer 0.01N KOH-Lösung auf einmal hinzudosiert. Die Reaktionslösung färbt sich sofort intensiv türkisblau. Die Reaktionsmischung wird noch 1 Stunde nachgerührt und anschließend tiefgefroren und lyophilisiert. Ausbeute: 14.2mg (100%d. Th.). Tiefblaue Kristallmasse.

### Beispiel 3

**(8S)-4',9,9'-trihydroxy-6'-methoxy-1,1',3',5',8'-pentaoxo-1,1',2,3',5',6,7,8'-octahydrospiro[cyclopenta[g]isoquinoline-8,2'-cyclopenta[b]naphtalene]-3-carbaldehyde (4)**
1.) 50mg ( 82.3 µmol ) Tetrahydroxy Fredericamycin (Tetrol **(2)**) werden in 4ml DMF gelöst. Unter kräftigem Rühren wird einen wässrige Natriumjodat-Lösung ( 300mg NaI04 in 1ml Wasser ) innerhalb einer Stunde zugetropft. Nach 1h rühren bei Raumtemperatur wird mit 2 Tropfen Trifluoressigsäure versetzt. Nach weiteren 30 Minuten Rühren wird die Reaktionslösung mit 3ml DMF verdünnt und anschließend mit 150mg NaIO4 gelöst in 0.5ml Wasser versetzt.
   Nach einer weiteren Stunde trägt man in 100ml Wasser ein. Man saugt vom Niederschlag ab und trocknet im HV. Dunkelrotes Kristallpulver. Ausbeute: 41mg (100% d.Th.). M/e= 501.3; UVₘₐₓ: 504,0nm
2.) 109mg ( 179 µmol ) Fredericamycin tetrol **(2)** werden in 8ml Pyridin gelöst. Zugabe von 180µl Wasser. In die Reaktionsmischung werden 450mg (1.08mmol, 6eq )( Polystyrylmethyl )trimethylammonium perjodat Harz zugegeben. Anschließend lässt man 12h bei RT rühren. Man filtriert vom Harz ab, wäscht nach und engt zur Trockene ein. Dunkelroter Rückstand. Ausbeute 89.9mg( 100% d.Th.).
M/e=501.3; UVₘₐₓ: 504.0nm

### Beispiel 4

**1-[2-oxo-2-((2E)-2-([(8S)-4',9,9'-trihydroxy-6'-methoxy-1,1',3',5'-,8'-pentaoxo-1,1',2,3",5',6,7,8'-octahydrospiro[cyclopenta[g]-iaoquinoline-8,2'-cyclopenta[b]naphtalen]-3yl]methylene}ethyl]-dimethylamino trifluoroacetate (118)** 20mg ( 39.9 µmol ) Fredericamycin Aldehyd **(4)** werden unter Argon in 1.5ml absolutem DMF gelöst. Zugabe von 9.1mg ( 47.9 µmol, 1.2eq ) Acetylhydrazid-dimethylammoniumchlorid ( Girard Reagenz D ) und 20mg Polyvinylpyridin (2% DVB). Die Mischung lässt man 2.5h rühren. Anschließend werden 27mg ( 80 µmol, 2.0eq ) Aldehyd-Wang-Resin ( Belegung 3.0 mmol/g) zugegeben und noch 1h nachgerührt. Anschließend wird vom Harz abfiltriert und das Harz noch 3x mit DMF nachgewaschen. Einengen im Hochvakuum. Der Rückstand wird in 1ml Trifluoressigsäure gelöst und nach 10 Minuten zur Trockene eingeengt.

Roter Feststoff: Ausbeute: 28.5mg (100%); ES⁺: M/e=601.3, UVₘₐₓ: 504.0nm

### Beispiel 5

**1-[2-oxo-2-((2E)-2-([(8S)-4',9,9'-trihydroxy-6'-methoxy-1,1',3',-5',8'-pentaoxo-1,1',2,3',5',6,7,8'-octahydrospiro[cyclopenta[g]-isoquinoline-8,2'-cyclopenta[b] naphtalen-3-yl)methylene}hydrazino)-ethyl]pyridinium chloride (119)**

15mg (29.9 µmol) Fredericamycin Aldehyd **(4)** werden in 3ml DMF gelöst.

Bei Raumtemperatur werden 7.5mg (40.0 µmol) Acethydrazinopyridiniumchlorid ( Girard Reagenz P ) gelöst in 75µl Wasser eingetragen. Die Reaktionsmischung wird 1.5h bei Raumtemperatur gerührt wobei der Verlauf der Reaktion mittels HPLC kontrolliert wird. Nach dem Beenden fügt man solange Essigsäureethylester zur Reaktionsmischung, bis ein Niederschlag auszufällen beginnt. Man saugt nach vollendeter Kristallisation den roten Feststoff ab.

Ausbeute: 9.1mg ( 44% d.Th. ). M/e=635.2; λₘₐₓ = 486.0

### Beispiel 6

**(8S)-4',9,9'-trihydroxy-6'-methoxy-1,1',3',5',8'-pentaoxo-1,1',2,3',5',6,7,8'-octahydrospiro[cyclopenta[g]isoquinoline-8,2'-cyclopenta[b]naphthalene]-3-carbaldehyde oxime (122)**

10mg ( 19.4µmol ) Fredericamycinaldehyd **(4)** werden in 2ml DMF gelöst. Nach der Zugabe von 3.1mg ( 44.6 µmol ) Hydroxylammonium chlorid wird mit 3.2µl Pyridin versetzt. Man rührt 2h bei Raumtemperatur. Die Reaktionsmischung wurde in 50ml Wasser eingetragen und 3x mit Essigester extrahiert. Nach dem Trocknen und einengen hinterblieb ein tiefrotes amorphes Kristallpulver ( hplc sauber ).

Ausbeute: 7.9mg ( 72% d.Th. ). ES⁻: M/e=516.1; λₘₐₓ = 500.0nm

### Beispiel 7

**(8S)-4',9,9',-trihydroxy-6'-methoxy-1,1',3',5',8'-pentaoxol,1',-2,3',5',6,7,8'-octahydrospiro[cyclopenta[g]isoquinoline-8,2'-cyclopenta[b]naphthalene]-3-carbaldehyde O-methyloxime (8)**

10mg (19.4 µmol) Fredericamycin aldehyd **(4)** werden in 2ml DMF gelöst. Nach der Zugabe von 3.4mg ( 40.7 µmol ) O-methylhydroxylammoniumchlorid und 3.2 µl Pyridin lässt man die Reaktionsmischung 2h bei Raumtemperatur rühren. Anschließend trägt man in 100ml Wasser ein und saugt vom ausgefallenen roten Niederschlag ab (hplc sauber ).

Ausbeute: 7.6mg ( 71% d.Th. ). ES⁺: M/e=531..2; λₘₐₓ =500.0nm.

### Beispiel 8

**(8S)-4',9,9',-trihydroxy-6'-methoxy-1,1',3',5',8'-pentaoxo-1,1',2,3',5',6,7,8'-octahydrospiro[cyclopenta[g]isoquinoline-8,2'-cyclopenta[b]naphthalene]-3-carbaldehyde O-benzyloxime (9)** 10mg (19.4 µmol) Fredericamycin aldehyd (3) werden in 2ml DMF gelöst. Nach der Zugabe von 6.4mg ( 43.2 µmol ) O-benzylhydroxylammoniumchlorid und 3.2 µl Pyridin lässt man die Reaktionsmischung 2h bei Raumtemperatur rühren. Anschließend trägt man in 50ml Wasser ein und saugt vom ausgefallenen roten Niederschlag ab (hplc sauber).

Ausbeute: 6.8mg ( 57% d.Th. ). ES⁺: M/e=607.2; λₘₐₓ =504.0nm.

### Beispiel 9

**1-O-({(1E)-[(8S)-4',9,9',-trihydroxy-6'-methoxy-1,1'3',5',8'-pentaoxo 1,1',2,3',5',6,7,8'-octahydrospiro[cyclopenta[g]-isoquinoline-8,2'-cyclopenta[b]naphthalene]-3-yl]methylene}amino)-β-D-glucopyranose (10)**

2.0mg ( 4.0µmol ) Fredericamycin aldehyd **(4)** wurden in 150µl DMF gelöst und mit 0.86mg (4.4 µmol ) β-Aminoxy-D-glucopyranose versetzt. Man rührt 24 Stunden bei Raumtemperatur und fügt 5mg ( 15.0 µmol ) Aldehyd-Wang-resin (Belegung 3.0 mmol/g) zu. Nach weiterem 3 stündigem Rühren filtriert man vom Harz ab, wäscht mit DMF nach und engt im Hochvakuum zur Trockene ein.

Ausbeute: 2,7mg ( 99%d.Th. ) rotes Pulver; ES⁻: M/e=678.1; λₘₐₓ =504.0nm.

### Beispiel 10

**(8S)-4',9,9'-trihydroxy-3-(hydroxymethyl)-6'-methoxy-6,7-dihydrospiro [[cyclopenta[g]isoquinoline-8,2'-cyclopenta[b]naphtalene]-1,1',3',5',8'(2H)-pentone (11)**

30mg ( 49.4 µmol ) Tetrahydroxy Fredericamycin **(2)** wurde in 2ml Pyridin gelöst. Es wurden 20mg ( 93.0 µmol ) Natriummetaperjodat gelöst in 0.3ml Wasser hinzugegeben. Nach 4 stündigem Rühren versetzt man mit 10mg ( 260 µmol ) Natriumborhydrid. Nach 12 Stunden wurde zur Trockene eingeengt und der Rückstand mittels präparativer HPLC aufgetrennt.

Ausbeute: 2.6mg ( 13% d. Th. ) rotes Pulver. ES⁻: M/e=503.2; λₘₐₓ = 504.0nm

### Beispiel 11

**(8S)-4',9,9'-trihydroxy-6'-methoxy-1,1',3',5',8'-pentaoxo-1,1',2,3',5',6,7,8'-octahydrospiro[cyclopenta[g]isoquinoline-8,2'-cyclopenta[b]naphtalene]-3-carboxylic acid (12)**

15mg ( 29.9 µmol ) Fredericamcin Aldehyd **(4)** werden in 1ml Dichlormethan und 0.5ml t-Butanol gelöst. Zugabe von 250 µl 2,4-Dimethylbuten. Unter Rühren bei Raumtemperatur wird eine Lösung aus 6.0mg ( 53.1 µmol )) Natriumchlorit ( 80%ig ) und 5.1mg Natriumhydrogenphosphat in 250 µl Wasser zugetropft.

Nach'2.5 Stunden wird nochmals eine Lösung aus 10.0mg ( 88.5 µmol ) Natrium-chlorit und 5mg Natriumdihydrogenphosphat in 200µl Wasser zugegeben. Nach insgesamt 4h wird auf Wasser gegeben und mit Essigester extrahiert.

Das Rohgemisch wurde mittels präparativer HPLC (RP18, Acetonitril-Wasser-Essigsäure) gereinigt. Rotes amorphes Pulver.

Ausbeute: 8.3mg ( 53.5% d.Th. ). E⁻: M/e=516.1; λₘₐₓ = 504.0nm.

### Beispiel 12

**Kalium (8S)-4',9,9'-trihydroxy-6'-methoxy-1,1',3',5',8'-pentaoxo-1,1',2,3',5',6,7,8'-octahydrospiro[cyclopenta[g]isoquinoline-8,2'-cyclopenta[b]naphtalene]-3-carboxylate (13)**

6.9mg ( 13.3 µmol ) Fredericamycin carbonsäure **(12)** werden unter Stickstoff in 5ml DMF gelöst. Bei Raumtemperatur und unter Sauerstoffausschluß werden unter kräftigem Rühren 1.27ml ( 12.7 µmol ) einer wässrigen 0.01N KOH-Lösung eingetropft. Man rührt 15 Minuten bei Raumtemperatur nach und engt im Hochvakuum zur Trockene ein.

Ausbeute: 7.40mg (100 d.Th.); E⁻: M/e= 516.1; λₘₐₓ = 504.0nm

### Beispiel 13

**(8S)-5-bromo-4',9.9'-trihydroxy-6'-methoxy-3-[(1E,3E)-penta-1,3-dienyl]-6,7-dihydrospiro[cyclopenta[g]isoquinoline-8,2'-cyclopenta[b]naphtalene]-1,1'-3',5',8'(2H)-pentone(14)**

20mg ( 37.1µmol ) Fredericamycin (1) werden in 250µl DMF gelöst und anschließend bei 0°C innerhalb einer Stunde mit 6.3mg (35.3 µmol N-Bromsuccinimid in 250µl DMF versetzt. Die Reaktion rührt bei langsam auftauendem Eisbad über Nacht. Das DMF wird anschließend im Hochvakuum abgezogen und der Rückstand durch präparative HPLC gereinigt.

Ausbeute: 7mg ( 32% d.Th. ) rote Kristallmasse. M/e = 616.1/618.1; λₘₐₓ = 486.0nm

### Beispiel 14

**(8S)-5-iodo-4',9.9'-trihydroxy-6'-methoxy-3-[(1E,3E)-penta-1,3-dienyl]-6,7-dihydrospiro[eyclopenta[g]isoquinoline-8,2'-cyclopenta[b]naphtalene]-1,1'-3',5',8'(2H)-pentone (15)**

84mg (158µmol ) Fredericamycin **(1)** werden in 1.0 ml DMF gelöst und anschließend bei 0°C innerhalb einer Stunde mit 33.0 mg (150.0 µmol ) N-Jodsuccinimid in 500µl DMF versetzt. Die Reaktion rührt bei langsam auftauendem Eisbad über Nacht. Das DMF wird anschließend im Hochvakuum abgezogen und der Rückstand (120mg (14) mit einem Gehalt von 80% durch präparative HPLC gereinigt (Gradient CH₃CN 50 -90% innerhalb 16mein ).

Ausbeute: 18mg ( 17% d.Th. ) rote Kristallmasse. M/e = 665.0; λₘₐₓ = 484.0nm

### Beispiel 15

**Methyl-2-([(benzyloxy)carbonyl]amino)-3-[(8S)-4',9.9.'-trihydroxy-6'-Methoxy-1,1',3',5',8'-pentaoxo-1,1',2,3',5',6,7,8'-octahydrospiro-[cyclopenta[g]isoquinoline-8,2'-cyclopenta[b]naphtalen]-3-yl]acrylate (23)**

66mg ( 200µmol ) Z-α-phosphonoglycintrimethylester werden unter Argon in 1ml absolutem Pyridin gelöst und bei 0°C mit 25µl 1,1,3,3-Tetramethylguanidin versetzt. Nach 40 Minuten fügt man bei 0°C 20mg ( 40µmol) Fredericamycin Aldehyd (4) hinzu. Nach 15 Minuten trägt man in 20ml 1M Essigsäure ein und extrahiert 3x mit Essigester. Das Rohprodukt wurde mittels präparativer HPLC ( RP18, Acetonitril-Wasser ) gereinigt. Ausbeute: 10.0mg ( 36%d.Th. ). M/e=706.4; λₘₐₓ = 492.0nm

### Beispiel 16

**(8S)-9-hydroxy-4',6',9'-trimethoxy-2-methyl-3-[(1E,3E)-penta-1,3-dienyl]-6,7-dihydrospiro[cyclopenta[g]isoquinoline-8,2'-cyclopenta[b]naphtalene]-1,1',3',5',8'(2H)-pentone (24)**

10mg ( 15µmol ) Fredericamycin (1) werden unter Schutzgas in 4ml absolutem DMF gelöst. Bei RT werden 400µl (4311µmol) Methyljodid und 81mg gepulvertes Kaliumcarbonat eingetragen. Die Reaktionsmischung wird 20h bei RT gerührt und anschließend auf Wasser gegeben. Extrahieren mit Essigester und reinigen des Rückstandes mittels Chromatographie an Chloroform/Methanol 30/1 aufgetrennt.

Ausbeute: 4mg ( 37%d.Th. ). Gelber Rückstand. M/e=582.3; λₘₐₓ = 368.0nm.

### Beispiel 17

### Fredericamycin A 1:2 Komplex mit α-Cyclodextrin (22)

Eine 10mg Fredericamycin ( 0.025 mMol ) werden zu einer Lösung von 50mg α-Cyclodextrin ( 0.050 mMol ) in 500µl Dimethylsulfoxid gegeben. Die Lösung wird dann mit 5ml Wasser verdünnt.

Eine so hergestellte Stammlösung lässt sich beliebig mit Wasser verdünnen.
λ ₘₐₓ = 504.0nm

### Beispiel 18

**4',9,9'-trihydroxy-6'-methoxy-1,1',3',5',8'-pentaoxo-1,1',2,3'_{,}5',-6,7,8'-octahydrospiro[cyclopenta[*g*]isoquinoline-8,2'-cyclopenta[b]-naphthalene]-3-carbaldehyde(4-methylpiperazin-1-yl)hydrazone (111)**

5.00 mg ( 9.42 µmol ) Fredericamycin Aldehyd 4 werden in 500µl DMF sowie 25µl Trifluoressigsäure gelöst. Bei Raumtemperatur fügt man 1.30 mg ( 11.3 µmol ) 1-Amino-4-methyl-piperazin hinzu. Nach 4.5h rühren bei Raumtemperatur wurden jeweils läquivalent Wang-Aldehyd Harz sowie Sulfonhydrazid Harz zugegeben und 2h gerührt. Abfiltrieren und einengen der Reaktionslösung im Hochvakuum.

Rotes Pulver. Ausbeute: 5.4mg (91% d. Th.). M/e = 599 (M+H)⁺, λₘₐₓ = 504.0nm.

### Beispiel 19

**4',9,9'-trihydroxy-6'-methoxy-1,1',3',5',8'-pentaoxo-1,1',2,3',5',-6,7,8'-octahydrospiro[cyclopenta[*g*]isoquinoline-8,2'-cyclopenta[b]-naphthalene]-3-carbaldehyde-4,5-dihydro-1H-imidazol-2-ylhydrazone (123)**

5.00 mg ( 9.42 µmol ) Fredericamycin Aldehyd 4 werden in 500µl DMF sowie 25µl Trifluoressigsäure gelöst. Bei Raumtemperatur fügt man 2.05mg ( 11.3 µmol ) 2-Hydrazino-2-imidazolin hydrobromid hinzu. Nach 4.5h rühren bei Raumtemperatur wurden jeweils läquivalent Wang-Aldehyd Harz sowie Sulfonhydrazid Harz zugegeben und 2h gerührt. Vom Harz abfiltrieren und einengen der Reaktionslösung.

Im Hochvakuum.

Rotes Pulver. Ausbeute: 3.9mg ( 67% d. Th. ). M/e = 584 (M+H)⁺, λₘₐₓ = 504.0nm.

### Beispiel 20

**4',9,9'-trihydroxy-6'-methoxy-3-{(*E*)-[(4-oxo-2-thioxo-1,3-thiazolidin-3-yl)imino]methyl}-6,7-dihydrospiro[cyclopenta[g]-isoquinoline-8,2'-cyclopenta[b]naphthalene]-1,1',3',5',8'(2H)-pentone (113)**

5.00 mg ( 9.42 µmol ) Fredericamycin Aldehyd 4 werden in 500µl DMF sowie 25µl Trifluoressigsäure gelöst. Bei Raumtemperatur fügt man 1.67 mg ( 11.3 µmol ) N-Aminorhodanid hinzu. Nach 4.5h rühren bei Raumtemperatur wurden jeweils läquivalent Wang-Aldehyd Harz sowie Sulfonhydrazid Harz zugegeben und 2h gerührt. Abfiltrieren und einengen der Reaktionslösung.

Rotes Pulver. Ausbeute: 4.1mg (65% d. Th.). M/e = 599 (M+H)⁺, λₘₐₓ = 504.0nm.

### Beispiel 21

**4',9,9'-trihydroxy-6'-methoxy-1,1',3',5',8'-pentaoxo-1,1',2,3',5',-6,7,8'-octahydrospiro[cyclopenta[g]isoquinoline-8,2'-cyclopenta[b]-naphthalene]-3-carbaldehyde-*O*-(2-morpholin-4-ylethyl)oxime (27)**

5.00 mg (9.42 µmol ) Fredericamycin Aldehyd 4 werden in 500µl DMF sowie 25µl Trifluoressigsäure gelöst. Bei Raumtemperatur fügt man 2.47 mg (11.3 µmol ) N-( Aminoxyethyl) morpholin dihydrochlorid hinzu. Nach 4.5h rühren bei Raumtemperatur wurden jeweils läquivalent Wang-Aldehyd Harz (3.1mg, 9.4µmol, Belegung: 3.0mmol/g ) sowie läquivalent Sulfonhydrazid Harz (6.1mg, 9.4mMol, 1.5mmol ) zugegeben und 2h gerührt. Abfiltrieren und einengen der Reaktionslösung im Hochvakuum.

Rotes Pulver. Ausbeute: 6.1mg (98% d. Th.). M/e = 630 (M+H)⁺, λₘₐₓ = 504.0nm.

### Beispiel 22

**(8S)-5-chloro-4',9.9'-trihydroxy-6'-methoxy-3-[(1E,3E)-penta-1,3-dienyl]-6,7-dihydrospiro[cyclopenta[*g*]isoquinoline-8,2'-cyclopenta[b]naphtalene]-1,1'-3',5',8'(2H)-pentone (34)**

300mg ( 556.6µmol ) Fredericamycin **(1)** werden unter Argon in 10ml DMF gelöst und anschließend mit 75.0mg (556.6 µmol) N-Chlorsuccinimid versetzt. Die Reaktion rührt 5h bei 40°C. Das Reaktionsgemisch wird anschließend in 400ml Methanol/Wasser 1:1 eingetragen und der ausgefallenen rote Niederschlag abgesaugt und im Hochvakuum getrocknet.

Ausbeute: 305mg ( 96% d.Th. ) rote Kristallmasse. M/e = 573/575; λₘₐₓ = 504.0nm

### Beispiel 23

**(8S)-5-fluoro-4',9.9'-trihydroxy-6'-methoxy-3-[(1E,3E)-penta-1,3-dienyl]-6,7-dihydrospiro[cyclopenta[g]isoquinoline-8,2'-cyclopenta[b]naphtalene]-1,1'-3',5',8'(2H)-pentone (35)**

50mg (92.8µmol) Fredericamycin **(1)** werden unter Argon in 5ml DMF gelöst und anschließend mit 33.0mg (93.5 µmol) 1-Chlormethyl-4-fluoro-1,4-diazoniaycyclo[2.2.2]octan bis-(tetrafluoroborat) Selectfluor. ® versetzt. Die Reaktion rührt 24h bei Raumtemperatur. Das Reaktionsgemisch wird anschließend in 200ml Wasser eingetragen und mit Essigester extrahiert. Das Rohprodukt wurde nach dem Einengen durch präparative HPLC ( RP18, Acetonitril-Wasser-Essigsäure ) gereinigt.

Ausbeute: 7.1mg ( 14% d.Th. ) rote Kristallmasse. M/e = 557; λₘₐₓ = 504.0nm

### Beispiel 24

**1-Deoxy-5-C-[(8R)-5-chloro-4',9,9'-trihydroxy-6'-methoxy-1,1',3',5'-,8'-pentaoxo-1,1',2,3',5',6,7,8'-octahydrospiro[cyclopenta[g]-isoquinoline-8,2'-cyclopenta[b]-naphtalen]-3-yl]pentitol (36)**

120mg (209 mmol) Chlorofredericamycin **34** werden in 25.0ml Dichlormethan gelöst. Nach der Zugabe von 3.6ml Methanol und 0.8ml Wasser werden 197mg (1.46mmol ) N-methylmorpholin-N-oxid eingetragen. Unter kräftigem Rühren tropft man 0.12ml einer 2.5%igen Osmium(IV)oxid-Lösung in t-Butanol zu. Nach 27 stündigem Rühren ist die Reaktion laut HPLC-Kontrolle ( RP18, Acetonitril-Wasser (0.2%Essigsäure) vollständig. Die Reaktionsmischung wird unter kräftigem Rühren in 200ml Wasser eingetragen und der dunkelrote kristalline Feststoff abgesaugt. Im HV trocknen.

Ausbeute: 101mg (75% d.Th.) dunkelrotes Pulver. M/e= 641/643, λₘₐₓ 504.0.

### Beispiel 25

**(8S)-4',9,9'-trihydroxy-5₋bromo-6'-methoxy-1,1',3',5',8'-pentaoxo-1,1',2,3',5',6,7,8'-octahydrospiro[cyclopenta[g]isoquinoline-8,2'-cyclopenta[b]naphtalene]-3-carbaldehyde (37)**

100mg (200µmol) Fredericamycinaldehyd werden unter Argon in 5ml DMF gelöst. Anschließend wird mit 200µl einer 1M Bromlösung in DMF versetzt. Nach 1.5h rühren bei RT werden noch einmal 20µl Bromlösung eingetragen. Die Reaktionsmischung ist laut HPLC nach insgesamt 3.5h vollständig.

In 150ml Wasser eintragen und mit Dichlormethan ausschütteln.

Ausbeute: 96mg ( 83% d.Th. ) dunkelrotes Pulver. M/e= 579/581, λₘₐₓ: 504.0.

### Beispiel 26

**1,2,3,4-Tetrahydro-5-bromo-4',9,9'-trihydroxy-6'-methoxy-3-[(1E,3E)-penta-1,3-dienyl]-6,7-dihydrospiro[cyclopenta[g]isoquinoline-8,2'-cyclopenta[b]naphtalene]-1,1',3',5',8'(2H)-pentone (26)**

8.0mg(0.0128mmol) 1,2,3,4-Tetrahydrofredericamycin 25 werden unter Stickstoff in 1ml absolutem DMF gelöst. Zu der Lösung tropft man bei Raumtemperatur eine Lösung von 2.3mg(0.0128mmol) Brom in 0.25ml DMF zu. Man rührt bei Raumtemperatur über 24h. Das Reaktionsgemisch wird im Hochvakuum zur Hälfte eingeengt und anschließend auf 100ml Wasser gegeben. Man saugt vom ausgefallenen Niederschlag ab und trocknet im Vakuum.
rotes Kristallpulver 8.1mg (88%d.Th.) m/e: 621/623; λₘₐₓ: 499nm.

### Beispiel 27

**(8S)-4',9.9'-trihydroxy-6'-benzylamino-3-[(1E,3E)-penta-1,3-dienyl]-6,7-dihydrospiro[cyclopenta[g]isoquinoline-8,2'-cyclopenta[b]naphtalene]-1,1'-3',5',8'(2H)-pentone**

20mg (37.1mol ) Fredericamycin wird unter Argon in 1ml DMF gelöst und anschließend bei Raumtemperatur mit 4.76mg (44.50µmol) Benzylamin versetzt. Nach 3h hat sich laut HPLC ( RP18,

Acetonitril/Wasser ) ein einheitliches neues Produkt gebildet. Man engt die Reaktionsmischung im Hochvakuum zur Trockene ein.

Rote Kristallmasse Ausbeute: 23mg ( 100% ). M/e=615.3 (M+H), λmax=492nm

### Beispiel 28

**(8S)-5-chloro-4',9.9'-trihydroxy-6'-benzylamino-3-[(1E,3E)-penta-1,3-dienyl]-6,7-dihydrospiro[cyclopenta[g]isoquinoline-8,2'-cyclopenta[b]naphtalene]-1,1'-3',5',8'(2H)-pentone**

5.0mg (8.71µmol ) 5-Chlor Fredericamycin wird unter Argon in 1ml DMF gelöst und anschließend bei Raumtemperatur mit 1.12mg (10.45µmol) Benzylamin versetzt. Nach 29h engt man die Reaktionsmischung im Hochvakuum zur Trockene ein.

Rote Kristallmasse Ausbeute: 5mg (89% ). M/e=649.1 (M+H), λmax=492nm

### Beispiel 28

**(8S)-4',9.9'-trihydroxy-6'-ethanolamino-3-[(1E,3E)-penta-1,3-dienyl]-6,7-dihydrospiro[cyclopenta[g]isoquinoline-8,2'-cyclopenta[b]naphtalene]-1,1'-3',5',8'(2H)-pentone**

10mg ( 18.6µmol ) Fredericamycin wird unter Argon in 1ml DMF gelöst und anschließend bei Raumtemperatur mit 1.36mg (22.3µmol) Ethanolamin versetzt. Nach 3h hat sich laut HPLC ( RP18,

Acetonitril/Wasser ) ein einheitliches neues Produkt gebildet. Man engt die Reaktionsmischung im Hochvakuum zur Trockene ein.

Rote Kristallmasse Ausbeute: 9mg (85% ). M/e=569.3 (M+H), λmax=500nm

### Beispiel 29

**(8S)-4',9.9'-trihydroxy-6'-(4-piperidylmethylamino)-3-[(1E,3E)-penta-1,3-dienyl]-6,7-dihydrospiro[cyclopenta[g]isoquinoline-8,2'-cyclopenta[b]naphtalene]-1,1'-3',5',8'(2H)-pentone**

10mg (18.6µmol ) Fredericamycin wird unter Argon in 1ml DMF gelöst und anschließend bei Raumtemperatur mit 2.7µl (22.3µmol) 4-Aminomethylpiperidin versetzt. Nach 24h engt die Reaktionsmischung im Hochvakuum zur Trockene ein.

Rote Kristallmasse Ausbeute: 11mg (99% ). M/e=622.3 (M+H), λmax=492nm

### Beispiel 100 - 242

In analoger Weise wie Beispiel 7, 8, 9, 10, 18, 19 und 20 können die Verbindungen 100 - 242, hergestellt werden:

### Beispiel 100

**4',9,9'-trihydroxy-6'-methoxy-1,1',3',5',8'-pentaoxo-1,1',2,3',5',6,7,8'-octahydrospiro[cyclopenta[*g*]isoquinoline-8,2_{'}-cyclopenta[b]naphthalene]-3-carbaldehydepyridin-2-yl-hydrazone 100**

Ausbeute: ( 95% d.Th. ). MS: M/e=593.1; λₘₐₓ = 500.0nm

### Beispiel 101

**4',9,9'-trihydroxy-6'-methoxy-1,1',3',5',8'-pentaoxo-1,1',2,3',5',-6,7,8'-octahydrospiro[cyclopenta[*g*]isoquinoline-8,2'-cyclopenta[*b*]-naphthalene]-3-carbaldehyde [4-(trifluoromethyl)pyrimidin-2-yl]hydrazone 101**

Ausbeuten: ( 95% d.Th. ). MS: M/e=562.1; λₘₐₓ = 500.0nm

### Beispiel 102

**N'-[(1E)-(4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta[b]naphthalen]-3-yl)methylene]pyridyl-3-carbohydrazide 102** Ausbeute: ( 95% d.Th. ). MS: M/e=621.1; λₘₐₓ= 492.0nm

### Beispiel 103

**N'-[(1E)-(4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta[b]naphthalen]-3-yl)methylene]isonicotinohydrazide 103** Ausbeute: ( 95% d.Th. ). MS: M/e=621.1; λₘₐₓ= 500.0nm

### Beispiel 104

**4',9,9'-trihydroxy-6'-methoxy-1,1',3',5',8'-pentaoxo-1,1',2,3',5',-6,7,8'-octahydrospiro[cyclopenta[g]isoquinoline-8,2'-cyclopenta[b]-naphthalene]-3-carbaldehyde-1,2,4-triazole-4-ylhydrazone 104** Ausbeute: (80% d.Th.). MS: M/e=568.1; λₘₐₓ₄= 500.0nm

### Beispiel 105

**4'_{,}9,9'-trihydroxy-6'-methoxy-1,1',3',5',8'-pentaoxo-1,1',2,3'_{,}5'_{,}-6,7,8'-octahydrospiro[cyclopenta[g]isoquinoline-8,2'-cyclopenta[b]-naphthalene]-3-carbaldehyde-4,5-dihydro-1H-imidazol-2ylhydrazone 105** Ausbeute: ( 95% d.Th. ). MS: M/e=584.1; λₘₐₓ= 492.0nm

### Beispiel 106

**N'-[(1E)-(4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta[b]naphthalen]-3-yl)methylene]-2-furohydrazide 106** Ausbeute: ( 95% d.Th. ). MS: M/e=610.0; λₘₐₓ= 492.0nm

### Beispiel 107

**4-amino-N'-[(1E)-(4',9,9'-trihydroxy-6'-methoxy-1,1',3',5',8'-pentaoxo-1,1',2,3',5',6,7,8'-tahydrospiro[cyclopenta[g]isoquinoline-8,2'-cyclopenta[b]naphthalen]-3-yl)methylene]benzohydrazide 107** Ausbeute: ( 95% d.Th. ). MS: M/e=635.1; λₘₐₓ= 492.0nm

### Beispiel 108

**4',9,9'-trihydroxy-6'-methoxy-1,1',3',5',8'-pentaoxo-1,1',2,3',5',6,7,8'-octahydrospiro[cyclopenta[g]isoquinoline-8,2'-cyclopenta[b]naphthalene]-3-carbaldehydethiosemicarbazone 108** Ausbeute: (95% d.Th.). MS: M/e=558.0; λₘₐₓ= 492.0nm

### Beispiel 109

**N'-[(1E)-(4',9,9'-trihydroxy-6'-methoxy-1,1',3',5',8'-pentaoxo-1,1',2,3',5',6,7,8'-octahydrospiro[cyclopenta[g]isoquinoline-8,2'-cyclopenta[b]naphthalen]-3-yl)methylene]thiophene-2-carbohydrazide 109**
Ausbeute: ( 95% d.Th. ). MS: M/e=626.0; λₘₐₓ= 492.0nm

### Beispiel 110

**2-(1H-indol-3-yl)-N'-[(1E)-(4',9,9'-trihydroxy-6'-methoxy-1,1',3',5',8'-pentaoxo-1,1',2,3',5',6,7,8'-octahydrospiro [cyclopenta[g]isoquinoline-8,2'-cyclopenta[b]naphthalen]-3-yl)methylene]acetohydrazide 110**
Ausbeute: ( 95% d.Th. ). MS: M/e=673.1; λₘₐₓ= 492.0nm

### Beispiel 111

**4',9,9'-trihydroxy-6'-methoxy-1,1',3'_{,}5',8'-pentaoxo-1,1',2,3',5'₋,6,7,8'-octahydrospiro[cyclopenta[*g*]isoquinoline-8,2'-cyclopenta[*b*]-naphthalene]-3-carbaldehyde(4-methylpiperazin-1-yl)hydrazone 111** Ausbeute: ( 95% d.Th. ). MS: M/e=599.1; λₘₐₓ= 492.0nm

### Beispiel 112

**2-oxo-2-((2E)-2-[(4',9,9'-trihydroxy-6'-methoxy-1,1'3',5',8'-pentaoxo-1,1',2,3',5',6,7,8'-octahydrospiro[cyclopenta[*g*]-isoquinoline-8,2'-cyclopenta[b]naphthalen]-3-yl)methylene]-hydrazino)acetamide 112**
Ausbeute: ( 95% d.Th. ). MS: M/e=587.1; λₘₐₓ= 492.0nm

### Beispiel 113

**4',9,9'-trihydroxy-6'-methoxy-3-{(E)-[(4-oxo-2-thioxo-1,3-thiazolidin-3-yl)imino]methyl}-6,7-dihydrospiro[*g*]isoquinoline-8,2'-cyclopenta[b]naphtalene]-1,1',3',5',8'(2H)-pentone 113**
Ausbeute: ( 95% d.Th. ). MS: M/e=632.0; λₘₐₓ= 500.0nm

### Beispiel 114

**{(2*E*)-2-[(4',9,9'-trihydroxy-6'-mothoxy-1,1',3',5',8'-pentaoxo-1,1',2,3',5',6,7,8'-octahydrospiro[cyclopenta[*g*]isoquinoline-8,2'-cyclopenta[b]naphthalen]-3-yl)methylene]hydrazino}acetonitrile 114** Ausbeute: (95% d.Th.). MS: M/e=583.1; λₘₐₓ= 492.0nm

### Beispiel 115

**2-amino-N'-[(1E)-(4',9,9'-trihydroxy-6'-methoxy-1,1',3',5',8'-pentaoxo-1,1',2,3',5',6,7,8'-octahydrospiro[cyclopenta[g]-isoquinoline-8,2'-cyclopenta[*b*]naphthalen]-3-yl)methylene]-benzohydrazide 115**
Ausbeute: ( 70% d.Th. ). MS: M/e=635.1; λₘₐₓ= 492.0nm

### Beispiel 116

**4',9,9'-trihydroxy-6'-methoxy-1,1',3',5',8'-pentaoxo-1,1',2,3',5',-6,7,8'-octahydrospiro[cyclopenta[*g*]isoquinoline-8,2'-cyclopenta[b]-naphthalene]-3-carbaldehyde *O*-[2-morpholin-4-yl-ethyl)oxime 116**
Ausbeute: ( 85% d.Th. ). MS: M/e=630.1; λₘₐₓ= 492.0nm

### Beispiel 117

**(2E)-2-[(4',9,9'-trihydroxy-6'-methoxy-1,1',3' ,5',8'-pentaoxo-1,1',2,3',5',6,7,8'-octahydrospiro[cyclopenta[*g*]isoquinoline-8,2'-cyclopenta[b]naphthalen]-3-yl)methylene]hydrazinecarboximidamide 117** Ausbeute: ( 95% d.Th. ). MS: M/e=558.1; λₘₐₓ= 500.0nm

### Beispiel 118

**2-(dimethylamino)-N'-[(1E)-(4',9,9'-trihydroxy-6'-methoxy-1,1',3',5',8'-pentaoxo-1,1',2,3',5',6,7,8'-octahydrospiro-[cyclopenta[g]isoquinoline-8,2'-cyclopenta[b]naphthalen]-3-yl)methylene]acetohydrazide 118**
Ausbeute: ( 85% d.Th. ). MS: M/e=601.1; λₘₐₓ= 492.0nm

### Beispiel 119

**1-[2-oxo-2-((2E)-2-([(8S)-4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta[b] naphtalen-3-yl]methylene}hydrazino)ethyl]pyridinium chloride 119** Ausbeute: (85% d.Th.). MS: M/e=635.1; λₘₐₓ= 492.0nm

### Beispiel 120

**(8S)-4-,9,9-,-trihydroxy-6'-methoxy-1,1',3',5',8'-pientaoxol,1',2,-3',5',6,7,8'-octahydrospiro [cyclopenta[*g*]isoquinoline-8,2'-cyclopenta[b]naphthalene]-3-carbaldehyde O-methyloxime 120**
Ausbeute: (90% d.Th.). MS: M/e=531.1; λₘₐₓ= 492.0nm

### Beispiel 121

**4',9,9'-trihydroxy-6'-methoxy-l,l',3',5',8'-pentaoxo-1,1',2,3',5',-6,7,8'-octahydrospiro[cyclopenta[*g*]isoquinoline-8,2'-cyclopenta[b]-naphthalene]-3-carbaldehyde O-benzyloxime 121**
Ausbeute: ( 95% d.Th. ). MS: M/e=607.1; λₘₐₓ= 492.0nm

### Beispiel 122

**4',9,9'-trihydroxy-6'-methoxy-1,1',3',5',8'-pentaoxo-1,1_{'},2,3',5',6,7,8'-octahydrospiro[cyclopenta[g]isoquinoline-8,2'-cyclopenta[b]naphthalene]-3-carbaldehyde oxime 122**
Ausbeute: ( 95% d.Th. ). MS: M/e=517.1; λₘₐₓ= 482.0nm

### Beispiel 123

**1-O-({(1E)-[(8S)-4',9,9',-trihydroxy-6'-methoxy-1,1',3',5',8'-pentaoxo 1,1',2,3',5',6,7,8'-octahydrospiro[cyclopenta[g]-isoquinoline-8,2'-cyclopenta[b]naphthalene]-3-yl]methylene}amino)-β-D-glucopyranose 123**
Ausbeute: (95% d.Th.). MS: M/e=679.1; λₘₐₓ= 500.0nm

### Beispiel 124

**4',9,9'-trihydroxy-6'-methoxy-1,1',3',5',8'-pentaoxo-1,1',2,3',5',-6,7,8'-octahydrospiro[cyclopenta[*g*]isoquinoline-8,2'-cyclopenta[b]-naphthalene]-3-carbaldehyde-phenylsemicarbazone 124**
Ausbeute: ( 95% d.Th. ). MS: M/e=635.1; λₘₐₓ= 492.0nm

### Beispiel 125

**4',9,9'-trihydroxy-6'-methoxy-1,1',3',5',8'-pentaoxo-1,1',2,3',5',6,7,8'-octahydrospiro[cyclopenta[g]isoquinoline-8;2'-cyclopenta[b]naphthalene]-3-carbaldehydesemicarbazone 125**
Ausbeute: ( 95% d.Th. ). MS: M/e=559.1; λₘₐₓ= 492.0nm

### Beispiel 126

**2-piperidino-4-yl-N'-[(1E)-(4',9,9'-trihydroxy-6'-methoxy-1,1',3',-5',8'-pentaoxo-1,1',2,3',5',6,7,8'-octahydrospiro[cyclopenta[g]-isoquinoline-8,2'-cyclopenta[*b*]naphthalen]-3-yl)methylene]-acetohydrazide 126**
Ausbeute: ( 95% d.Th. ). MS: M/e=641.1; λₘₐₓ= 492.0nm

### Beispiel 127

**4'_{,}9,9'-trihydroxy-6'-methoxy-1,1',3',5',8'-pentaoxo-1,1',2,3',5',-6,7,8'-octahydrospiro[cyclopenta[*g*]isoquinoline-8,2'-cyclopenta[b]-naphthalene]-3-carbaldehyde *O*-(3-chlorobenzyl)oxime 127**
Ausbeute: ( 95% d.Th. ). MS: M/e=641.1; λₘₐₓ= 492.0nm

### Beispiel 128

**N'-[(lE)-(4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta[b]naphthalen]-3-yl)methylene]-(2-methyl-1,3-thiazole-4yl)-carbohydrazide 128**
Ausbeute: ( 95% d.Th. ). MS: M/e=641.1; λₘₐₓ= 492.0nm

### Beispiel 129

**.2-(1E-imidazol-1-yl)-N'-[(E)-(4',9,9'-trihydroxy-6'-methoxy-1,1',3',5',8'-pentaoxo-1,1',2,3',5',6,7,8'-octahydrospiro-[cyclopenta[g]isoquinoline-8,2'-cyclopenta-[*b*]naphthalen]-3-yl)methylene]acetohydrazide 129**
Ausbeute: ( 90% d.Th. ). MS: M/e=624.1; λₘₐₓ= 500.0nm

### Beispiel 130

**2-(acetylamino)-N'-[(1E)-(4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta[b]naphthalen]-3-yl)methylene]acetohydrazide 130**
Ausbeute: (95% d.Th.). MS: M/e=615.1; λₘₐₓ= 492.0nm

### Besipiel 131

**2-(4-methylpiperazin-1-yl)-N'-[(1E)-(9',9,9'-trihydroxy-6'-methoxy-1,1',3',5',8'-pentaoxo-1,1_{'},2,3',5',6,7,8'-octahydrospiro-[cyclopenta[*g*]isoquinoline-8,2'-cyclopenta[*b*]naphthalen]-3-yl)methylene]acetohydrazide 131**
Ausbeute: ( 50% d.Th. ). MS: M/e=656.1; λₘₐₓ= 492.0nm

### Beispiel 132

**2-morpholin-4-yl-N'-[(1E)-(4',9,9'-trihydroxy-6'-methoxy-1,1',3',-5',8'-pentaoxo-1,1',2,3',5',6,7,8'-octahydrospiro[cyclopenta[g]-isoquinoline-8,2'-cyclopenta[b]naphthalen]-3-yl)methylene]-acetohydrazide 132**
Ausbeute: ( 60% d.Th. ). MS: M/e=643.1; λₘₐₓ= 492.0nm

### Beispiel 133

**2-(methylamino)-N'-[(1E)-(4',9,9'-trihydroxy-6'-methoxy-1,1',3',5',8'-pentaoxo-1,1',2,3',5_{'},6,7,8'-octahydrospiro[cyclopenta[*g*]isoquinoline-8,2'-cyclopenta[b]naphthalen]-3-yl)methylene]acetohydrazide 133** Ausbeute ( 70% d.Th. ). Ms: M/e=587.1; λₘₐₓ= 492.0nm

### Beispiel 134

**2-[isopropyl(methyl)amino]-N'-[(1E)-(9',9,9'-trihydroxy-6'-methoxy-1,1',3',5',8'-pentaoxo-1,1',2,3',5',6,7,8'-octahydrospiro-[cyclopenta[*g*]isoquinoline-8,2'-cyclopenta[b]naphthalen]-3-yl)methylene]acetohydrazide 134**
Ausbeute: ( 70% d.Th. ). MS: M/e=629.1; λₘₐₓ= 492.0nm

### Beispiel 135

**4',9,9'-trihydroxy-6'-methoxy-1,1',3',5',8'-pentaoxo-1,1',2,3',5',-6,7,8'-octahydrospiro[cyclopenta[*g*]isoquinoline-8,2'-cyclopenta[b]-naphthalene]-3-carbaldehyde *O*-[2-(dimethylamino)ethyl]oxime 135**
Ausbeute: ( 90% d.Th. ). MS: M/e=588.1; λₘₐₓ= 492.0nm

### Beispiel 136

**4',9,9'-trihydroxy-6'-methoxy-1,1',3',5',8'-pentaoxo-1,1',2,3',5',-6,7,8'-octahydrospiro[cyclopenta[*g*]isoquinoline-8,2'-cyclopenta[*b*]-naphthalene]-3-carbaldehyde *O*-[3-(4-(3-chlorphenyl)-piperazin-1-yl)propyl]oxime 136**
Ausbeute: ( 85% d.Th. ). MS: M/e=753.1; λₘₐₓ= 492.0nm

### Beispiel 137

**4',9,9'-trihydroxy-6'-methoxy-1,1',3',5',8'-pentaoxo-1,1',2,3',5',-6,7,8'-octahydrospiro[cyclopenta[*g*]isoquinoline-8,2'-cyclopenta[b]-naphthalene]-3-carbaldehyde *O*-[3-(dimethylamino)propyl]oxime 137** Ausbeute: ( 70% d.Th. ). MS: M/e=602.1; λₘₐₓ= 492.0nm

### Beispiel 138

**(8S)-5-chloro-4',9,9'-trihydroxy-6'-methoxy-1,1',3',5',8'-pentaoxo-1,1',2,3',5',6,7,8'-octahydrospiro[cyclopenta[g]isoquinoline-8,2'-cyclopenta[b]naphthalene]-3-carbaldehydepyridin-2-yl-hydrazone 138** Ausbeute: ( 95% d.Th. ). MS: M/e=627.0; λₘₐₓ= 500.0nm

### Beispiel 139

**(8S)-5-chloro-4',9,9'-trihydroxy-6'-methoxy-1,1',3',5',8'-pentaoxo-1,1',2,3',5',6,7,8'-octahydrospiro[cyclopenta[g]isoquinoline-8,2'-cyclopenta[*b*]naphthalene]-3-carbaldehyde [4-(trifluoromethyl)pyrimidin-2-yl]hydrazone 139**
Ausbeute: ( 95% d.Th. ). MS: M/e=696.0; λₘₐₓ= 500.0nm

### Beispiel 140

**(8S)-5-chloro-N'-[(1E)-(4',9,9'-trihydroxy-6'-methoxy-1,1',3',5',8'-pentaoxo-1,1',2,3',5',6,7,8'-octahydrospiro [cyclopenta g]isoquinoline-8,2'-cyclopenta[b]naphthalen]-3-yl)methylene]pyridyl-3-carbohydrazide 140**
Ausbeute: ( 95% d.Th. ). MS: M/e=655.0; λₘₐₓ= 500.0nm

### Beispiel 141

**(8S)-5-chloro-N'-[(1E)-(4',9,9'-trihydroxy-6'-methoxy-1,1',3',5',8'-pentaoxo-1,1',2,3',5',6,7,8'-octahydrospiro [cyclopenta [*g*]-isoquinoline-8,2'-cyclopenta[b]naphthalen]-3-yl) methylene]-isonicotinohydrazide 141**
Ausbeute: ( 95% d.Th. ). MS: M/e=655.0; λₘₐₓ= 500.0nm

### Beispiel 142

**(8S)-5-chloro-4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta[b]naphthalene]-3-carbaldehyde-1,2,4-triazole-4-ylhydrazone 142**
Ausbeute: ( 90% d.Th. ). MS: M/e=602.0; λₘₐₓ= 500.0nm

### Beispiel 143

**(8S)-5-chloro-4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta[b]naphthalene]-3-carbaldehyde-4,5-dihydro-1H-imidazol-2-ylhydrazone 143**
Ausbeute: ( 95% d.Th. ). MS: M/e=618.01; λₘₐₓ= 500.0nm

### Beispiel 144

**(8S)-5-chloro-N'-((1E)-(4',9,9'-trihydroxy-6'-methoxy-1,1',3',5',8'-pentaoxo-1,1',2,3',5',6,7,8'-octahydrospiro[cyclopenta[*g*]-isoquinoline-8,2'-cyclopenta[b]naphthalen]-3-yl)methylene]-2-furohydrazide 144**
Ausbeute: ( 95% d.Th. ). MS: M/e=644.0; λₘₐₓ= 500.0nm

### Beispiel 145

**(8S)-5-chloro-4-amino-N'-[(lE)-(4',9,9'-trihydroxy-6'-methoxy-1,1',3',5',8'-pentaoxo-1,1',2,3',5',6,7,8'-tahydrospiro[cyclopenta-[*g*]isoquinoline-8,2'-cyclopenta[b]naphthalen]-3-yl)methylene]-benzohydrazide 145**
Ausbeute: ( 95% d.Th. ). MS: M/e=669.0; λₘₐₓ= 500.0nm

### Beispiel 146

**(8S)-5-chloro-4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta[*b*]naphthalene]-3-carbaldehydethiosemicarbazone 146**
Ausbeute: ( 95% d.Th. ). MS: M/e=609.0; λₘₐₓ= 500.0nm

### Beispiel 147

**(8S)-5-chloro-N'-[(1E)-(4',9,9'-trihydroxy-6'-methoxy-1,1',3',5',8'-pentaoxo-1,1',2,3',5',6,7,8'-octahydrospiro [cyclopenta[ g]isoquinoline-8,2'-cyclopenta[b]naphthalen]-3-yl)methylene]thiophene-2-carbohydrazide 147**
Ausbeute: ( 95% d.Th. ). MS : M/e=660.0; λₘₐₓ= 500.0nm

### Beispiel 148

**(8S)-5-chloro-2-(1H-indol-3-yl)-N'-[(1E)-(4',9,9'-trihydroxy-6'-methoxy-1,1',3',5',8'-pentaoxo-1,1',2,3',5',6,7,8'-octahydrospiro [cyclopenta[*g*]isoquinoline-8,2'-cyclopenta[*b*]naphthalen]-3-yl)methylene]acetohydrazide 148**
Ausbeute: ( 95% d.Th. ). MS: M/e=707.1; λₘₐₓ= 500.0nm

### Beispiel 149

**(8S)-5-chloro-4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta[*b*]naphthalene]-3-carbaldehyde(4-methylpiperazin-1-yl)hydrazone 149**
Ausbeute: ( 95% d.Th. ). MS: M/e=633.1; λₘₐₓ= 500.0nm

### Beispiel 150

**(8S)-5-chloro-2-oxo-2-((2E)-2-[(4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta[*b*]naphthalen]-3-yl)methylene]hydrazino}acetamide 150**
Ausbeute: ( 95% d.Th. ). MS: M/e=621.0; λₘₐₓ= 500.0nm

### Beispiel 151

**(8S)-5-chloro-4',9,9'-trihydroxy-6'-methoxy-3-{(E)-[(4-oxo-2-thioxo-1,3-thiazolidin-3-yl)imino]methyl}-6,7-dihydrospiro[g]isoquinoline-8,2'-cyclopenta[*b*]naphtalene)-1,1',3',5',8'(2H)-pentone 151**
Ausbeute: ( 95% d.Th. ). MS: M/e=665.3; λₘₐₓ= 500.0nm

### Beispiel 152

**(8S)-5-chloro-((2E)-2-[(4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta[*b*]naphthalen]-3-yl)methylene]hydrazino}acetonitrile 152**
Ausbeute: ( 95% d.Th. ). MS: M/e=617.1; λₘₐₓ= 500.0nm

### Beispiel 153

**(8S)-5-chloro-2-amino-N'-[(lE)-(4',9,9'-trihydroxy-6'-methoxy-1,1',3',5',8'-pentaoxo-1,1',2,3',5',6,7,8'-octahydrospiro [cyclopenta[*g*]isoquinoline-8,2'-cyclopenta[*b*]naphthalen]-3-yl)methylene]benzohydrazide 153**
Ausbeute: ( 95% d.Th. ). MS: M/e=669.1; λₘₐₓ= 500.0nm

### Beispiel 154

**(8S)-5-chloro-4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta[*b*]naphthalene]-3-carbaldehyde *O*-[2-morpholin-4-yl-ethyl)oxime 154**
Ausbeute: ( 95% d.Th. ). MS: M/e=664.1; λₘₐₓ= 500.0nm

### Beispiel 155

**(8S)-5-chloro-(2E)-2-[(4',9,9'-trihydroxy-6'-methoxy-1,1',3',5',8'-pentaoxo-1,1',2,3',5',6,7,8'-octahydrospiro [cyclopenta *g*]isoquinoline-8,2'-cyclopenta[b]naphthalen)-3-yl) methylene]hydrazinecarboximidamide 155**
Ausbeute: ( 95% d.Th. ). MS: M/e=592.1; λₘₐₓ= 500.0nm

### Beispiel 156

**(8S)-5-chloro-2-(dimethylamino)-*N*'-[(1E)-(4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta[b]naphthalen]-3-yl)methylene]acetohydrazide 156**
Ausbeute: ( 95% d.Th. ). MS: M/e=635.1; λₘₐₓ= 500.0nm

### Beispiel 157

**(8S)-5-chloro-1-[2-oxo-2-((2E)-2-{[(8S)-4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta[b] naphtalen-3-yllmethylene)hydrazino)ethyl]pyridinium chloride 157**
Ausbeute: ( 95% d.Th. ). MS: M/e=669.1; λₘₐₓ= 500.0nm

### Beispiel 158

**(8S)-5-chloro--4',9,9',-trihydroxy-6'-methoxy-1,1',3',5',8'-pentaoxol,1',2,3',5',6,7,8'-octahydrospiro [cyclopenta[*g*]-isoquinoline-8,2'-cyclopenta[b]naphthalene]-3-carbaldehyde O-methyloxime 158**
Ausbeute: ( 95% d.Th. ). MS: M/e=565.0; λₘₐₓ= 500.0nm

### Beispiel 159

**(8S)-5-chloro-4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta[b]naphthalene]-3-carbaldehyde-*O*-benzyloxime 159**
Ausbeute: ( 95% d.Th. ). MS: M/e=641.1; λₘₐₓ= 500.0nm

### Beispiel 160

**(8S)-5-chloro-4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta[b]naphthalene]₋3-carbaldehyde oxime 160**
Ausbeute: ( 95% d.Th. ). MS: M/e=551.0; λₘₐₓ= 500.0nm

### Beispiel 161

**(8S)-5-chloro-1-O-({(1E)-[(8S)-4',9,9',-trihydroxy-6'-methoxy-1,1',3',5',8'-pentaoxo 1,1',2,3',5',6,7,8'-octahydrospiro-[cyclopenta[g]isoquinoline-8,2'-cyclopenta[b]naphthalene]-3-yl]methylene)amino)-β-D-glucopyranose 161**
Ausbeute: ( 95% d.Th. ). MS: M/e=713.1; λₘₐₓ= 500.0nm

### Beispiel 162

**(8S)-5-chloro-4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta[*b*]naphthalene]-3-carbaldehyde-phenylsemicarbazone 162**
Ausbeute: ( 95% d.Th. ). MS: M/e=669.0; λₘₐₓ= 500.0nm

### Beispiel 163

**(8S)-5-chloro-4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta[b]naphthalene]-3-carbaldehydesemicarbazone 163**
Ausbeute: ( 90% d.Th. ). MS: M/e=593.0; λₘₐₓ= 500.0nm

### Beispiel 164

**(8S)-5-chloro-2-piperidino-4-yl-N'-[(1E)-(4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta[b]naphthalen]-3-yl)methylene]acetohydrazide 164**
Ausbeute: ( 95% d.Th. ). MS: M/e=675.1; λₘₐₓ= 500.0nm

### Beispiel 165

**(8S)-5-chloro-4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta[*b*]naphthalene]-3-carbaldehyde *O*-(3-chlorobenzyl)oxime 165**
Ausbeute: ( 90% d.Th. ). MS: M/e=675.0; λₘₐₓ= 500.0nm

### Beispiel 166

**(8S)-5-chloro-N'-[(1E)-(4',9,9'-trihydroxy-6'-methoxy-1,1',3',5',8'-pentaoxo-1,1',2,3',5',6,7,8'-octahydrospiro[cyclopenta[g]isoquinoline-8,2'-cyclopenta[b]naphthalen]-3-yl)methylene]-2-methyl-1,3-thiazole-4y1-carbohydrazide 166**
Ausbeute: ( 95% d.Th. ). MS: M/e=675.0; λₘₐₓ= 500.0nm

### Beispiel 167

**(8S)-5-ehloro-2-(1H-imidazol-1-yl)-*N*'-[(1*E*)-(4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta [b]naphthalen]-3-yl)methylene]acetohydrazide 167**
Ausbeute: ( 90% d.Th. ). MS: M/e=658.0: λₘₐₓ= 500.0nm

### Beispiel 168

**(8S)-5-chloro-2-(acetylamino)-*N*'-[(1*E*)-(4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta[*b*]naphthalen]-3-yl)methylene]acetohydrazide168**
Ausbeute: ( 95% d.Th. ). MS: M/e=649.0; λₘₐₓ= 500.0nm

### Beispiel 169

**(8S)-5-chloro-2-(4-methylpiperazin-1-yl)-*N*'-[(1*E*)-(4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta[b]naphthalen]-3-yl)methylene]acetohydrazide 169**
Ausbeute: ( 60% d.Th. ). MS: M/e=690.1; λₘₐₓ= 500.0nm

### Beispiel 170

**(8S)-5-chloro-2-morpholin-4-yl-*N*'-[(1*E*)-(4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta[*b*]naphthalen]-3-yl)methylene]acetohydrazide 170**
Ausbeute: ( 60% d.Th. ). MS: M/e=677.1; λₘₐₓ= 500.0nm

### Beispiel 171

**(8S)-5-chloro-2-(methylamino)-*N'*-[(1*E*)-(41,9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta[*b*]naphthalen]-3-yl)methylene]acetohydrazide 171** Ausbeute: ( 70% d.Th. ). MS: M/e=621.0; λₘₐₓ= 500.0nm

### Beispiel 172

**(8S)-5-chloro-2-[isopropyl(methyl)amino]-*N*'-[(1*E*)-(4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta [*b*]naphthalen]-3-yl)methylene]acetohydrazide 172**
Ausbeute: ( 70% d.Th. ). MS: M/e=663.1; λₘₐₓ= 500.0nm

### Beispiel 173

**(8S)-5-chloro-4',9,9'-trihydroxy-6'-methoxy-1,1',3',5',8'-pentaoxo-1,1',2,3',5'.,6,7,8'-octahydrospiro[cyclopenta[*g*]isoquinoline-8,2'-cyclopenta[b]naphthalene]-3-carbaldehyde *O*-[2-(dimethylamino)ethyl]-oxime 173**
Ausbeute: ( 60% d.Th. ). MS: M/e=622.0; λₘₐₓ= 500.0nm

### Beispiel 174

**(8S)-5-chloro-4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta[*b*]naphthalene]-3-carbaldehyde *O*-[3-(4-(3-chlorphenyl)-piperazin-1-yl)propyl]oxime 174**
Ausbeute: ( 90% d.Th. ). MS: M/e=787.1; λₘₐₓ= 500.0nm

### Beispiel 175

**(8S)-5-chloro-4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta[*b*]naphthalene]-3-carbaldehyde *O*-[3-(dimethylamino)propyl]oxime 175**
Ausbeute: ( 75% d.Th. ). MS: M/e=636.1; λₘₐₓ= 500.0nm

### Beispiel 176

**(8S)-5-bromo-4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta[*b*]naphthalene]-3-carbaldehydepyridin-2-yl-hydrazone 176** Ausbeute: ( 95% d.Th. ). MS: M/e=670.9; λₘₐₓ= 500.0nm

### Beispiel 177

**(8S)-5-bromo-4',9,9'-trihydroxy-6'-methoxy-1,1',3',5',8'-pentaoxo-1.,1',2,3',5',6,7,8'-octahydrospiro[cyclopenta[g]isoquinoline-8,2'-cyclopenta[b]naphthalene]-3-carbaldehyde [4-(trifluoromethyl)pyrimidin-2-yl]hydrazone 177**
Ausbeute: ( 95% d.Th. ) . MS: M/e=739.9 λₘₐₓ= 500.0nm

### Beispiel 178

**(8S)-5-bromo-N'-[(1E)-(4',9,9'-trihydroxy-6'-methoxy-1,1',3',5',8'-pentaoxo-1,1',2,3',5',6,7,8'-ctahydrospiro [cyclopenta [g]isoquinoline-8,2'-cyclopenta[b]naphthalen]-3-yl)methylene]pyridyl-3-carbohydrazide 178**
Ausbeute: ( 90 d.Th. ). MS: M/e=699.0 λₘₐₓ= 500.0nm

### Beispiel 179

**(8S)-5-bromo-N'-[(1E)-(4',9,9'-trihydroxy-6'-methoxy-1,1',3',5',8'-pentaoxo-1,1',2,3',5',6,7,8'-octahydrospiro cyclopenta[g]isoquinoline-8,2'-cyclopenta[b]naphthalen]-3-yl)methylene]isonicotinohydrazide 179**
Ausbeute: ( 90% d.Th. ). MS: M/e=699.0; λₘₐₓ= 500.0nm

### Beispiel 180

**(8S)-5-bromo-4',9,9'-trihydroxy-6'-methoxy-1,1',3',5',8'-pentaoxo-1,1',2,3',5',6,7,8'-octahydrospiro[cyclopenta[g]isoquinoline-8,2'-cyclopenta[b]naphthalene]-3-carbaldehyde-1,2,4-triazole-4-ylhydrazone 180**
Ausbeute: ( 70% d.Th. ) . MS: M/e=645.9; λₘₐₓ= 492.0nm

### Beispiel 181

**(8S)-S-bromo-4',9,9'-trihydroxy-6'-methoxy-1,1',3',5',8'-pentaoxo-1,1',2,3',5',6,7,8'-octahydrospiro[cyclopenta[g]isoquinoline-8,2'-cyclopenta[b]naphthalene]-3-carbaldehyde-4,5-dihydro-1H-imidazol-2-ylhydrazone 181**
Ausbeute: ( 95% d.Th. ). Ms: M/e=662.0; λₘₐₓ= 492.0nm

### Beispiel 182

**(8S)-5-bromo-N'-[(1E)-(4',9,9'-trihydroxy-6'-methoxy-1,1',3',5',8'-pentaoxo-1,1',2,3',5',6,7,8'-octahydrospiro [cyclopenta[g]isoquinoline-8,2'-cyclopenta[b]naphthalen]-3-yl)methylene]-2-furohydrazide 182**
Ausbeute: ( 95% d.Th. ) . MS: M/e=688.9; λₘₐₓ= 492.0nm

### Beispiel 183

**(8S)-5-bromo-4-amino-*N*'-[(1*E*)-(4',9,9'-trihydroxy-6'-methoxy-1,1',3',5',8'-pentaoxo-1,1',2,3',5',6,7,8'-tahydrospiro[cyclopenta[g]isoquinoline-8,2'-cyclopenta[b]naphthalen]-3-yl)methylene]benzohydrazide 183**
Ausbeute: ( 95% d.Th. ). MS : M/e=713.0; λₘₐₓ= 500.0nm

### Beispiel 184

**(8S)-5-bromo-4',9,9'-trihydroxy-6'-methoxy-1,1',3',5',8'-pentaoxo-1,1',2,3',5-,6,7,8'-octahydrospiro[cyclopenta[g]isoquinoline-8,2'-cyclopenta[b]naphthalene]-3-carbaldehydethiosemicarbazone 184**
Ausbeute: ( 95% d.Th. ). MS: M/e=653.0; λₘₐₓ= 500.0nm

### Beispiel 185

**(8S)-5-bromo-N'-[(1E)-(4',9,9'-trihydroxy-6'-methoxy-1,1',3',5',8'-pentaoxo-1,1',2,3',5',6,7,8'-octahydrospiro [cyclopenta[g]isoquinoline-8,2'-cyclopenta[b]naphthalen]-3-yl)methylene]thiophene-2-carbohydrazide 185**
Ausbeute: ( 95% d.Th. ). MS: M/e=704.0; λₘₐₓ= 492.0nm

### Beispiel 186

**(BS)-5-bromo-2-(1*H*-indol-3-yl)-*N'*-[(1E)-(4',9,9'-trihydroxy-6'-methoxy-1,1',3',5',8'-pentaoxo-1,1',2,3',5',6,7,8'-octahydrospiro [cyclopenta[g]isoquinoline-8,2'-cyclopenta[b]naphthalen]-3-yl)methylene]acetohydrazide 186**
Ausbeute: ( 95% d.Th. ). MS: M/e=751.1; λₘₐₓ= 500.0nm

### Beispiel 187

**(BS)-5-bromo-4',9,9'-trihydroxy-6'-methoxy-1,1',3',5',8'-pentaoxo-1,1',2,3',5',6,7,8'-oetahydrospiro[cyclopenta[*g*]isoquinoline-8,2'-cyclopenta[b]naphthalene]-3-carbaldehyde(4-methylpiperazin-1-yl)hydrazone 187**
Ausbeute: ( 95% d.Th. ). MS: M/e=677.1; λₘₐₓ= 500.0nm

### Beispiel 188

**(8S)-5-bromo-2-oxo-2-{(2*E*)-2-[(4',9,9'-trihydroxy-6'-methoxy-1,1',3_{'},5_{'},8_{'}-pentaoxo-1,1',2,3',5',6,7,8'-octahydrospiro[cyclopenta[*g*]isoquinoline-8,2'-cyclopenta[b]naphthalen]-3-yl)methylene]hydrazino}acetamide 188**
Ausbeute: ( 95% d.Th. ). MS: M/e=665.0; λₘₐₓ= 500.0nm

### Beispiel 189

**(8S)-5-bromo-4',9,9'-trihydroxy-6'-methoxy-3-{(E)-[(4-oxo-2-thioxo-1,3-thiazolidin-3-yl)imino]methyl}-6,7-dihydrospiro[g]isoquinoline-8,2'-cyclopenta[b]naphtalene]-1,1',3',5',8'(2H)-pentone 189**
Ausbeute: ( 95% d.Th. ). MS: M/e=709.9; λₘₐₓ= 492.0nm

### Beispiel 190

**(8S)-5-bromo-{(2E)-2-[(4',9,9'-trihydroxy-6'-methoxy-1,1',3',5',8'-pentaoxo-1,1',2,3',5',6,7,8'-octahydrospiro [cyclopenta[g]isoquinoline-8,2'-cyclopenta[b]naphthalen]-3-yl)methylene]hydrazino}acetonitrile 190**
Ausbeute: ( 95% d.Th. ). MS: M/e=661.0; λₘₐₓ= 500.0nm

### Beispiel 191

**(8S)-5-bromo-2-amino-*N*'-[(1*E*)-(4',9,9'-trihydroxy-6'-methoxy-1,1',3',5',8'-pentaoxo-1,1',2,3',5',6,7,8'-octahydrospiro[cyclopenta[g]isoquinoline-8,2'-cyclopenta[b]naphthalen]-3-yl)methylene]benzohydrazide 191**
Ausbeute: ( 70% d.Th. ). MS: M/e=713.0; λₘₐₓ= 492.0nm

### Beispiel 192

**(8S)-5-bromo-4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta[*b*]naphthalene]-3-carbaldehyde *O*-[2-morpholin-4-yl-ethyl)oxime 192**
Ausbeute: ( 95% d.Th. ). MS: M/e=708.0; λₘₐₓ= 500.0nm

### Beispiel 193

**(8S)-5-bromo-(2*E*)-2-[(4',9,9'-trihydroxy-6'-methoxy-1,1',3',5',8'-pentaoxo-1,1',2,3',5',6,7,8'-octahydrospiro [cyclopenta[g]isoquinoline-8,2'-cyclopenta[b]naphthalen]-3-yl)methylene]hydrazinecarboximidamide 193**
Ausbeute: ( 95% d.Th. ) . MS: M/e=636.0; λₘₐₓ= 500.0nm

### Beispiel 194

**(8S)-5-bromo-2-(dimethylamino)-*N*'-[(1E)-(4',9,9'-trihydroxy-6'-methoxy-1,1',3',5',8'-pentaoxo-1,1',2,3',5',6,7,8'-octahydrospiro[cyclopenta[*g*] isoquinoline-8,2'-cyclopenta[b]naphthalen]-3-yl)methylene]acetohydrazide 194**
Ausbeute: ( 95% d.Th. ). MS: M/e=679.0; λₘₐₓ= 500.0nm

### Beispiel 195

**(8S)-5-bromo-1-[2-oxo-2-((2E)-2-{[(8S)-4',9,9'-trihydroxy-6'-methoxy-1,1',3',5',8'-pentaoxo-1,1',2,3',5',6,7,8'-octahydrospiro[cyclopenta[g]isoquinoline-8,2'-cyclopenta[b] naphtalen-3-yl]methylene}hydrazino)ethyl]pyridinium chloride 195**
Ausbeute: ( 95% d.Th. ) . MS: M/e=713.0; λₘₐₓ= 500.0nm

### Beispiel 196

**(8S)-5-bromo--4',9,9',-trihydroxy-6'-methoxy-1,1',3',5',8'-pentaoxo-1,1',2,3',5',6,7,8'-octahydrospiro [cyclopenta[g]isoquinoline-8,2'-cyclopenta[b]naphthalene]-3-carbaldehyde O-methyloxime 196**
Ausbeute: ( 95% d.Th. ). MS: M/e=609.0; λₘₐₓ= 492.0nm

### Beispiel 197

**(8S)-5-bromo-4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta[*b*]naphthalene]-3-carbaldehyde-*O*-benzyloxime 197**
Ausbeute: ( 95% d.Th. ) . MS: M/e=685.0; λₘₐₓ= 492.0nm

### Beispiel 198

**(8S)-5-bromo-4',9,9'-trihydroxy-6'-methoxy-1,1',3',5',8'-pentaoxo-1,1',2,3',5',6,7,8'-octahydrospiro[cyclopenta[g]isoquinoline-8,2'-cyclopenta[b]naphthalene]-3-carbaldehyde oxime 198**
Ausbeute: ( 95% d.Th. ). MS: M/e=595.0; λₘₐₓ= 492.0nm

### Beispiel 199

**(SS)-5-bromo-1-O-({(1E)-[(8S)-4',9,9',-trihydroxy-6'-methoxy-1,1',3',5',8'-pentaoxo 1,1',2,3',5',6,7,8'-octahydrospiro cyclopenta[g]isoquinoline-8,2'-cyclopenta[b]naphthalene]-3-yl]methylene}amino)-β-D-glucopyranose 199**
Ausbeute: ( 90% d.Th. ). MS: M/e=757.0; λₘₐₓ= 500.0nm

### Beispiel 200

**(8S)-5-bromo-4',9,9'-trihydroxy-6'-methoxy-1,1',3',5',8'-pentaoxo-1,1',2,3',5',6,7,8'-octahydrospiro[cyclopenta[g]isoquinoline-8,2'-cyclopenta[b]naphthalene]-3-carbaldehyde-phenylsemicarbazone 200**
Ausbeute: ( 90% d.Th. ). MS: M/e=713.0; λₘₐₓ= 500.0nm

### Beispiel 201

**(8S)-5-bromo-4',9,9'-trihydroxy-6'-methoxy-1,1',3',5',8'-pentaoxo-1,1',2,3',5',6,7,8'-octahydrospiro[cyclopenta[g]isoquinoline-8,2'-cyclopenta[b]naphthalene]-3-carbaldehydesemicarbazone 201**
Ausbeute: ( 90% d.Th. ). MS: M/e=637.0; λₘₐₓ= 492.0nm

### Beispiel 202

**(8S)-5-bromo-2-piperidino-4-yl-N'-[(1E)-(4',9,9'-trihydroxy-6'-methoxy-1,1',3',5',8'-pentaoxo-1,1',2,3',5',6,7,8'-octahydrospiro[cyclopenta[g]isoquinoline-8,2'-cyclopenta[b]naphthalen]-3-yl)methylene]acetohydrazide 202**
Ausbeute: ( 90% d.Th. ). MS: M/e=719.0; λₘₐₓ= 500.0nm

### Beispiel 203

**(8S)-5-bromo-4',9,9'-trihydroxy-6'-methoxy-1,1',3',5',8'-pentaoxo-1,1',2,3',5',6,7,8'-octahydrospiro[cyclopenta[g]isoquinoline-8,2'-cyclopenta[b]naphthalene]-3-carbaldehyde O-(3-chlorobenzyl)oxime 203**
Ausbeute: ( 95% d.Th. ). MS: M/e=718.0; λₘₐₓ= 492.0nm

### Beispiel 204

**(8S)-5-bromo-N'-[(1E)-(4',9,9'-trihydroxy-6'-methoxy-1,1',3',5_{'},8_{'}-pentaoxo-1,1',2,3',5',6,7,8'-octahydrospiro [cyclopenta[g]isoquinoline-8,2'-cyclopenta[b]naphthalen]-3-yl)methylene]-2-methyl-1,3-thiazole-4yl-carbohydrazide 204**
Ausbeute: ( 95% d.Th. ). MS: M/e=718.9; λₘₐₓ= 492.0nm

### Beispiel 205

**(8S)-5-bromo-2-(1*H*-imidazol-1-yl)-*N*'-[(1*E*)-(4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta [b]naphthalen]-3-yl)methylene]acetohydrazide 205**
Ausbeute: ( 95% d.Th. ). MS: M/e=702.0; λₘₐₓ= 500.0nm

### Beispiel 206

**(8S)-5-bromo-2-(acetylamino)-N'-[(lE)-(4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta[*b*]naphthalen]-3-yl)methylene]acetohydrazide 206**
Ausbeute: ( 95% d.Th. ). MS: M/e=693.0; λₘₐₓ= 492.0nm

### Beispiel 207 ,

**(8S)-5-bromo-2-(4-methylpiperazin-1-yl)-*N*'-[(1*E*)-(4',9,9'-trihydroxy-6'-methoxy-1,1',3',5',8'-pentaoxo-1,1',2,3',5',6,7,8'-octahydrospiro[cyclopenta[g]isoquinoline-8,2'-cyclopenta[b]naphthalen]-3-yl)methylene]acetohydrazide 207**
Ausbeute: ( 90% d.Th. ). MS: M/e=734.1; λₘₐₓ= 500.0nm

### Beispiel 208

**(8S)-5-bromo-2-morpholin-4-yl-*N*'-[(1E)-(4',9,9'-trihydroxy-6'-methoxy-1,1',3',5',8'-pentaoxo-1,1',2,3',5',6,7,8'-octahydrospiro[cyclopenta[g]isoquinoline-8,2'-cyclopenta[b]naphthalen]-3-yl)methylene]acetohydrazide 208**
Ausbeute: ( 95% d.Th. ). MS: M/e=721.1; λₘₐₓ= 500.0nm

### Beispiel 209

**(8S)-5-bromo-2-(methylamino)-*N*'-[(1*E*)-(4',9,9'-trihydroxy-6'-methoxy-1,1',3',5',8'-pentaoxo-1,1',2,3',5',6,7,8'-octahydrospiro[cyclopenta[g]isoquinoline-8,2'-cyclopenta[b]naphthalen]-3-yl)methylene]acetohydrazide 209** Ausbeute: ( 95% d.Th. ). MS: M/e=665.0; λₘₐₓ= 500.0nm

### Beispiel 210

**(8S)-5-bromo-2-[isopropyl(methyl)amino]-*N*'-[(1*E*)-(4',9,9'-trihydroxy-6'-methoxy-1,1',3',5',8'-pentaoxo-1,1',2,3',5',6,7,8'-octahydrospiro[cyclopenta[g]isoguinoline-8,2'-cyclopenta[*b*]naphthalen]-3-yl)methylene]acetohydrazide 210**
Ausbeute: ( 90% d.Th. ). MS: M/e=707.0; λₘₐₓ= 500.0nm

### Beispiel 211

**(8S)-5-bromo-4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta[*b*]naphthalene]-3-carbaldehyde *O*-[2-(dimethylamino)ethyl]oxime 211**
Ausbeute: ( 95% d.Th. ). MS: M/e=666.0; λₘₐₓ= 500.0nm

### Beispiel 212

**(8S)-5-bromo-4',9,9'-trihydroxy-6'-methoxy-1,1',3',5',8'-pentaoxo-1,1',2,3',5',6,7,8'-octahydrospiro[cyclopenta[g]isoquinoline-8,2'-cyclopenta[b]naphthalene]-3-carbaldehyde *O*-[3-(4-(3-ehlorphenyl)-piperazin-1-yl)propyl]oxime 212**
Ausbeute: ( 95% d.Th. ). MS: M/e=831.0; λₘₐₓ= 500.0nm

### Beispiel 213

**(8S)-5-bromo-4',9,9'-trihydroxy-6'-methoxy-1,1',3',5',8'-pentaoxo-1,1',2,3',5',6,7,8'-octahydrospiro[cyclopenta[g]isoquinoline-8,2'-cyclopenta[*b*]naphthalene]-3-carbaldehyde *O*-[3-(dimethylamino)propyl]oxime 213**
Ausbeute: ( 95% d.Th. ). MS: M/e=680.0; λₘₐₓ= 492.0nm

### Beispiel 214

**(8S)-4-,9,9-,-trihydroxy-6'-methoxy-1,1',3',5',8'-pentaoxo-1,1',2,3',5',6,7,8'-octahydrospiro [cyclopenta[g]isoquinoline-8,2'-cyclopenta[b]naphthalene]-3-carbaldehyde O-isopropyloxime 214** Ausbeute: ( 99% d.Th. ). MS: M/e=559.2; λₘₐₓ= 500.0nm

### Beispiel 215

**(8S)-4',9,9',-trihydroxy-6'-methoxy-1,1',3',5',8'-pentaoxol,1',2-,3',5',6,7,8'-octahydrospiro [cyclopenta[g]isoquinoline-8,2'-cyclopenta[b]naphthalene]-3-carbaldehyde O-n-hexyloxime 215**
Ausbeute: ( 99% d.Th. ). MS: M/e=601.3; λₘₐₓ= 500.0nm.

### Beispiel 216

**(8S)-4',9,9',-trihydroxy-6'-methoxy-1,1',3',5',8'-pentaoxol,1',2-,3',5',6,7,8'-octahydrospiro [cyclopenta[g]isoquinoline-8,2'-cyclopenta[b]naphthalene]-3-carbaldehyde *O*-(4-fluorobenzyl)oxime 216**
Ausbeute: ( 99% d.Th. ). MS: M/e=625.2; λₘₐₓ= 500.0nm

### Beispiel 217

**(8S)-4',9,9',-trihydroxy-6'-methoxy-1,1',3',5',8'-pentaoxol,1',2-,3',5',6,7,8'-octahydrospiro [cyclopenta[g]isoquinoline-8,2'-cyclopenta[b]naphthalene]-3-carbaldehyde O-(4-chlorobenzyl)oxime 217**
Ausbeute: ( 99% d.Th. ) . MS: M/e=641.2; λₘₐₓ= 500.0nm

### Beispiel 218

**(8S)-4',9,9',-trihydroxy-6'-methoxy-1,1',3',5',8'-pentaoxol,1',2,-3',5',6,7,8'-octahydrospiro [cyclopenta[g]isoquinoline-8,2'-cyclopenta[b]naphthalene]-3-carbaldehyde O-(3-fluorobenzyl)oxime 218**
Ausbeute: ( 99% d.Th. ). MS: M/e=625.3; λₘₐₓ= 500.0nm

### Beispiel 219

**(8S)-5-chloro-4',9,9',-trihydroxy-6'-methoxy-1,1',3',5',8'-pentaoxo-1,1',2,3',5',6,7,8'-octahydrospiro [cyclopenta[g]isoquinoline-8,2'-cyclopenta[b]naphthalene]-3-carbaldehyde *O*-isopropyloxime 219**
Ausbeute: ( 80% d.Th. ) . MS: M/e=593.2; λₘₐₓ= 500.0nm

### Beispiel 220

**(8S)-5-chloro-4',9,9',-trihydroxy-6'-methoxy-1,1',3',5',8'-pentaoxol,1',2,3',5',6,7,8'-octahydrospiro [cyclopenta[g]isoquinoline-8,2'-cyclopenta[b]naphthalene]-3-carbaldehyde O-n-hexyloxime 220**
Ausbeute: ( 90% d.Th. ). MS: M/e=635.3; λₘₐₓ= 500.0nm

### Beispiel 221

**(8S)-5-chloro-4',9,9',-trihydroxy-6'-methoxy-1,1',3',5',8'-pentaoxol,1',2,3',5',6,7,8'-octahydrospiro [cyclopenta[g]isoquinoline-8,2'-cyclopenta[b]naphthalene]-3-carbaldehyde O-(4-fluorobenzyl)oxime 221**
Ausbeute: ( 85% d.Th. ). MS: M/e=659.3; λₘₐₓ= 500.0nm

### Beispiel 222

**(SS)-5-chloro-4',9,9',-trihydroxy-6'-methoxy-1,1',3',5',8'-pentaoxol,1',2,3',5',6,7,8'-octahydrospiro [cyclopenta[g]isoquinoline-8,2'-cyclopenta[b]naphthalene]-3-carbaldehyde O-(4-chlorobenzyl)oxime 222**
Ausbeute: (80% d.Th. ). MS: M/e=675.3; λₘₐₓ= 500.0nm

### Beispiel 223

**(8S)-5-chloro-4',9,9',-trihydroxy-6'-methoxy-1,1',3',5',8'-pentaoxol,1',2,3',5',6,7,8'-octahydrospiro [cyclopenta[g]isoquinoline-8,2'-cyclopenta[b]naphthalene]-3-carbaldehyde O-(3-fluorobenzyl)oxime 223**
Ausbeute: ( 80% d.Th. ). MS: M/e=659.3; λₘₐₓ= 500.0nm

### Beispiel 224

**(8S)-5-bromo-4',9,9',-trihydroxy-6'-methoxy-1,1',3',5',8'-pentaoxo1,1',2,3',5',6,7,8'-octahydrospiro [cyclopenta[g]isoquinoline-8,2'-cyclopenta[b]naphthalene]-3-carbaldehyde O-isopropyloxime 224**
Ausbeute: ( 90% d.Th. ). MS: M/e=639.3; λₘₐₓ= 492.0nm

### Beispiel 225

**(8S)-5-bromo-4',9,9',-trihydroxy-6'-methoxy-1,1',3',5',8'-pentaoxol,1',2,3',5',6,7,8'-octahydrospiro [cyclopenta[g]isoquinoline-8,2'-cyclopenta[b]naphthalene]-3-carbaldehyde O-n-hexyloxime 225**
Ausbeute: ( 95% d.Th. ). MS: M/e=679.3; λₘₐₓ= 492.0nm

### Beispiel 226

**(8S)-5-bromo-4',9,9',-trihydroxy-6'-methoxy-1,1',3',5',8'-pentaoxol,1',2,3',5',6,7,8'-octahydrospiro [cyclopenta[g]isoquinoline-8,2'-cyclopenta[b]naphthalene]-3-carbaldehyde O-(4-fluorobenzyl)oxime 226**
Ausbeute: ( 95% d.Th. ). MS: M/e=703.3; λₘₐₓ= 492.0nm

### Beispiel 227

**(8S)-5-bromo-4',9,9',-trihydroxy-6'-methoxy-1,1',3',5',8'-pentaoxol,1',2,3',5',6,7,8'-octahydrospiro [cyclopenta[g]isoquinoline-8,2'-cyclopenta[b]naphthalene]-3-carbaldehyde O-(4-chlorobenzyl)oxime 227**
Ausbeute: ( 95% d.Th. ). MS: M/e=719.3: λₘₐₓ= 492.0nm

### Beispiel 228

**(8S)-5-bromo-4',9,9',-trihydroxy-6'-methoxy-1,1',3',5',8'-pentaoxol,1',2,3',5',6,7,8'-octahydrospiro [cyclopenta[g]isoquinoline-8,2'-cyclopenta[b]naphthalene]-3-carbaldehyde O-(3-fluorobenzyl)oxime 228**
Ausbeute: ( 95% d.Th. ). MS: M/e=705.3; λₘₐₓ= 492.0nm

### Beispiel 229

**(8S)-5-iodo-4',9,9',-trihydroxy-6'-methoxy-1,1',3',5',8'-pentaoxol,1',2,3',5',6,7,8'-octahydrospiro [cyclopenta[g]isoquinoline-8,2'-cyclopenta[b]naphthalene]-3-carbaldehyde O-isopropyloxime 229**
Ausbeute: ( 99% d.Th. ). MS: M/e=685.3; λₘₐₓ= 500.0nm

### Beispiel 230

**(8S)-5-iodo-4',9,9',-trihydroxy-6'-methoxy-1,1',3',5',8'-pentaoxol,1',2,3',5',6,7,8'-octahydrospiro [cyclopenta[g]isoquinoline-8,2'-cyclopenta[b]naphthalene]-3-carbaldehyde O-n-hexyloxime 230**
Ausbeute: ( 99% d.Th. ). MS: M/e=727.4; λₘₐₓ= 500.0nm

### Beispiel 231

**(8S)-5-iodo-4',9,9',-trihydroxy-6'-methoxy-1,1',3',5',8'-pentaoxol,1',2,3',5',6,7,8'-octahydrospiro [cyclopenta[g]isoquinoline-8,2'-cyclopenta[b]naphthalene]-3-carbaldehyde O-(4-fluorobenzyl)oxime 231**
Ausbeute: ( 99% d.Th. ). MS: M/e=751.3; λₘₐₓ= 500.0nm

### Beispiel 232

**(8S)-5-iodo-4',9,9',-trihydroxy-6'-methoxy-1,1',3',5',8'-pentaoxol,1',2,3',5',6,7,8'-octahydrospiro [cyclopenta[g]isoquinoline-8,2'-cyclopenta[b]naphthalene]-3-carbaldehyde O-(4-chlorobenzyl)oxime 232**
Ausbeute: ( 99% d.Th. ) . MS: M/e=767.3; λₘₐₓ= 500.0nm

### Beispiel 233

**(8S)-5-iodo-4',9,9',-trihydroxy-6'-methoxy-1,1',3',5',8'-pentaoxol,1',2,3',5',6,7,8'-octahydrospiro. [cyclopenta[g]isoquinoline-8,2'-cyclopenta[b]naphthalene]-3-carbaldehyde O-(3-fluorobenzyl)oxime 233**
Ausbeute: ( 99% d.Th. ). MS: M/e=751.3; λₘₐₓ= 500.0nm

### Beispiel 234

**(8S)-5-iodo-4',9,9',-trihydroxy-6'-methoxy-1,1',3',5',8'-pentaoxol,1',2,3',5',6,7,8'-octahydrospiro [cyclopenta[g]isoquinoline-8,2'-cyclopenta[b]naphthalene]-3-carbaldehyde O-benzyloxime 234**
Ausbeute: ( 99% d.Th. ). MS: M/e=733.3; λₘₐₓ= 500.0nm

### Beispiel 235

**(8S)-5-iodo-4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta[*b*]naphthalene]-3-carbaldehyde *O*-[2-morpholin-4-yl-ethyl)oxime 235**
Ausbeute: ( 99% d.Th. ). MS: M/e=756.3; λₘₐₓ= 500.0nm

### Beispiel 236

**(8S)-5-iodo--4',9,9',-trihydroxy-6'-methoxy-1,1',3',5',8'-pentaoxol,1',2,3',5',6,7,8'-octahydrospiro [cyclopenta[g]isoquinoline-8,2'-cyclopenta[b]naphthalene]-3-carbaldehyde O-methyloxime 236**
Ausbeute: ( 95% d.Th. ) . MS: M/e=657.3; λₘₐₓ= 492.0nm

### Beispiel 237

**(8S)-5-iodo-4',9,9'-trihydroxy-6'₋methoxy-1,1',3',5',8'-pentaoxo-1,1',2,3',5',6,7,8'-octahydrospiro[cyclopenta[*g*]isoquinoline-8,2'-cyclopenta[b]naphthalene]-3-carbaldehyde *O*-(3-chlorobenzyl)oxime 237**
Ausbeute: ( 99% d.Th. ). MS: M/e=767.3; λₘₐₓ= 492.0nm

### Beispiel 238

**(8S)-5-iodo-4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta[*b*]naphthalene]-3-carbaldehyde *O*-[3-(4-(3-chlorphenyl)-piperazin-1-yl)propyl]oxime 238**
Ausbeute: ( 99% d.Th. ). MS: M/e=879.4; λₘₐₓ= 500.0nm

### Beispiel 239

**(8S)-5-iodo-4',9,9'-trihydroxy-6'-methoxy-1,1',3',5',8'-pentaoxo-1,1',2,3_{'},5',6,7,8'-octahydrospiro[cyclopenta[g]isoquinoline-8,2'-cyclopenta[b]naphthalene]-3-carbaldehyde oxime 239**
Ausbeute: ( 99% d.Th. ). MS: M/e=643.3; λₘₐₓ= 492.0nm

### Beispiel 240

**(8S)-5-iodo-2-(4-methylpiperazin-1-yl)-*N*'-[(1*E*)-(4',9,9'-trihydroxy-6'-methoxy-1,1',3',5',8'-pentaoxo-1,1',2,3',5',6,7,8'-octahydrospiro[cyclopenta[*g*]isoquinoline-8,2'-cyclopenta[b]naphthalen]-3-yl)methylene]acetohydrazide 240**
Ausbeute: ( 99% d.Th. ). MS: M/e=782.3; λₘₐₓ= 500.0nm

### Beispiel 241

**(8S)-5-iodo-2-morpholin-4-yl-*N'*-[(1*E*)-(4',9,9'-trihydroxy-6'-methoxy-1,1',3',5',8'-pentaoxo-1,1',2,3_{'},5',6,7,8'-octahydrospiro[cyclopenta[*g*]isoquinoline-8,2'-cyclopenta[*b*]naphthalen]-3-yl)methylene]acetohydrazide 241**
Ausbeute: ( 99% d.Th. ). MS: M/e=782.3; λₘₐₓ= 500.0nm

### Beispiel 242

**(8S)-5-iodo-2-oxo-2-{(2*E*)-2-[(4',9,9'-trihydroxy-6'-methoxy-1,1',3',5',8'-pentaoxo-1,1',2,3',5',6,7,8'-octahydrospiro-[cyclopenta[*g*]isoquinoline-8,2'-cyclopenta[*b*]naphthalen]-3-yl)methylene]hydrazino}acetamide 242**
Ausbeute: ( 99% d.Th. ). MS: M/e=713.3; λₘₐₓ= 500.0nm

### Beispiel 243

**(8S)-4',9.9'-trihydroxy-6'-ethoxy-3-[(1E,3E)-penta-1,3-dienyl]-6,7-dihydrospiro[cyclopenta[g]isoquinoline-8,2'-cyclopenta[b]naphtalene]-1,1'-3',5',8'(2H)-pentone (243)** 5mg (0.0095mmol) Fredericamycin (1)werden in 2.0ml Ethanol suspendiert. Unter N2-Atmosphäre werden 90mg Natriumacetat hinzugefügt und unter Rückfluss gekocht. Nach wenigen Minuten geht die Suspension in eine tiefblaue Lösung über. Nach 24h lässt man abkühlen, gibt auf Wasser und schüttelt mit Essigester (0.1% CF3COOH) aus. Nach dem trocknen und einengen hinterbleibt ein chromatographisch einheitliches rotes Pulver. Ausbeute: 5.0mg ( 97%.d.Th.); MS: 554 (M+H)+; λₘₐₓ= 504.0nm
**Beispiel 244**

**(8S)-4',9.9'-trihydroxy-6'-n-butoxy-3-[(1E,3E)-penta-1,3-dienyl]-6,7-dihydrospiro[cyclopenta[g]isoquinoline-8,2'-cyclopenta[b]naphtalene]-1,1'-3',5',8'(2H)-pentone (244)** 6mg (0.0114mmol) Fredericamycin (1)werden in 3.0ml n-Butanol suspendiert. Unter N2-Atmosphäre werden 50mg Kaliumacetat hinzugefügt und auf 100oC erwärmt. Nach wenigen Minuten geht die Suspension in eine tiefblaue Lösung über. Man lässt 1h bei dieser Temperatur und lässt abkühlen. Man gibt auf Wasser und schüttelt mit Essigester (0.1% CF3COOH) aus. Nach dem trocknen und einengen hinterbleibt ein chromatographisch einheitliches rotes Pulver. Ausbeute: 6.2mg ( 96%.d.Th.); MS: 582 (M)+; λₘₐₓ= 500.0nm

### Beispiel 245

**(8S)-4',9.9'-trihydroxy-6'-isopropyloxy-3-[(1E,3E)-penta-1,3-dienyl]-6,7-dihydrospiro[cyclopenta[g]isoquinoline-8,2'-cyclopenta[b)naphtalenel-1,1'-3',5',8'(2B)-pentone (245)** 5mg (0.0095mmol) Fredericamycin (1)werden in 3.0ml n-Propanol suspendiert. Unter N2-Atmosphäre werden 50mg Kaliumacetat (wasserfrei) hinzugefügt und auf 80oC erwärmt. Nach wenigen Minuten geht die Suspension in eine tiefblaue Lösung über. Man lässt 48h bei dieser Temperatur und lässt abkühlen. Man gibt auf Wasser und schüttelt mit Essigester (0.1% CF3COOH) aus. Nach dem trocknen und einengen hinterbleibt ein chromatographisch einheitliches rotes Pulver.
Ausbeute: 3.7mg ( 70%.d.Th.); MS: 568 (M+H)+; λₘₐₓ= 500.0nm

### Beispiel 246

**(8S)-4',9.9'-trihydroxy-6'-(2-dimethylaminoethoxy)-3-[(1E,3E)-penta-1,3-dienyl]-6,7-dihydrospiro[cyclopenta[g]isoquinoline-8,2'-cyclopenta[b]naphtalene]-1,1'-3',5',8'(2H)-pentone (246)** 6.1mg (0.01159mmol) Fredericamycin (1)werden in 3.5ml N,N-Dimethylaminoethanol suspendiert. Unter N2-Atmosphäre werden 52mg Kaliumacetat (wasserfrei) hinzugefügt und auf 80oC erwärmt. Nach wenigen Minuten geht die Suspension in eine tiefblaue Lösung über. Man lässt 1.5h bei dieser Temperatur und lässt abkühlen. Man gibt auf Wasser und schüttelt mit Essigester (0.1% CF3COOH) aus. Nach dem trocknen und einengen hinterbleibt ein chromatographisch einheitliches rotes Pulver. Ausbeute: 2.4mg ( 36%.d.Th.); MS: 597 (M+H)+; λₘₐₓ= 504.0nm
**Beispiel 247**

**(8S)-5-bromo-4',9.9'-trihydroxy-6'-ethoxy-3-[(1E,3E)-penta-1,3-dienyl]-6,7-dihydrospiro[cyclopenta[g]isoquinoline-8,2'-cyclopenta[b]naphtalene]-1,1'-3',5',8'(2H)-pentone (247)** 10.0mg (0.019mmol) Bromo Fredericamycin (14) werden in 3.0ml Ethanol suspendiert. Unter N2-Atmosphäre werden 50mg Kaliumacetat (wasserfrei) hinzugefügt und auf 80oC erwärmt. Nach wenigen Minuten geht die Suspension in eine tiefblaue Lösung über. Man lässt 48h bei dieser Temperatur und lässt abkühlen. Man gibt auf Wasser und schüttelt mit Essigester (0.1% CF3COOH) aus. Nach dem trocknen und einengen hinterbleibt ein chromatographisch einheitliches rotes Pulver.
Ausbeute: 7.2mg ( 71%.d.Th.); MS: 632/634 (M+H)+; λₘₐₓ= 504.0nm

### Beispiel 248

**(85)-4',9.9'-trihydroxy-6'-allyloxy-3-[(1E,3E)-penta-1,3-dienyl]-6,7-dihydrospiro[cyclopenta[g]isoquinoline-8,2'-cyclopenta[b]naphtalene]-1,1'-3',5',8'(2H)-pentone (248)** 9.6mg (0.01824mmol) Fredericamycin (1)werden in 3.0ml Allylalkohol suspendiert. Unter N2-Atmosphäre werden 58mg Kaliumacetat (wasserfrei) hinzugefügt und auf 70oC erwärmt. Nach wenigen Minuten geht die Suspension in eine tiefblaue Lösung über. Man belässt 2.5h bei dieser Temperatur und lässt abkühlen. Man gibt auf Wasser und schüttelt mit Essigester (0.1% CF3COOH) aus. Nach dem trocknen und einengen hinterbleibt ein chromatographisch einheitliches rotes Pulver.
Ausbeute: 9.2mg ( 91%.d.Th.); MS: 566 (M+H)+; λₘₐₓ= 500.0nm

Die Verbindungen 249, 250, 251, 252, 253, 254, 255 wurden analog den Vorschriften 244 - 248 hergestellt.

### Beispiel 249

**(8S)-4',9.9'-trihydroxy-6'-(2-hydroxyethoxy)-3-[(1E,3E)-penta-1,3-dienyl]-6,7-dihydrospiro[cyclopenta[g]isoquinoline-8,2'-cyclopenta[b]naphtalene]-1,1'-3',5',8'(2H)-pentone (249)**
Ausbeute:5.2mg ( 52%.d.Th.); MS: 569 (M)+; λₘₐₓ= 499.0nm

### Beispiel 250

**(8S)-4',9.9'-trihydroxy-6'-benzyloxy-3-[(1E,3E)-penta-1,3-dienyl]-6,7-dihydrospiro[cyclopenta[g]isoquinoline-8,2'-cyclopenta[b]naphtalene]-1,1'-3',5',8'(2H)-pentone (250)**
Ausbeute:10.2mg ( 99%.d.Th.); MS: 616 (M+H)+; λₘₐₓ= 504.0nm

### Beispiel 251

**(8S)-4',9.9'-trihydroxy-6'-cyclopropylmethoxy-3-[(1E,3E)-penta-1,3-dienyl]-6,7-dihydrospiro[cyclopenta[g]isoquinoline-8,2'-cyclopenta[b]naphtalene]-1,1'-3',5',8'(2H)-pentone (251)** Ausbeute:12.9mg ( 99%.d.Th.); MS: 580 (M)+; λₘₐₓ= 500.0nm

### Beispiel 252

**1-Deoxy-5-C-[(8R)-4',9,9'-trihydroxy-6'-ethoxy-1,1',3',5',8'-pentaoxo-1,1',2,3',5',6,7,8'-octahydrospiro [cyclopenta [g]isoquinoline-8,2'-cyclopenta[b]-naphtalen]-3-yl]pentitol (252)**
Ausbeute:2.0mg ( 20%.d.Th.); MS: 622 (M+H)+; λₘₐₓ= 499.0nm

### Beispiel 253

**(8S)-4',9.9'-trihydroxy-6'-(2-t-butoxycarbonylaminoethoxy)-3-[(1E,3E)-penta-1,3-dienyl]-6,7-dihydrospiro [cyclopenta[g]-isoquinoline-8,2'-cyclopenta[b]naphtalene]-1,1'-3',5',8'(2H)-pentone (251)**
Ausbeute:12.9mg ( 99%.d.Th.); MS: 669 (M)+; λₘₐₓ= 500.0nm

### Beispiel 254

**(8S)-4',9.9'-trihydroxy-6'-(2-N,N-diisopropylaminoethoxy)-3-[(1E,3E)-penta-1,3-dienyl]-6,7-dihydrospiro [cyclopenta[g]-isoquinoline-8,2'-cyclopenta[b]naphtalene]-1,1'-3',5',8'(2H)-pentone (254)**
Ausbeute:5.8mg ( 48%.d.Th.); MS: 653 (M+H)+; λₘₐₓ= 500.0nm

### Beispiel 255

**1-Deoxy-5-C-[(8R)-4',9,9'-trihydroxy-6'-ethoxy-1,1',3',5',8'-pentaoxo-1,1',2,3',5',6,7,8'-octahydrospiro [cyclopenta [g]isoquinoline-8,2'-cyclopenta[b]-naphtalen]-3-yl]pentitol (255)**
Ausbeute:5.5mg ( 50%.d.Th.); MS: 594 (M+H)+; λₘₐₓ= 500.0nm

### Beispiel 256

**(8S)-4',9.9"-trihydroxy-6"-(2-bromethoxy)-3-[(1E,3E)-penta-1,3-dienyl]-6,7-dihydrospiro[cyclopenta[g]isoquinoline-8,2'-cyclopenta[b]naphtalene]-1,1'-3',5',8'(2H)-pentone (256)** 10.6mg (0.02014mmol) Fredericamycin (1)werden in 2.0ml Bromethanol suspendiert. Unter N2-Atmosphäre werden 150mg Kaliumacetat (wasserfrei) hinzugefügt und auf 120oC erwärmt. Nach wenigen Minuten geht die Suspension in eine tiefblaue Lösung über. Nach 12h weitere Zugabe von 150mg Kaliumacetat. Man belässt nocheinaml 12h bei dieser Temperatur und lässt abkühlen. Man gibt auf Wasser und schüttelt mit Essigester (0.1% CF3COOH) aus. Nach dem trocknen und einengen hinterbleibt ein chromatographisch einheitliches rotes Pulver., Ausbeute:11.5mg (99%.d.Th.); MS: 632/634 (M+H)+; λₘₐₓ= 499.0nm

### Beispiel 257

**(8S)-5-iodo-4',9.9'-trihydroxy-6'-cyclopropylamino-3-[(1E,3E)-penta-1,3-dienyl]-6,7-dihydroapiro[cyclopenta[g]isoquinoline-8,2'-cyclopenta[b]naphtalene]-1,1'-3',5',8'(2H)-pentone (257)** 5.0mg (7.5µmol) 5-Iodo-fredericamycin **(15)**werden unter Argon in 1ml wasserfreiem DMF gelöst. Nach der Zugabe von 0.64mg (11.2µmol) Cyclopropylamin rührt man 3h bei Raumtemperatur. Überschüssiges Cyclopropylamin und DMF werden im Hochvakuum abgezogen. Nach dem trocknen und einengen hinterbleibt ein chromatographisch einheitliches rotes Pulver.
Ausbeute: 5.1mg (99%); MS: 691.3 (M+H)+; λₘₐₓ= 504.0nm

### Beispiel 258

**(8S)-5-iodo-4',9.9'-trihydroxy-6'-n-butylamino-3-(1E,3E)-penta-1,3-dienyl]-6,7-dihydrospiro[ayclopenta[g]isoquinoline-8,2'-cyclopenta[b]naphtalene]-1,1'-3',5',8'(2H)-pentone (258)**
5.0mg (7.5µmol) 5-Iodo-fredericamycin **(15)** werden unter Argon in 1ml wasserfreiem DMF gelöst. Nach der Zugabe von 0.82mg (11.2µmol) n-Butylamin rührt man 20h bei Raumtemperatur. Überschüssiges n-Butylamin und DMF werden im Hochvakuum abgezogen. Nach dem trocknen und einengen hinterbleibt ein chromatographisch einheitliches rotes Pulver.
Ausbeute: 5.3mg (99%); MS: 707.3 (M+H)+; λₘₐₓ= 504.0nm

### Beispiel 259

**(8S)-5-bromo-4',9.9'-trihydroxy-6'-n-butylamino-3-[(1E,3E)-penta-1,3-dienyl]-6,7-dihydrospiro[cyclopenta[g]isoquinoline-8,2'-cyclopenta[b]naphtalene]-1,1'-3',5',8'(2H)-pentone (259)**
5.0mg (8.1µmol) 5-Bromo-fredericamycin **(14)** werden unter Argon in 1ml wasserfreiem DMF gelöst. Nach der Zugabe von 0.89mg (12.2µmol) n-Butylamin rührt man 20h bei Raumtemperatur. Überschüssiges n-Butylamin und DMF werden im Hochvakuum abgezogen. Nach dem trocknen und einengen hinterbleibt ein chromatographisch einheitliches rotes Pulver.
Ausbeute: 5.3mg (99%); MS: 659.4/661.4 (M+H)+; λₘₐₓ= 504.0nm

### Beispiel 260

**(8S)-4',9.9'-trihydroxy-6'-cyclopropylamino-3-[(1E,3E)-penta-1,3-dienyl]-6,7-dihydrospiro[cyclopenta[g]isoquinoline-8,2"-cyclopenta[b]naphtalene]-1,1'-3',5',8'(2H)-pentone (260)**
5.0mg (9.3µmol) Fredericamycin **(1)** werden unter Argon in 1ml wasserfreiem DMF gelöst. Nach der Zugabe von 2.12mg (37.2µmol) Cyclopropylamin rührt man 2h bei Raumtemperatur. Überschüssiges Cyclopropylamin und DMF werden im Hochvakuum abgezogen. Nach dem trocknen und einengen hinterbleibt ein chromatographisch einheitliches rotes Pulver.
Ausbeute: 5.1mg (99%); MS: 565.4 (M+H)+; λₘₐₓ= 510.0nm

### Beispiel 261

**(8S)-4',9.9'-trihydroxy-6'-anilino-3-[(1E,3E)-penta-1,3-dienyl]-6,7-dihydrospiro[cyclopenta[g]isoquinoline-8,2'-cyclopenta[b]naphtalene]-1,1'-3',5',8'(2H)-pentone (261)**
5.0mg (9.3µmol) Fredericamycin **(1)** werden unter Argon in 1ml wasserfreiem DMF gelöst. Nach der Zugabe von 3.46mg (37.2µmol) Anilin und 37.2µg Zinn(IV)chlorid (1.0M in CH2Cl2) erwärmt man auf 60oC. Die Reaktionsmischung wird 24h gerührt und anschließend mit überschüssigem Diethanolaminomethyl-polystyrol Harz versetzt. 1h rühren. Vom Harz absaugen und mit DMF waschen. Die organische Phase wird im Hochvakuum eingeengt. Es hinterbleibt ein chromatographisch einheitliches rotes Pulver.
Ausbeute: 5.5mg (99%); MS: 601.1 (M+H)+; λₘₐₓ= 504.0nm

### Beispiel 262

**(8S)-4',9.9'-trihydroxy-6'-piperidino-3-[(1E,3E)-penta-1,3-dienyl]-6,7-dihydrospiro[cyclopenta[g]isoquinoline-8,2'-cyclopenta[b]naphtalene]-1,1'-3',5',8'(2H)-pentone (262)**
5.0mg (9.3µmol) Fredericamycin **(1)** werden unter Argon in 1ml wasserfreiem DMF gelöst. Nach der Zugabe von 3.16mg (37.2µmol) Piperidin rührt man 22h bei Raumtemperatur. Überschüssiges Amin und DMF werden im Hochvakuum abgezogen. Es hinterbleibt ein chromatographisch einheitliches rotes Pulver.
Ausbeute: 5.5mg (99%); MS: 593.4 (M+H)+; λₘₐₓ= 504.0nm

### Beispiel 263

**(8S)-4',9.9'-trihydroxy-6'-dimethylamino-3-[(1E,3E)-penta-1,3-dienyl]-6,7-dihydrospiro[cyclopenta[g]isoquinoline-8,2'-cyclopenta[b]naphtalene]-1,1'-3',5',8'(2H)-pentone (263)**
5.0mg (9.3µmol) Fredericamycin **(1)** werden unter Argon in 1ml wasserfreiem DMF gelöst. Nach der Zugabe von 1.67mg (37.2µmol) Dimethylamin ( 2M in MeOH ) rührt man 4h bei Raumtemperatur. Überschüssiges Amin und DMF werden im Hochvakuum abgezogen. Es hinterbleibt ein chromatographisch einheitliches rotes Pulver. Ausbeute: 5.5mg (99%); MS: 553.6 (M+H)+; λₘₐₓ= 526.0nm

### Beispiel 264

**(8S)-4',9.9'-trihydroxy-6'-isopropylamino-3-[(1E,3E)-penta-1,3-dienyl]-6,7-dihydrospiro[cyclopenta[g]isoquinoline-8,2'-cyclopenta[b]naphtalene]-1,1'-3',5',8'(2H)-pentone (264)**
5.0mg (9.3µmol) Fredericamycin **(1)** werden unter Argon in 1ml wasserfreiem DMF gelöst. Nach der Zugabe von 2.19mg (37.2µmol) Isopropylamin rührt man 4h bei Raumtemperatur. Überschüssiges Amin und DMF werden im Hochvakuum abgezogen. Es hinterbleibt ein chromatographisch einheitliches rotes Pulver.
Ausbeute: 5.2mg (99%); MS: 567.3 (M+H)+; λₘₐₓ= 504.0nm

### Beispiel 265

**(8S)-4',9.9'-trihydroxy-6'-methylamino-3-[(1E,3E)-penta-1,3-dienyl]-6,7-dihydrospiro[cyclopenta[g]isoquinoline-8,2'-cyclopenta[b]naphtalene]-1,1'-3',5',8'(2H)-pentone (265)**
5.0mg (9.3µmol) Fredericamycin **(1)** werden unter Argon in 1ml wasserfreiem DMF gelöst. Nach der Zugabe von 0.34mg (11.1µmol) Methylamin (2M in CH3OH) rührt man 19h bei Raumtemperatur. Überschüssiges Amin und DMF werden im Hochvakuum abgezogen. Es hinterbleibt ein chromatographisch einheitliches rotes Pulver. Ausbeute: 5.0mg (99%); MS: 539.2 (M+H)+; λₘₐₓ= 504.0nm

### Beispiel 266

**(8S)-5-iodo-4',9.9'- -trihydroxy-6'-methylamino-3-[(1E,3E)-penta-1,3-dienyl]-6,7-dihydrospiro[cyclopenta[g]isoquinoline-8,2'-cyclopenta[b]naphtalene]-1,1'-3',5',8'(2H)-pentone (266)**
5.0mg (7.5µmol) 5-Iodo-fredericamycin **(15)** werden unter Argon in 1ml wasserfreiem DMF gelöst. Nach der Zugabe von 0.28mg (9.0µmol) Methylamin (2M in CH3OH) rührt man 2h bei Raumtemperatur. Überschüssiges Amin und DMF werden im Hochvakuum abgezogen. Es hinterbleibt ein chromatographisch einheitliches rotes Pulver. Ausbeute: 5.0mg (99%); MS: 665.2 (M+H)+; λₘₐₓ= 492.0nm

### Beispiel 267

**(8S)-4',9.9'-trihydroxy-6'-morpholino-3-[(1E,3E)-penta-1,3-dienyl]-6,7-dihydrospiro[cyclopenta[g]isoquinoline-8,2'-cyclopenta[b]naphtalene]-1,1'-3',5',8'(2H)-pentone (267)**
5.0mg (9.3µmol) Fredericamycin **(1)** werden unter Argon in 1ml wasserfreiem DMF gelöst. Nach der Zugabe von 3.24mg (37.2µmol) Morpholin rührt man 18h bei Raumtemperatur. Überschüssiges Amin und DMF werden im Hochvakuum abgezogen. Es hinterbleibt ein chromatographisch einheitliches rotes Pulver.
Ausbeute: 5.5mg (99%); MS: 595.5 (M+H)+; λₘₐₓ= 518.0nm

### Beispiel 268

**(8S)-4',9.9'- -trihydroxy-6'-amino-3-[(1E,3E)-penta-1,3-dienyl]-6,7-dihydrospiro[cyclopenta[g]isoquinoline-8,2'-cyclopenta[b]naphtalene]-1,1'-3',5',8'(2H)-pentone (268)**
5.0mg (9.3µmol) Fredericamycin **(1)** werden unter Argon in 1ml wasserfreiem DMF gelöst. Nach der Zugabe von 0.67mg (37.2µmol) Ammoniak (2M in EtOH) rührt man 24h bei Raumtemperatur. Überschüssiger Ammoniak und DMF werden im Hochvakuum abgezogen. Es hinterbleibt ein chromatographisch einheitliches rotes Pulver. Ausbeute: 4.8mg (99%); MS: 525.4 (M+H)+; λₘₐₓ= 504.0nm

### Beispiel 269

**(8S)-4',9.9'-trihydroxy-6'-pyrrolidino-3-[(1E,3E)-penta-1,3-dienyl]-6,7-dihydrospiro[cyclopenta[g]isoquinoline-8,2'-cyclopenta[b]naphtalene]-1,1'-3',5',8'(2H)-pentone (269)**
5.0mg (9.3µmol) Fredericamycin **(1)** werden unter Argon in 1ml wasserfreiem DMF gelöst. Nach der Zugabe von 0.99mg (13.9µmol) Pyrrolidin rührt man 19h bei Raumtemperatur. Überschüssiges Amin und DMF werden im Hochvakuum abgezogen. Es hinterbleibt ein chromatographisch einheitliches rotes Pulver.
Ausbeute: 5.3mg (99%); MS: 579.2 (M+H)+; λₘₐₓ= 554.0nm

### Beispiel 270

**(8S)-5-bromo-4',9.9'-trihydroxy-6'-cyclopropylamino-3-[(1E,3E)-penta-1,3-dienyl]-6,7-dihydrospiro[cyclopenta[g]isoquinoline-8,2'-cyclopenta[b]naphtalene]-1,1'-3',5',8'(2H)-pentone (270)**
5.0mg (8.1µmol) 5-Bromo-fredericamycin **(14)** werden unter Argon in 1ml wasserfreiem DMF gelöst. Nach der Zugabe von 0.70mg (12.2µmol) Cyclopropylamin rührt man 5h bei Raumtemperatur. Überschüssiges Cyclopropylamin und DMF werden im Hochvakuum abgezogen. Nach dem trocknen und einengen hinterbleibt ein chromatographisch einheitliches rotes Pulver.

Ausbeute: 5.2mg (99%); MS: 643.4/645.4 (M+H)+; λₘₐₓ= 492.0nm

### Beispiel 271

**2-[Acetyl]-3-[(8S)-4',9.9.'-trihydroxy-6'-Methoxy-1,1',3',5',8'-pentaoxo-1,1',2,3',5',6,7,8'-octahydrospiro-[cyclopenta[g]isoquinoline-8,2'-cyclopenta[b]naphtalen]-3-yl]ethen (271)**
79.5mg ( 479µmol ) (2-Oxo-propyl)-phosphonic acid dimethylester werden unter Argon in 8ml absolutem Pyridin gelöst und bei 0°C mit 60.2µl (479µmol) 1,1,3,3-Tetramethylguanidin versetzt. Nach 5 Minuten fügt man bei 0°C 80.0mg ( 159.7µmol) Fredericamycin Aldehyd **(4)** hinzu. Nach 2 Stunden trägt man in 100ml 1M Salzsäure ein und saugt vom ausgefallenenen Produkt ab. Trocknen im Hochvakuum. Ausbeute: 60.0mg ( 69%d.Th. ). M/e=542.2; λₘₐₓ = 492.0nm

### Beispiel 272

**2-[Bromacetyl]-3-[(8S)-5-bromo-4',9.9.'-trihydroxy-6'-Methoxy-1,1',3',5',8'-pentaoxo-1,1',2,3',5',6,7,9'-octahydrospiro-[cyclopenta[g]isoquinoline-8,2'-cyclopenta[b]naphtalen]-3-yl]ethen (272)**
50mg (92.4µmol) Acetyl-Fredericamycin werden unter Argon in 5ml absolutem DMF gelöst und dann unter Lichtausschluss mit 36.9mg (231.1µmol)Brom als 1M Brom-Lösung in DMF versetzt. Man rührt 23h unter Lichtausschluss und trägt dann in 100ml Wasser ein. Das ausgefallene Produkt wird abgesaugt und im Hochvakuum getrocknet. Ausbeute: 57.0mg (87% d.Th. ) rotes Pulver; M/e=697.9/699.9/701.9
M+; λₘₐₓ = 504.0nm

### Beispiel 273

**2-[2-Amino-thiazol-4-yl]-3-[(8S)-5-bromo-4',9.9.'-trihydroxy-6'-Methoxy-1,1',3',5',8'-pentaoxo-1,1',2,3',5',6,7,8'-octahydrospiro-[cyclopenta[g]isoquinoline-8,2'-cyclopenta[b]naphtalen]-3-yl]ethen (273)** 20.0mg (28.7µmol) Bromacetyl Fredericamycin **(273)** werden unter Argon in 4ml DMF gelöst. Bei Raumtemperatur versetzt man nacheinander mit 3.3mg (43.0µmol) Thioharnstoff und 20mg IR120 H+. Nach 2 Stunden wird vom Harz abfiltriert und in 50ml Wasser eingetragen. Das ausgefallene Produkt wird im Hochvakuum getrocknet. Rotes Pulver. Ausbeute: 18.0mg (93% d.Th. ); M/e=676.1/678.1 (M+H); λₘₐₓ, = 492.0nm

### Beispiel 274

**2-[2-Phenyl-thiazol-4-yl]-3-[(8S)-5-bromo-4',9.9.'-trihydroxy-6'-Methoxy-1,1',3',5',8'-pentaoxo-1,1',2,3',5',6,7,8'-octahydrospiro-[cyclopenta[g]isoquinoline-8,2'-cyclopenta[b]naphtalen]-3-yl]ethen (274)**
5.0mg (7.2µmol) Bromacetyl Fredericamycin **(273)** werden unter Argon in 1ml DMF gelöst. Bei Raumtemperatur versetzt man nacheinander mit 1.5mg (10.8µmol) Thiobenzamid und 5mg IR120 H+. Nach 3.5 Stunden Zugabe von Hydrazinosulfonylharz und 2 Stunden rühren.Es wird vom Harz abfiltriert und in 10ml Wasser eingetragen. Das ausgefallene Produkt wird im Hochvakuum getrocknet. Rotes Pulver.
Ausbeute: 3.0mg (57% d.Th. ); M/e=737.2/739.2 (M+H); λₘₐₓ = 492.0nm

### Beispiel 275

**2-[2-Acetylamino-thiazol-4-yl]-3-[(8S)-5-bromo-4',9.9.'-trihydroxy-6'-Methoxy-1,1',3',5',8'-pentaoxo-1,1',2,3',5',6,7,8'-octahydrospiro-[cyclopenta[g]isoquinoline-8,2'-cyclopenta[b]naphtalen]-3-yl]ethen (275)**
5.0mg (7.2µmol) Bromacetyl Fredericamycin **(273)** werden unter Argon in 1ml DMF gelöst. Bei Raumtemperatur versetzt man nacheinander mit 1.3mg (10.8µmol) Acetylthioharnstoff und 5mg IR120 H+. Nach 22 Stunden Zugabe von 5mg Hydrazinosulfonylharz und 2 Stunden nachrühren. Vom Harz abfiltriert und in 10ml Wasser eingetragen.

Das ausgefallene Niederschlag wird abgesaugt und im Hochvakuum getrocknet. Rotes Pulver.

Ausbeute: 2.0mg (39% d.Th. ); M/e=718.3/720.4 (M+H); λₘₐₓ = 492.0nm

### Beispiel 276

**2-[2-methyl-thiazol-4-yl]-3-[(8S)-5-bromo-4',9.9.'-trihydroxy-6'-Methoxy-1,1',3',5',8'-pentaoxo-1,1',2,3',5',6,7,8'-octahydrospiro-[cyclopenta[g]isoquinoline-8,2'-cyclopenta[b]naphtalenj-3-yl]ethen (276)**
5.0mg (7.2µmol) Bromacetyl Fredericamycin **(273)** werden unter Argon in 1ml DMF gelöst. Bei Raumtemperatur versetzt man nacheinander mit 0.81mg (10.8µmol) Thioacetamid und 5mg IR120 H+. Nach 2 Stunden Zugabe von Hydrazinosulfonylharz und 2 Stunden weiterühren. Es wird vom Harz abfiltriert und in 10ml Wasser eingetragen. Das ausgefallene Produkt wird im Hochvakuum getrocknet. Rotes Pulver. Ausbeute: 3.0mg (62% d.Th. ); M/e=675.2/677.2 (M+H); λₘₐₓ = 492.0nm

### Beispiel 277

**(8S)-4',9.9'-trihydroxy-6'-methoxy-3-[(1E,3E)-penta-1,3-dienyl]-6,7-dihydrospiro[cyclopenta[g]isoquinoline-8,2'-cyclopenta[b]naphta-lene]-1-thio-,1'-3',5',8'(2H)-tetrone -Thiofredericamycin- (277)**
10.0mg (18.5mmol) Fredericamycin **(1)** wird unter Argon in 2ml absolutemn Pyridin vorgelegt. Nach der Zugabe von 20.5mg (92.5mmol) Phosphor-V-sulfid erwärmt man 12h auf 60oC. Weitere zugabe von 20.5mg (92.5mmol) Phosphor-V-sulfid. Nach 1h war die reaktion laut HPLC (Acetonitril/Wasser/CF3COOH) beendet. Man gibt auf Wasser und schüttelt mit Essigester aus. Trocknen und einengen.

Violettrotes Pulver.

Ausbeute: 5.0mg (49% d.Th.) M/e=55.7(M+H); λₘₐₓ = 504.0nm

### Beispiel A

Wasserlöslichkeit der Fredericamycin-Derivate

Die Wasserlöslichkeit der verschiedenen Fredericamycin-Derivate kann in 0.9 %iger NaCl-Lösung mit einem pH-wert von 7 bestimmt werden.

Die Verbindungen (22) und (3) sind sehr gut löslich. Die Verbindung (6) ist gut löslich und die Verbindungen (2), (10) und (13) sind löslich. Die Verbindungen (5), (7), (11) und (12) sind ausreichend und deutlich besser löslich als Fredericamycin (Verbindung (1)).

## Patentansprüche

1. Verbindungen gemäß allgemeiner Formel Ia oder Ib: 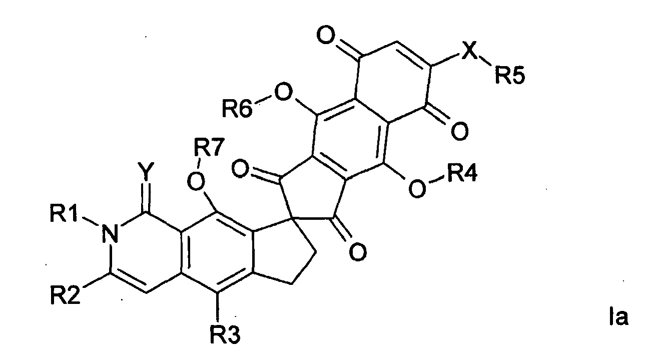 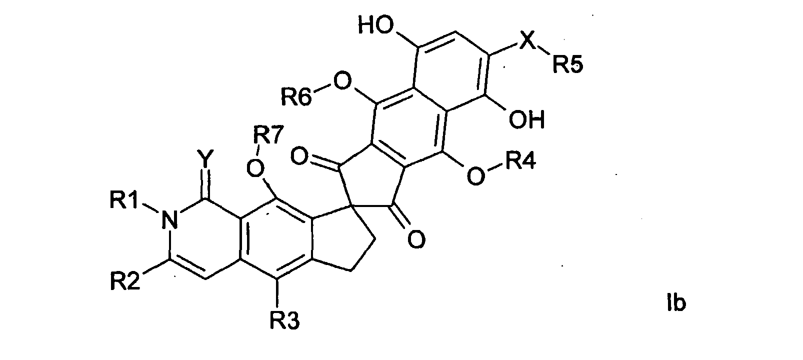 wobei jeweils
R1 H, C₁-C₆-Alkyl, Cycloalkyl, C₁-C₄-Alkyl-Cycloalkyl,
R2 H, C₁-C₁₄-Alkyl, C₂-C₁₄-Alkenyl, Aryl, C₁-C₄-Alkyl-Aryl, Heteroaryl, C₁-C₄-Alkyl-Heteroaryl, C₂-C₄-Alkenyl-Heteroaryl, Cycloalkyl, C₁-C₄-Alkyl-Cycloalkyl, Heterocycloalkyl, C₁-C₄-Alkyl-Heterocycloalkylj, CₘH₂ₘ₊ₒ₋ₚYₚ (mit m = 1 bis 6, für o = 1, p =.1 bis 2m+o; für m = 2 bis 6, o = -1, p .= 1 bis 2m+o; für m = 4 bis 6, o = -2, p = 1 bis 2m+o; Y = unabhängig von einander ausgewählt aus der Gruppe Halogen, OH, OR21, NH₂, NHR21, NR21R22, SH, SR21), (CH₂)ᵣCH₂NHCOR21, (CH₂)ᵣCH₂OCOR21, (CH₂)ᵣCH₂NHCSR21, (CH₂)ᵣCH₂S(O)nR21 mit n = 0,1,2, (CH₂)ᵣCH₂SCOR21, (CH₂)ᵣCH₂OSO₂-R21, (CH₂)ᵣCHO, (CH₂)ᵣCH=NOH, (CH₂)ᵣCH(OH)R21, -(CH₂)ᵣCH=NOR21, (CH₂)ᵣCH=NOCOR21, (CH₂)ᵣCH=NOCH₂CONR21R22, (CH₂)ᵣCH=NOCH(CH₃)CONR21R22, - -(CH₂)ᵣCH=NOC(CH₃)₂CONR21R22, (CH₂)ᵣCH=N-NHCO-R23, (CH₂)ᵣCH=N-NHC(O)NH-R23, (CH₂)ᵣCH=N-NHC(S)NH-R23, (CH₂)ᵣCH=N-NHC(NH)NH-R23, (CH₂)ᵣCH=N-NHC(NH)-R23, (CH₂)ᵣCH=N-NHCO-CH₂NHCOR21, (CH₂) ᵣCH=N-O-CH₂NHCOR21, (CH₂)ᵣCH=N-NHCS-R23, (CH₂)ᵣCH=CR24R25 ( trans oder cis ), (CH₂)ᵣCOOH, (CH₂)ᵣCOOR21, (CH₂)ᵣCONR21R22, -(CH₂)ᵣCH=NR21,(CH₂)ᵣCH=N-NR21R22, 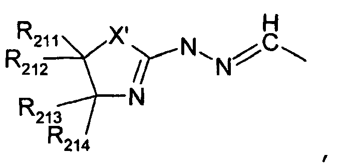 und der
(CH₂)ᵣ-kettenverlängerte Rest (CH₂)ᵣCH=N-N-(C₃NX'R211R212R213R214) (mit X' = NR215, O, S und R211, R212, R213, R214, R215 unabhängig voneinander H oder C₁-C₆-Alkyl), -(CH₂)ᵣCH=N-NHSO₂-Aryl, -(CH₂)ᵣCH=N-NHSO₂-Heteroaryl, mit r = 0,1,2,3,4,5, bevorzugt 0,
R21, R22 unabhängig voneinander H, C₁-C₁₄-Alkyl, C₁-C₁₄-Alkanoyl, C₁₋ C₆-Alkylhydroxy, C₁-C₆-Alkoxy, C₁-C₆-Alkylamino, C₁-C₆-Alkylamino-C₁-C₆-Alkyl, C₁-C₆-Alkylamino-di-C₁-C₆-Alkyl, Cycloalkyl, C₁-C₄-Alkyl-Cycloalkyl, Heterocycloalkyl, C₁-C₄-Alkyl-Heterocycloalkyl, Aryl, Aryloyl, C₁-C₄-Alkyl-Aryl, Heteroaryl, Heteroaryloyl, C₁-C₄-Alkyl-Heteroaryl, Cycloalkanoyl, C₁-C₄-Alkanoyl-Cycloalkyl, Heterocycloalkanoyl, C₁-C₄-Alkanoyl-Heterocycloalkyl, C₁-C₄-Alkanoyl-Aryl, C₁-C₄-Alkanoyl-Heteroaryl, Mono- und Di-Zuckerreste, die verknüpft sind über ein C-Atom, das im Zucker eine OH-Gruppe tragen würde, wobei die Zucker unabhängig voneinander ausgewählt sind aus der Gruppe bestehend aus Glucuronsäure und ihren Stereoisomeren an allen optischen C-Atomen, Aldopentosen, Aldohexosen einschließlich ihren Desoxyverbindungen (wie z.B. Glucose, Desoxyglucose, Ribose, Desoxyribose), oder R21 und R22 zusammen mit dem N eine 4, 5, 6, 7 oder 8 gliedrigen Ring bilden, der optional noch ein weiteres Herteroatom ausgewählt aus der Gruppe N, O, S enthalten kann,
R23 unabhängig von R21, die selben Bedeutungen wie R21 oder CH₂pyridinium-salze, CH₂tri-C₁-C₆-alkylammonmium-salze, CONH₂, CSNH₂, CN, CH₂CN,
R24 unabhängig von R21, die selben Bedeutungen wie R21 oder H, CN, COCH₃, COOH, COOR21, CONR21R22, NH₂, NHCOR21
R25 unabhängig von R21, die selben Bedeutungen wie R21 oder H, CN, COCH₃, COOH, COOR21, CONR21R22, NH₂, NHCOR21
R24,R25 zusammen mit dem N eine 4, 5, 6, 7 oder 8 gliedrigen Ring bilden, der optional noch ein weiteres Herteroatom ausgewählt aus der Gruppe N, O, S enthalten kann,
R3 H, F, Cl, Br, I, , OH, OR31, NO₂, NH₂, NHR31, NR31R32, NHCHO, NHCOR31, NHCOCF₃, CH₃₋ₘHalₘ (mit Hal = Cl, F, insbesondere F, und m = 1, 2, 3), OCOR31,
R31,R32 unabhängig voneinander C₁-C₆-Alkyl, oder R31 und R32 zusammen mit dem N eine 4, 5, 6, 7 oder 8 gliedrigen Ring bilden, der optional noch ein weiteres Herteroatom ausgewählt aus der Gruppe N, O, S enthalten kann,
R5 H, C₁-C₂₀-Alkyl, Cycloalkyl, C₂-C₂₀-Alkenyl, C₂-C₁₀Alkinyle C₁-C₄-Alkyl-Cycloalkyl, Heterocycloalkyl, C₁-C₄-Alkyl-Heterocycloalkyl, Aryl, C₁-C₄-Alkyl-Aryl, Heteroaryl, C₁-C₄-Alkyl-Heteroaryl, CₘH₂ₘ₊ₒ₋ₚYp (mit m = 1 bis 6, für O= 1, P = 1 bis 2m+o; für m = 2 bis 6, o = -1, P= 1 bis 2m+o; für m = 4 bis 6, O = -2, P = 1 bis 2m+o; Y = unabhängig von einander ausgewählt aus der Gruppe Halogen, OH, OR51, NH₂, NHR51, NR51R52, SH, SR51), (CH₂)ₛCH₂NHCOR51, (CH₂)ₛCH₂NHCSR51 , (CH₂)ₛCH₂S(O)nR51 mit n=0, 1, 2, (CH₂)ₛCH₂SCOR51, (CH₂)ₛCH2OCOR511 (CH₂)ₛCH₂OSO₂-R51, (CH₂)ₛCH(OH)R51, (CH₂)ₛCOOH, (CH₂)ₛCOOR51, (CH₂)ₛCONR51R52, mit s = 0,1,2,3,4,5, bevorzugt 0, Mono- und Di-Zuckerreste, die verknüpft sind über ein C-Atom, das im Zucker eine OH-Gruppe tragen würde, wobei die Zucker unabhängig voneinander ausgewählt sind aus der Gruppe bestehend aus Glucuronsäure und ihren Stereoisomeren an allen optischen C-Atomen, Aldopentosen, Aldohexosen einschließlich ihren Desoxyverbindungen (wie z.B. Glucose, Desoxyglucose, Ribose, Desoxyribose), wobei die Monozuckerreste wie Aldopentosen, Aldohexosen einschließlich ihren Desoxyverbindungen (wie z.B. Glucose, Desoxyglucose, Ribose, Desoxyribose) bevorzugt sind, mit R51, R52 die unabhängig voneinander die Bedeutungen von R21, R22 annehmen können,
R4,R6,R7 unabhängig voneinander H, C₁-C₆-Alkyl, CO-R41
R41 unabhängig von R21, die selben Bedeutungen wie R21
X O, S, NH, N-R8 , wobei R8 unabhängig von R5 die gleichen Bedeutung wie R5 annehmen kann oder R5 und R8 zusammen mit dem N eine 4, 5, 6, 7 oder 8 gliedrigen Ring bilden, der optional noch ein weiteres Herteroatom ausgewählt aus der Gruppe N, O S enthalten kann,
oder X-R5 zusammen gleich H,
Y O, S, NR9 wobei R9 H oder C₁-C₆-Alkyl sein kann,
bedeutet, deren Stereoisomere, Tautomere und deren physiologisch verträglichen Salze oder Einschlussverbindungen, wobei die Reste außer bei Cyclodextrin-Einschlussverbindungen folgende Bedeutung nicht gleichzeitig annehmen dürfen für Formel Ia: R1: H, C₁-C₆-Alkyl, R2: C₁-C₆-Alkyl, C₂-C₆-Alkenyl, R3: H, R4 und R6 identisch und unabhängig voneinander H, C₁-C₆-Alkyl, CO-R41 mit R41 gleich C₁-C₆-Alkyl, Aryl, und R7 H, C₁-C₆-Alkyl., Y: O und für Formel Ib: R1: H, R2: Pentyl, 1-Pentenyl, 3- Pentenyl, 1,3-Pentdienyl, R3: H, R4 und R6 gleich H und X-R5 gleich Methoxy, Y: O.

2. Verbindungen gemäß Anspruch 1, wobei Formel I a oder I b, die Stereochemie von Formel II a oder II b 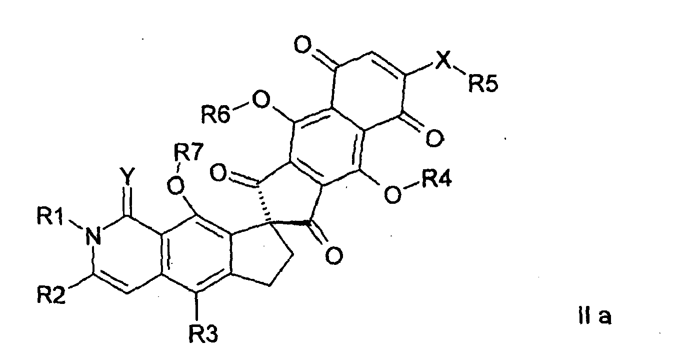 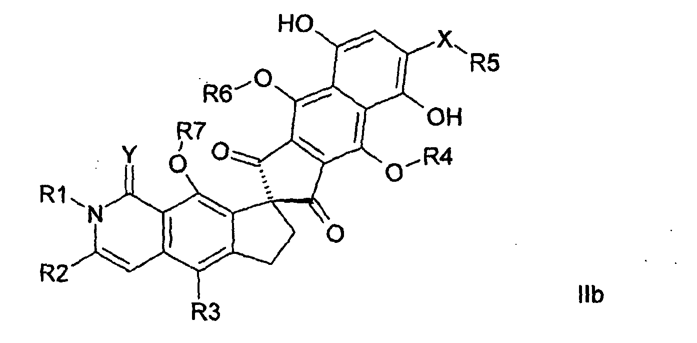 annimmt.

3. Verbindungen der Formel I a, I b, II a, II b gemäß Anspruch 1 oder 2, bei denen die Reste R bis auf R2, die oben angegebenen Bedeutungen haben und deren R2 gegenüber R2 gleich CH=CH-CH=CH-CH₃ die Wasserlöslichkeit bei Beibehaltung aller anderer Reste mindestens verdoppelt, bevorzugt mindestens verfünffacht, mehr bevorzugt mindestens verzehnfacht, besonders bevorzugt mindestens verfünfzigfacht, insbesondere verhundertfacht, oder sogar verfünfhundertfacht.

4. Verbindungen gemäß einem der Ansprüche 1 bis 3, wobei R3 F, Cl, Br, I, OH, OR31, NO₂, NH₂, NHR31, NR31R32, NHCHO, NHCOR31, NHCOCF₃, CH₃₋ₘHalₘ (mit Hal = Cl, F, und m = 1, 2, 3), OCOR31 bedeutet.

5. Verbindungen gemäß einem der Ansprüche 1 bis 4, wobei R3 (CH₂)ᵣCHO, (CH₂)ᵣCH=NOH, -(CH₂),CH=NOR21, (CH₂)ᵣCH=NOCOR21, (CH₂)ᵣCH=NOCH₂CONR21R22, (CH₂)ᵣCR=NOCH(CH₃)CONR21R22, (CH₂)ᵣCH=NOC (CH₃)₂CONR21R22, (CH₂)ᵣCH=N-NHCO-R23, (CH₂)ᵣeH=N-NHC(O)NH-R23, (CH₂)ᵣCH=N-MHC(S)NH-R23, (CH₂)ᵣCH=N-NHC(NH)NH-R23, (CH₂)ᵣCH=N-NHC(NH)-R23, (CH₂)ᵣCH=N-NHCO-CH₂NHCOR21, (CH₂)ᵣCH=N-O-CH₂NHCOR21, (CH₂)ᵣCH=N-NHCS-R23, (CH₂)ᵣCH=CR24R25 ( trans oder cis ), (CH₂) ᵣCH=NR21, (CH₂) ᵣCH=N-NR21R22, 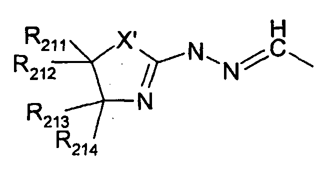 , und der (CH₂)ᵣ-kettenverlängerte Rest (CH₂)ᵣCH=N-N-(C₃NX'R211R212R213R214) (mit X' = NR215, O, S und R211, R212, R213, R214, R215 unabhängig voneinander H oder C₁-C₆-Alkyl), -(CH₂)ᵣCH=N-NHSO₂-Aryl, (CH₂)ᵣCH=N-NHSO₂-Heteroaryl, (CH₂)ᵣCH=C-Heteroaryl mit r = 0,1,2,3,4,5, bevorzugt 0,bedeutet.

6. Verbindungen gemäß einem der Ansprüche 1 bis 5, wobei X N oder S bedeutet oder X-R5 gleich OH ist.

7. Verbindungen gemäß einem der Ansprüche 1 bis 6, wobei
R1 H, C₁-C₅-Alkyl, Cycloalkyl, insbesondere H,
R2 C₁-C₅-Alkyl, C₁-C₄-Alkyl-Aryl, C₂-C₅-Alkenyl, Heteroaryl, C₁-C₄-Alkyl-Heteroaryl, CHF₂, CF₃, Polyolseitenkette insbesondere CHOH-CHOH-CHOH-CHOH-CH₃, CHOH-CHOH-CH=CH-CH₃, CH=CH-CHOH-CHOH-CH₃, CH₂Y (Y=F,Cl,Br,I),), CH₂NH₂, CH₂NR21R22, CH₂NHCOR23, CH₂NHCSR23, CH₂SH, CH₂S(O)nR21 mit n=0,1,2, CH₂SCOR21, insbesondere CH₂OH, CH₂OR21, CH₂OSO₂-R21, insbesondere CHO, CH(OR21)₂, CH(SR21)₂, CN, CH=NOH, CH=NOR21, CH=NOCOR21, CH=N-NHCO-R23, CH=CR24,R25 ( trans oder cis ), insbesondere COOH (insbesondere deren physiologisch verträglichen Salze), COOR21, CONR21R22, -CH=NR21, 112 -CH=N-NR21R22, 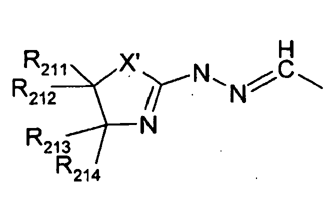 (mit X' = NR215, O, S und R211, R212, R213, R214, R215 unabhängig voneinander H oder C₁-C₆-Alkyl), -CH=N-NHSO₂-Aryl, -CH=N-NHSO₂-Heteroaryl, CH=N-NHCO-R23,
R21, R22 unabhängig voneinander C₁-C₆-Alkyl, Cycloalkyl, Aryl, C₁-C₄-Alkyl-Aryl, Heteroaryl, C₁-C₄-Alkyl-Heteroaryl
R23 unabhängig von R21, die selben Bedeutungen wie R21 oder CH₂pyridinium-salze, CH2tri-C₁-C₆-alkylammonmium-salze,
R24 unabhängig von R21, die selben Bedeutungen wie R21 oder H, CN, COCH₃, COOH, COOR21, CONR21R22, NH₂, NHCOR21
R25 unabhängig von R21, die selben Bedeutungen wie R21 oder H, CN, COCH₃, COOH, COOR21, CONR21R22, NH₂, NHCOR21
R24,R25 zusammen C₄-C₈-Cycloalkyl,
R3 F, Cl, Br, I, NO₂, NH₂, NHCOR31,
R31 unabhängig voneinander C₁-C₆-Alkyl,
R5 H, C₁-C₆-Alkyl, insbesondere C₁-C₃-Alkyl, C₃-C₈-Cycloalkyl, C₃-C₈-Cycloalkenyl, C₁-C₆-Alkenyl, C₁-C₆-Alkinyle, C₁-C₄-Alkyl-Cycloalkyl, Heterocycloalkyl, C₁-C₄-Alkyl-Heterocycloalkyl, Aryl, C₁-C₄-Alkyl-Aryl, Heteroaryl, C₁-C₄-Alkyl-Heteroaryl, CₘH₂ₘ₊ₒ-pYp (mit m = 1 bis 4, für O = 1, P = 1 bis 2m+o; für m = 2 bis 4, O = -1, P = 1 bis 2m+o; für m = 4, o = -2, P = 1 bis 2m+o; Y = unabhängig von einander ausgewählt aus der Gruppe Halogen, OH, OR21, NH₂, NHR21, NR21R22, SH, SR21), besonders bevorzugt ist Hydroxyalkyl mit einer oder mehreren OH Gruppen,
R4,R6,R7 unabhängig voneinander H, C₁-C₅-Alkyl, CO-R41
R41 unabhängig von R21, die selben Bedeutungen wie R21
X O, S, NH, N-R8
Y O, S
bedeutet.

8. Verbindungen gemäß einem der Ansprüche 1 bis 7 in der Form von Einschlussverbindungen mit Cyclodextrin, insbesondere alpha-Cyclodextrin.

9. Arzneimittel enthaltend Verbindungen nach einem der Ansprüche 1 bis 8 neben den üblichen Träger und Hilfsstoffen.

10. Arzneimittel nach Anspruch 9 in Kombination mit weitere Wirkstoffen zur Tumorbehandlung.

11. Verwendung von Verbindungen gemäß einem der Ansprüche 1 bis 8 zur Herstellung von Arzneimitteln zur Behandlung von Tumoren, insbesondere von solchen, die durch die Inhibierung der Topoisomerasen I und/oder II behandelt werden können oder bei denen eine Apoptose ausgelöst wird.

12. Verwendung von Verbindungen gemäß einem der Ansprüche 1 bis 8 oder Verbindungen bei denen folgende Bedeutungen gleichzeitig angenommen werden bei Formel Ia: R1: H, C₁-C₆-Alkyl, R2: C₁-C₆-Alkyl, C₂-C₆-Alkenyl, R3: H, R4 und R6 identisch und unabhängig voneinander H, C₁-C₆-Alkyl, CO-R41 mit R41 gleich C₁-C₆-Alkyl, Aryl, und R7 H, C₁-C₆-Alkyl und bei Formel Ib: R1: H, R2: Pentyl, 1-Pentenyl, 3-Pentenyl, 1,3-Pentdienyl, R3: H, R4 und R6 gleich H und X-R5 gleich Methoxy zur Herstellung von Arzneimitteln zur Behandlung von Parasiten.

13. Verwendung von Verbindungen gemäß einem der Ansprüche 1 bis 8 oder Verbindungen bei denen folgende Bedeutungen gleichzeitig angenommen werden bei Formel Ia: R1: H, C₁-C₆-Alkyl, R2: C₁-C₆-Alkyl, C₂-C₆-Alkenyl, R3: H, R4 und R6 identisch und unabhängig voneinander H, C₁-C₆-Alkyl, CO-R41 mit R41 gleich C₁-C₆-Alkyl, Aryl, und R7 H, C₁-C₆-Alkyl und bei Formel Ib: R1: H, R2: Pentyl, 1-Pentenyl, 3-Pentenyl, 1,3-Pentdienyl, R3: H, R4 und R6 gleich H und X-R5 gleich Methoxy zur Herstellung von Arzneimitteln zur Immunsupression.

14. Verwendung von Verbindungen gemäß einem der Ansprüche 1 bis 8 oder Verbindungen bei denen folgende Bedeutungen gleichzeitig angenommen werden bei Formel Ia: R1: H, C₁-C₆-Alkyl, R2: C₁-C₆-Alkyl, C₂-C₆-Alkenyl, R3: H, R4 und R6 identisch und unabhängig voneinander H, C₁-C₆-Alkyl, CO-R41 mit R41 gleich C₁-C₆-Alkyl, Aryl, und R7 H, C₁-C₆-Alkyl und bei Formel Ib: R1: H, R2: Pentyl, 1-Pentenyl, 3-Pentenyl, 1,3-Pentdienyl, R3: H, R4 und R6 gleich H und X-R5 gleich Methoxy zur Herstellung von Arzneimitteln zur Behandlung von Neurodermitis.
